# EUROPEAN PATENT APPLICATION

(11) **EP 4 194 449 A1**
(43) Date of publication of application: **14.06.2023**
(21) Application number: 21853576.3
(22) Date of filing: 05.08.2021
(51) Int. Cl.: C07D 401/06, C07D 401/14, C07D 413/06, C07D 417/06, C07D 405/06, C07D 407/06, A61K 31/454, A61K 31/4545, A61P 13/12, A61P 37/00, A61P 25/00

(54) **HETEROCYCLIC COMPOUND, PREPARATION METHOD THEREFOR AND USE THEREOF**

(30) Priority: 07.08.2020 CN 202010787537; 18.03.2021 CN 202110293149
(71) Applicant: Shanghai Meiyue Biotech Development Co., Ltd., Pudong New District, Shanghai 201206 (CN)
(72) Inventor: LUAN, Linbo, Shanghai 201206 (CN); CHEN, Yongkai, Shanghai 201206 (CN); WANG, Chaodong, Shanghai 201206 (CN)
(74) Representative: Gille Hrabal Partnerschaftsgesellschaft mbB Patentanwälte
(86) International application number: PCT/CN2021/110751
(87) International publication number: WO 2022/028507

(57) **Abstract**

A heterocyclic compound as represented by formula I, a preparation method therefor, a pharmaceutical composition comprising same and use thereof. The heterocyclic compound can be used as a complement factor B inhibitor, and is used for preparing a medicament for treating a disease related to abnormal activation of the complement system or occur in normal functioning of the complement system; and can be used as a therapeutic agent for a disease related to inflammation and immunity.

## Description

The present application claims priority to Chinese Patent Application No. 2020107875372 filed on Aug. 7, 2020 and Chinese Patent Application No. 2021102931493 filed on Mar. 18, 2021, which are incorporated herein by reference in their entirety.

### TECHNICAL FIELD

The present disclosure relates to a heterocyclic compound and a preparation method and use thereof.

### BACKGROUND

Complements are a class of soluble pattern recognition molecules in the immune system that can perform multiple effector functions. Under natural conditions, complement components are present as inactive zymogens, which are broken down through a variety of specific and nonspecific immunological mechanisms to produce large and small active fragments. The large fragments usually reside on the surface of pathogens or cells and lyse the latter or accelerate their clearance. The small fragments leave the cell surface and mediate multiple inflammatory responses. Complement activation consists of a process closely followed by another, and thus a cascade of reactions of complement activation form. Three primary complement activation pathways are known at present: the classical pathway, the lectin pathway, and the alternative pathway. Although the three complement activation pathways are started through different mechanisms and are activated in different orders, they share a common terminal pathway. The activation of the alternative pathway is independent of antigen-antibody complexes, and usually C3b deposited on the cell surface binds to factor B to be in such a state that it is easily decomposed by factor D in serum. In this process, factor B is decomposed into Ba and Bb. Then C3b and Bb form a complex as the C3 convertase C3bBb in the alternative pathway. In this process, complement factor B plays an early and leading role in the activation of the alternative pathway of the complement cascade. In this case, C3b is both a product of the C3 convertase's decomposition of C3 and a component of C3 convertase in the alternative pathway. As a result, there forms a feedback amplification mechanism of the interplay between the classical pathway and the alternative pathway. The current research reveals that many diseases such as blood, autoimmune, inflammatory and neurodegeneration diseases are associated with complement system dysfunction.

Paroxysmal nocturnal hemoglobinuria (PNH) is a chronic disease that causes constant hemolysis. It is a non-malignant clonal disease caused by acquired somatic mutation in the PIG-A gene of one or more hematopoietic stem cells, a very rare disease of the blood (Medicine (Baltimore) 1997, 76(2): 63-93). The course of the disease may manifest itself as various degrees of hemolytic exacerbation (paroxysmal), chronic or recurring episodes of acute intravascular hemolysis or subsequent venous/arterial thrombosis which may lead to progressive end-stage organ damage and death. Typical PNH is characterized by chronic intravascular hemolysis, hemoglobinuria and hemosiderinuria. However, in most patients, the disease is often atypical, insidious and persistent, and varies in severity.

There are more than ten kinds of proteins on the red cell surface that inhibit the activation of the complement pathways. They are all anchored to the cell membrane by glycosylated phosphatidylinositol (GPI) and thus are known collectively as GPI-anchored protein (AP). It is now believed that hematopoietic stem cells are mutated first under certain conditions and glycosylphosphatidylinositol-deficient PNH clones are produced; then because of some factors (immune factors are mostly believed now to be the cause), hematopoietic impairment or hematopoietic failure is caused, and PNH clones gain an advantage in proliferation over normal clones. The multiple antigens to which GPI is linked also contribute to the complexity of the interpretation of PNH cell biological behaviors. C3 convertase decay accelerating factor CD55 and membrane attack complex (MAC) inhibitor CD59, the most important proteins that inhibit complement pathway activation, are closely related to PNH in pathogenesis, clinical manifestations, diagnosis and treatment (Frontiers in Immunology 2019, 10, 1157). CD59 can prevent C9 from being incorporated into C5b-8 complex and thus the formation of membrane attack units, thereby achieving the inhibition of end-stage attack responses of complements. It is now believed that the typical manifestations of PNH-intravascular hemolysis and thrombosis-are due to CD59 deficiency. Congenital CD59 deficiency patients are reported to exhibit numerous typical manifestations of PNH, such as intravascular hemolysis, hemoglobinuria and venous thrombosis and the like. In PNH patients, CD59 is unable to bind to the cell membrane of red blood cells due to GPI synthesis defects and thus loses its function of inhibiting complement pathway activation. Therefore, the complement pathways are abnormally activated and red blood cells are attacked, leading to various clinical manifestations such as intravascular hemolysis, hemoglobinuria and smooth muscle dysfunction and the like. At present, there is no other effective clinical cure for PNH except that it can be cured by reconstitution of normal hematopoietic function through hematopoietic stem cell transplantation. As hematopoietic stem cell transplantation involves an element of risk and PNH is a benign clonal disease, controlling hemolysis episodes remains a major strategy for the clinical treatment of this disease. At present, only eculizumab is approved for treating PNH. However, many patients still experience anemia after being treated with eculizumab, and constant blood transfusion remains necessary for many of them. Moreover, eculizumab has to be intravenously injected when administered. Therefore, there is an unmet need to develop novel inhibitors of the complement pathways for treating PNH. IgAN is the most common primary glomerulonephritis. The disease is characterized by IgA deposition in the mesangial region indicated by immunofluorescence. It has diverse clinical manifestations, and usually manifests itself as recurrent microscopic or macroscopic hematuria. Available evidence suggests that the occurrence of IgAN is associated with congenital or acquired immune dysregulation. Due to irritation to the respiratory tract or the digestive tract caused by viruses, bacteria and food proteins and the like, mucosal IgA1 synthesis is increased, or IgA1-containing immune complexes are deposited in the mesangial region, thereby activating the alternative complement pathway and causing glomerular injury. Human IgA molecules are classified into 2 subtypes: IgA1 and IgA2. IgA1 is the major form (about 85%) of blood circulation in healthy individuals. It is also the major component of the deposition in the mesangial region in IgAN patients. IgA molecules can be present in monomeric form and in polymeric form. The IgA1 molecule contains a unique heavy chain hinge region between the first and second constant regions that can serve as a domain at the linking site for O-linked glycan groups. In recent years, it has been found that IgA molecules deposited in the serum and mesangial region of IgAN patients are mainly glycosylation-deficient IgA1(gd-IgA1). The abnormal increased production of gd-IgA1 is now believed to be the start of the pathogenesis of IgAN.

Complement C3 deposition occurs in the mesangial region of more than 90% of IgAN patients. Co-deposition of properdin, IgA and C3 occurs in kidney tissue of 75% to 100% of IgAN patients. Co-deposition of complement factors H, IgA and C3 occurs in kidney tissue of 30% to 90% of IgAN patients. In addition to deposition in kidney tissue, some studies have also revealed that the marker level of the alternative complement pathway in plasma of IgAN patients is also associated with the activity of IgAN (J Nephrol 2013, 26(4): 708-715). A study has confirmed that C3a in kidney tissue and urine and the C3a receptor in kidney tissue are significantly associated with the activity and severity of renal injury (J clin Immunol 2014, 34(2): 224-232). Other studies have confirmed that IgA is able to activate the alternative complement pathway *in vitro.* In this process, the abnormality in the IgA hinge region does not play a decisive role-rather, the IgA polymer formation is a critical step (Eur J Immunol 1987, 17(3): 321-326). Complement C3 deposition in the glomerular mesangial region has now become a marker that assists in diagnosis of IgAN. In a study, 163 IgAN patients were subjected to immunofluorescence assays for C3c and C3d. The results showed that IgAN patients in which the intensity of C3c deposition was stronger than that of C3d deposition had lower glomerular filtration rates, higher incidence of hyperplasia in the intraglomerular capillaries, and severer hematuria, which suggested that glomerular deposition of C3c was associated with the active pathological changes of IgAN (Am J Nephrol. 2000, 20(2): 122-128). At present, there is no specific drug for IgAN in clinical practice. General drugs such as renin-angiotensin inhibitors (ACEI or ARB), glucocorticoids and various immunosuppressive drugs and the like are mainly used. In addition, the safety of such drugs is also an important topic. For example, although glucocorticoids can ameliorate proteinuria, STOP-IgAN tests and TESTING-I tests have clearly confirmed the potential side effects of glucocorticoids. Therefore, it is necessary to have a talk with each patient about glucocorticoids' risks and benefits (IgA nephropathy 2019, 95, 4, 750-756).

Other diseases associated with the complement cascade comprise membranous nephropathy (MN), C3 glomerulonephritis (C3G), age-related macular degeneration (AMD), geographic atrophy (GA), atypical hemolytic uremic syndrome (aHUS), hemolytic uremic syndrome (HUS), hemodialysis complications, hemolytic anemia or hemodialysis, neuromyelitis optica (NMO), arthritis, rheumatoid arthritis, liver-related inflammations, dermatomyositis and amyotrophic lateral sclerosis, myasthenia gravis (MG), respiratory diseases and cardiovascular diseases and the like.

At present, there is no small-molecule complement factor B inhibitors for clinical treatment. In the discovery and development stage, there are the following published descriptions: an oligonucleotide drug developed by IONIS Pharmaceuticals Inc. is used as a complement factor B (CFB)-specific inhibitor for the treatment, prevention or alleviation of a disease associated with the dysregulation of the complement alternative pathway (WO2015038939). Small-molecule complement factor B inhibitors developed by Novartis AG Inc. are used for treating a disease such as age-related macular degeneration (AMD) and the like (WO2013164802, WO2013192345, WO2014143638, WO2015009616, WO2015066241) and for treating a disease such as C3G and IgAN and the like (WO2019043609A1). A small-molecule complement factor B inhibitor developed by Achillion Pharmaceuticals Inc. is used for treating a disease such as age-related macular degeneration (AMD) and the like (WO2018005552).

The characteristics of inflammation and immune-related diseases are diversity and refractoriness. Eculizumab is the only drug available for PNH disease. However, the drug places a heavy burden on patients due to its prize. In addition, many patients still experience anemia after being treated with eculizumab, and constant blood transfusion remains necessary for many of them. Moreover, eculizumab has to be intravenously injected when administered. At present, there is no specific drug for treating some diseases, such as IgAN and the like. There is an unmet clinical need in these areas. New small-molecule drugs need to be developed for medical treatment.

### SUMMARY

The present disclosure addresses the technical problem that the prior art lacks small-molecule compounds as complement factor B inhibitors, and provides a heterocyclic compound and a preparation method and use thereof. The heterocyclic compound provided herein can be used as a complement factor B inhibitor and used for the manufacturing of a medicament for treating a disease associated with abnormal activation of the complement system or occur in normal functioning of the complement system, and can be used as a therapeutic agent for inflammation and immune-related diseases. The compound is expected to meet the clinical need.

The present disclosure solves the technical problem described above through the following technical solutions.

The present disclosure provides a heterocyclic compound represented by formula I or a pharmaceutically acceptable salt, an isotopic analog or a prodrug thereof, which is optionally presented in a pharmaceutically acceptable carrier; 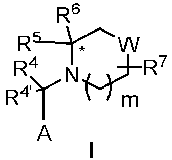 wherein,
W is O or C(R^{7'}R^{7"});
R⁷, R^{7'} and R^{7"} are independently hydrogen, hydroxy, halogen, C₁-C₄ alkyl or C₁-C₄alkyl-O-;
R⁶ is hydrogen, C₁-C₄ alkyl or hydroxy C₁-C₄ alkyl;
R^{4'} and R⁴ are independently hydrogen;
m is 0, 1 or 2;
R⁵ is 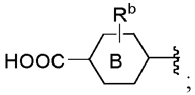 ring B is phenyl or 6-membered heteroaryl comprising 1, 2 or 3 heteroatoms selected from N, O and S;
R^{b} is H, hydroxy, =O, or a group ortho-fused to ring B, wherein the group is selected from phenyl, 3- to 6-membered cycloalkyl, 5- to 6-membered heterocycloalkyl and 5- to 6-membered heteroaryl; wherein the 5- to 6-membered heterocycloalkyl comprise 1, 2 or 3 heteroatoms selected from one or more of N, O, S, S(=O) and S(=O)₂ respectively; the 5- to 6-membered heteroaryl comprises 1, 2 or 3 heteroatoms selected from N, O and S; when multiple substituents are present, they are the same or different;
A is 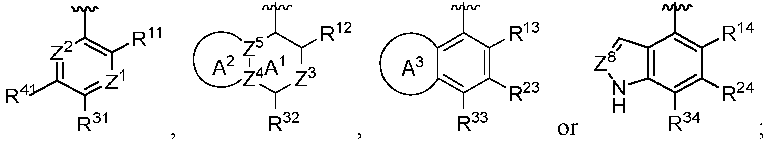
Z¹ is C(R²¹) or N; Z² is C(R⁵¹) or N; R⁴¹ is NH₂ or C(=O)NH₂;
ring A¹ is pyridinyl; wherein, Z³ is C(R²²) or N; Z⁴ and Z⁵ are independently C or N; (others are the shown C;)
R²¹, R²² and R⁵¹ are independently hydrogen;
ring A² is 5- to 6-membered heterocycloalkyl, 5- to 6-membered heterocycloalkenyl or 5- to 6-membered heteroaryl, or 5- to 6-membered heterocycloalkyl, 5- to 6-membered heterocycloalkenyl or 5- to 6-membered heteroaryl substituted with one or more R^{a1}; wherein the 5- to 6-membered heterocycloalkyl and the 5- to 6-membered heterocycloalkyl of the 5- to 6-membered heterocycloalkyl substituted with one or more R^{a1} comprise 1, 2 or 3 heteroatoms selected from N, O, S, S(=O) and S(=O)₂ respectively; the 5- to 6-membered heterocycloalkenyl and the 5- to 6-membered heterocycloalkenyl of the 5- to 6-membered heterocycloalkenyl substituted with one or more R^{a1} comprise 1, 2 or 3 heteroatoms selected from N, O, S, S(=O) and S(=O)₂ respectively; the 5- to 6-membered heteroaryl and the 5- to 6-membered heteroaryl of the 5- to 6-membered heteroaryl substituted with one or more R^{a1} comprise 1, 2 or 3 heteroatoms selected from N, O and S; when multiple substituents are present, they are the same or different;
ring A³ is 5- to 6-membered heterocycloalkyl, 5- to 6-membered heterocycloalkenyl, 6-membered heteroaryl or 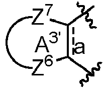 (end a indicates ortho-fusing to a benzene ring), or 5- to 6-membered heterocycloalkyl, 5- to 6-membered heterocycloalkenyl, 6-membered heteroaryl or 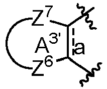 substituted with one or more R^{a2}; wherein the 5- to 6-membered heterocycloalkyl and the 5- to 6-membered heterocycloalkyl of the 5- to 6-membered heterocycloalkyl substituted with one or more R^{a2} comprise 1, 2 or 3 heteroatoms selected from N, O, S, S(=O) and S(=O)₂ respectively; the 5- to 6-membered heterocycloalkenyl and the 5- to 6-membered heterocycloalkenyl of the 5- to 6-membered heterocycloalkenyl substituted with one or more R^{a2} comprise 1, 2 or 3 heteroatoms selected from N, O, S, S(=O) and S(=O)₂ respectively; the 6-membered heteroaryl and in the 6-membered heteroaryl of the 5- to 6-membered heteroaryl substituted with one or more R^{a2} comprise 1, 2 or 3 heteroatoms selected from N, O and S; when multiple substituents are present, they are the same or different; ring A³ is ortho-fused to a benzene ring;
A^{3'} is 5-membered heteroaryl; wherein the 5-membered heteroaryl comprises 1 or 2 heteroatoms selected from N, O and S; and Z⁷ is N, O or S, and/or Z⁶ is CH, O or S;
R^{a1} and R^{a2} are independently hydroxy, =O, halogen, CN, C₁-C₄ alkyl or C₁-C₄ alkyl-O-;
R¹¹, R³¹, R¹², R³², R¹³ and R³³ are independently C₁-C₄ alkyl, C₁-C₄ alkyl-O- or 3- to 6-membered cycloalkyl;
Z⁸ is CH or N; R¹⁴ is C₁-C₄ alkyl-O-; R²³ and R²⁴ is H; R³⁴ is C₁-C₄ alkyl or 3- to 6-membered cycloalkyl;
the carbon atom with "*" means that when it is a chiral carbon atom, the compound has an S configuration or an R configuration, or a mixture thereof.

In certain preferred embodiments of the present disclosure, certain groups in the heterocyclic compound represented by formula I are defined as follows (the groups not mentioned are as described in any one of the embodiments of the present application),
W is C(R^{7'}R^{7"}).

In certain preferred embodiments of the present disclosure, certain groups in the heterocyclic compound represented by formula I are defined as follows (the groups not mentioned are as described in any one of the embodiments of the present application),
R^{7'} and R^{7"} are independently hydrogen, halogen, C₁-C₄ alkyl or C₁-C₄ alkyl-O-; for example, R^{7"} is independently hydrogen, C₁-C₄ alkyl -O- or halogen; R^{7'} is independently hydrogen or C₁-C₄ alkyl.

In certain preferred embodiments of the present disclosure, certain groups in the heterocyclic compound represented by formula I are defined as follows (the groups not mentioned are as described in any one of the embodiments of the present application),
R⁷ is hydrogen.

In certain preferred embodiments of the present disclosure, certain groups in the heterocyclic compound represented by formula I are defined as follows (the groups not mentioned are as described in any one of the embodiments of the present application),
m is 1.

In certain preferred embodiments of the present disclosure, certain groups in the heterocyclic compound represented by formula I are defined as follows (the groups not mentioned are as described in any one of the embodiments of the present application),
R^{b} is H.

In certain preferred embodiments of the present disclosure, certain groups in the heterocyclic compound represented by formula I are defined as follows (the groups not mentioned are as described in any one of the embodiments of the present application),
Z¹ is CH, and Z² is N; or Z¹ is CH, and Z² is CH; or Z¹ is N, and Z² is CH.

In certain preferred embodiments of the present disclosure, certain groups in the heterocyclic compound represented by formula I are defined as follows (the groups not mentioned are as described in any one of the embodiments of the present application),
Z⁴ is N, or Z⁵ is N.

In certain preferred embodiments of the present disclosure, certain groups in the heterocyclic compound represented by formula I are defined as follows (the groups not mentioned are as described in any one of the embodiments of the present application),
ring A² is 5- to 6-membered heteroaryl, e.g., 5-membered heteroaryl.

In certain preferred embodiments of the present disclosure, certain groups in the heterocyclic compound represented by formula I are defined as follows (the groups not mentioned are as described in any one of the embodiments of the present application),
ring A² is 5- to 6-membered heterocycloalkyl, 5- to 6-membered heterocycloalkenyl or 6-membered heteroaryl, or 5- to 6-membered cycloalkenyl, 5- to 6-membered heterocycloalkyl, 5-to 6-membered heterocycloalkenyl or 6-membered heteroaryl substituted with one or more R^{a1}. In certain preferred embodiments of the present disclosure, certain groups in the heterocyclic compound represented by formula I are defined as follows (the groups not mentioned are as described in any one of the embodiments of the present application),
ring A³ is 5- to 6-membered heterocycloalkyl, 5- to 6-membered heterocycloalkenyl or 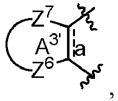 or 5- to 6-membered heterocycloalkyl, 5- to 6-membered heterocycloalkenyl, 6-membered heteroaryl or 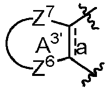 substituted with one or more R^{a2};
for example, 5-membered heterocycloalkyl, 5-membered heterocycloalkenyl or 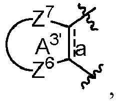 or 5-membered heterocycloalkyl, 5-membered heterocycloalkenyl or 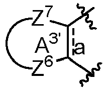 substituted with one or more R^{a2}.

In certain preferred embodiments of the present disclosure, certain groups in the heterocyclic compound represented by formula I are defined as follows (the groups not mentioned are as described in any one of the embodiments of the present application),
R^{a1} and R^{a2} are independently hydroxy, halogen, =O or C₁-C₄ alkyl, e.g., hydroxy, =O or C₁-C₄ alkyl.

In certain preferred embodiments of the present disclosure, certain groups in the heterocyclic compound represented by formula I are defined as follows (the groups not mentioned are as described in any one of the embodiments of the present application),
R¹¹, R¹² and R¹³ are independently C₁-C₄ alkyl or C₁-C₄ alkyl-O-, preferably C₁-C₄ alkyl-O-.

In certain preferred embodiments of the present disclosure, certain groups in the heterocyclic compound represented by formula I are defined as follows (the groups not mentioned are as described in any one of the embodiments of the present application),
R³¹, R³² and R³³ are independently C₁-C₄ alkyl.

In certain preferred embodiments of the present disclosure, certain groups in the heterocyclic compound represented by formula I are defined as follows (the groups not mentioned are as described in any one of the embodiments of the present application),
R¹⁴ is C₁-C₄ alkyl-O-.

In certain preferred embodiments of the present disclosure, certain groups in the heterocyclic compound represented by formula I are defined as follows (the groups not mentioned are as described in any one of the embodiments of the present application),
Z⁸ is N, and R³⁴ is C₁-C₄ alkyl; or Z⁸ is CH, and R³⁴ is 3- to 6-membered cycloalkyl.

In certain preferred embodiments of the present disclosure, certain groups in the heterocyclic compound represented by formula I are defined as follows (the groups not mentioned are as described in any one of the embodiments of the present application), and the heterocyclic compound represented by formula I is represented by formula Ia, Ib or Ic below: 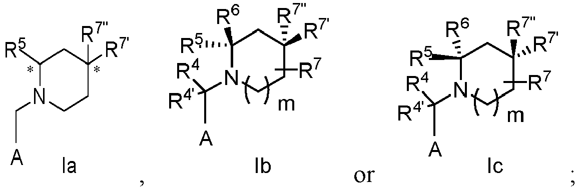 for example, R^{7"} is independently hydrogen, C₁-C₄ alkyl-O- or halogen; R^{7'} is independently hydrogen or C₁-C₄ alkyl; as another example,

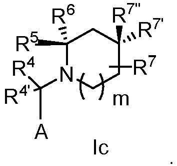

In certain preferred embodiments of the present disclosure, certain groups in the heterocyclic compound represented by formula I are defined as follows (the groups not mentioned are as described in any one of the embodiments of the present application),
W is C(R^{7'}R^{7"}); R⁷ is hydrogen;
R^{7'} and R^{7"} are independently hydrogen, halogen, C₁-C₄ alkyl or C₁-C₄ alkyl-O-; for example,
R^{7"} is independently hydrogen, C₁-C₄ alkyl -O- or halogen; R^{7'} is independently hydrogen or C₁-C₄ alkyl;
R⁶ is hydrogen;
R^{4'} and R⁴ are independently hydrogen;
m is 1;
R⁵ is 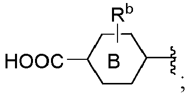 ring B is phenyl or 6-membered heteroaryl;
R^{b} is H, hydroxy, =O, or a group ortho-fused to ring B, wherein the group is selected from phenyl, 3- to 6-membered cycloalkyl, 5- to 6-membered heterocycloalkyl and 5- to 6-membered heteroaryl;
A is 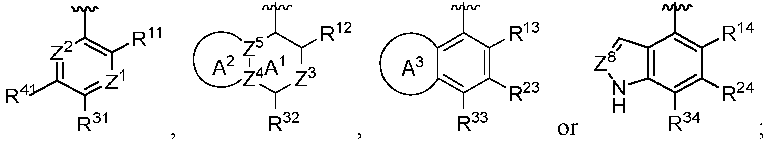
Z¹ is CH or N; Z² is CH or N; R⁴¹ is NH₂ or C(=O)NH₂;
ring A¹ is pyridinyl; Z³ is CH or N; Z⁴ and Z⁵ are independently C or N;
ring A² is 5- to 6-membered heterocycloalkyl, 5- to 6-membered heterocycloalkenyl or 5- to 6-membered heteroaryl, or 5- to 6-membered heterocycloalkyl, 5- to 6-membered heterocycloalkenyl or 5- to 6-membered heteroaryl substituted with one or more R^{a1};
ring A³ is 5- to 6-membered heterocycloalkyl, 5- to 6-membered heterocycloalkenyl, 6-membered heteroaryl or 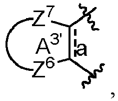 or 5- to 6-membered heterocycloalkyl, 5- to 6-membered heterocycloalkenyl, 6-membered heteroaryl or 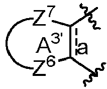 substituted with one or more R^{a2};
R^{a1} and R^{a2} are independently hydroxy, halogen, =O or C₁-C₄ alkyl, e.g., hydroxy, =O or C₁-C₄ alkyl;
R¹¹, R³¹, R¹², R³², R¹³, R²³ and R³³ are independently C₁-C₄ alkyl, C₁-C₄ alkyl-O- or 3- to 6-membered cycloalkyl;
Z⁸ is CH or N; R¹⁴ is C₁-C₄ alkyl-O-; R²³ and R²⁴ is H; R³⁴ is C₁-C₄ alkyl or 3- to 6-membered cycloalkyl;
the carbon atom with "*" means that when it is a chiral carbon atom, the compound has an S configuration or an R configuration, or a mixture thereof.

In certain preferred embodiments of the present disclosure, certain groups in the heterocyclic compound represented by formula I are defined as follows (the groups not mentioned are as described in any one of the embodiments of the present application),
W is C(R^{7'}R^{7"}); R⁷ is hydrogen;
R^{7'} and R^{7"} are independently hydrogen or C₁-C₄ alkyl-O-;
R⁶ is hydrogen;
R^{4'} and R⁴ are independently hydrogen;
m is 1;
R⁵ is 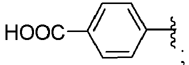
A is 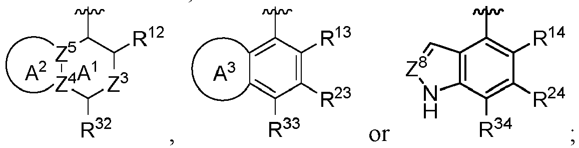
ring A¹ is pyridinyl; Z³ is N; Z⁴ and Z⁵ is C;
ring A² is 5-membered heteroaryl or 5-membered heteroaryl substituted with one or more R^{a1}, e.g., 5-membered heteroaryl;
ring A³ is 5- to 6-membered heterocycloalkyl, 5- to 6-membered heterocycloalkenyl, 6-membered heteroaryl or 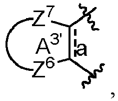 or 5- to 6-membered heterocycloalkyl, 5- to 6-membered heterocycloalkenyl, 6-membered heteroaryl or 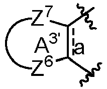 substituted with one or more R^{a2}, e.g., 5-membered heterocycloalkyl, 5-membered heterocycloalkenyl or 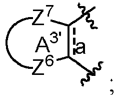
R^{a1} and R^{a2} are independently hydroxy, =O or C₁-C₄ alkyl, e.g., =O or C₁-C₄ alkyl;
R¹² and R¹³ are independently C₁-C₄ alkyl-O-;
R³² and R³³ are independently C₁-C₄ alkyl;
Z⁸ is CH or N; R¹⁴ is C₁-C₄ alkyl-O-; R²³ and R²⁴ is H; R³⁴ is C₁-C₄ alkyl or 3- to 6-membered cycloalkyl;
the carbon atom with "*" means that when it is a chiral carbon atom, the compound has an S configuration or an R configuration, or a mixture thereof.

In certain preferred embodiments of the present disclosure, certain groups in the heterocyclic compound represented by formula I are defined as follows (the groups not mentioned are as described in any one of the embodiments of the present application),
W is C(R^{7'}R^{7"}); R⁷ is hydrogen;
R^{7'} and R^{7"} are independently hydrogen or C₁-C₄ alkyl-O-;
R⁶ is hydrogen;
R^{4'} and R⁴ are independently hydrogen;
m is 1;
R⁵ is 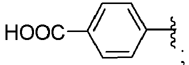
A is 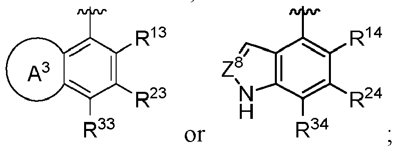
ring A³ is 5-membered heterocycloalkyl, 5-membered heterocycloalkenyl or 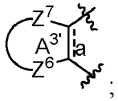
R¹³ is C₁-C₄ alkyl-O-;
R³³ is C₁-C₄ alkyl;
Z⁸ is CH or N; R¹⁴ is C₁-C₄ alkyl-O-; R²³ and R²⁴ is H; R³⁴ is C₁-C₄ alkyl or 3- to 6-membered cycloalkyl;
the carbon atom with "*" means that when it is a chiral carbon atom, the compound has an S configuration or an R configuration, or a mixture thereof.

In certain preferred embodiments of the present disclosure, certain groups in the heterocyclic compound represented by formula I are defined as follows (the groups not mentioned are as described in any one of the embodiments of the present application),
when R⁷, R^{7'} and R^{7"} are independently C₁-C₄ alkyl or C₁-C₄ alkyl-O-, the C₁-C₄ alkyl or the C₁-C₄ alkyl of the C₁-C₄ alkyl-O- is methyl, ethyl, n-propyl, isopropyl, n-butyl, isobutyl, sec-butyl or *tert-butyl,* e.g., methyl.

In certain preferred embodiments of the present disclosure, certain groups in the heterocyclic compound represented by formula I are defined as follows (the groups not mentioned are as described in any one of the embodiments of the present application),
when R⁶ is C₁-C₄ alkyl or hydroxy C₁-C₄ alkyl, the C₁-C₄ alkyl and the C₁-C₄ alkyl of the hydroxy C₁-C₄ alkyl is methyl, ethyl, *n*-propyl, isopropyl, *n*-butyl, isobutyl, *sec*-butyl or *tert*-butyl, e.g., methyl.

In certain preferred embodiments of the present disclosure, certain groups in the heterocyclic compound represented by formula I are defined as follows (the groups not mentioned are as described in any one of the embodiments of the present application),
when ring B is 6-membered heteroaryl, the 6-membered heteroaryl is pyridinyl, e.g., 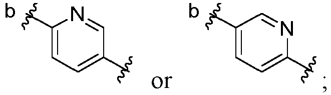 (end b indicates linking to COOH).

In certain preferred embodiments of the present disclosure, certain groups in the heterocyclic compound represented by formula I are defined as follows (the groups not mentioned are as described in any one of the embodiments of the present application),
when R^{b} is 3- to 6-membered cycloalkyl, the 3- to 6-membered cycloalkyl is cyclopropyl, cyclobutyl, cyclopentyl and cyclohexyl, e.g., cyclopentyl.

In certain preferred embodiments of the present disclosure, certain groups in the heterocyclic compound represented by formula I are defined as follows (the groups not mentioned are as described in any one of the embodiments of the present application),
when R^{b} is 5- to 6-membered heterocycloalkyl, the 5- to 6-membered heterocycloalkyl is tetrahydrofuranyl, e.g., 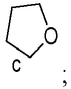 (end c indicates ortho-fusing to ring B).

In certain preferred embodiments of the present disclosure, certain groups in the heterocyclic compound represented by formula I are defined as follows (the groups not mentioned are as described in any one of the embodiments of the present application),
when R^{b} is 5- to 6-membered heteroaryl, the 5- to 6-membered heteroaryl is pyridinyl, e.g., 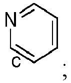 (end c indicates ortho-fusing to ring B) or imidazolyl (e.g., 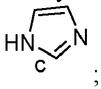 end c indicates ortho-fusing to ring B).

In certain preferred embodiments of the present disclosure, certain groups in the heterocyclic compound represented by formula I are defined as follows (the groups not mentioned are as described in any one of the embodiments of the present application),
when ring A² is 5- to 6-membered heterocycloalkyl, the 5- to 6-membered heterocycloalkyl is 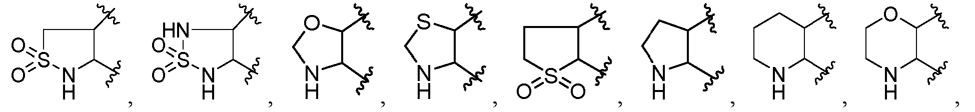 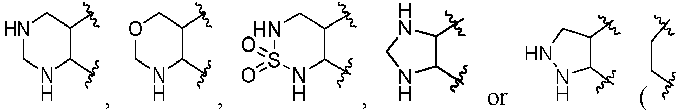 indicates a site for ortho-fusing to ring A¹).

In certain preferred embodiments of the present disclosure, certain groups in the heterocyclic compound represented by formula I are defined as follows (the groups not mentioned are as described in any one of the embodiments of the present application),
when ring A² is 5- to 6-membered heterocycloalkenyl, the 5- to 6-membered heterocycloalkenyl is 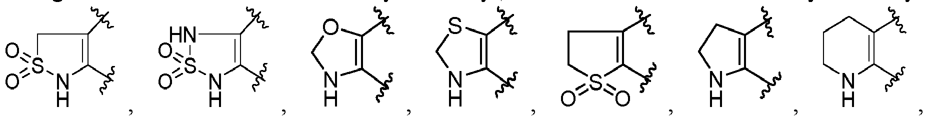 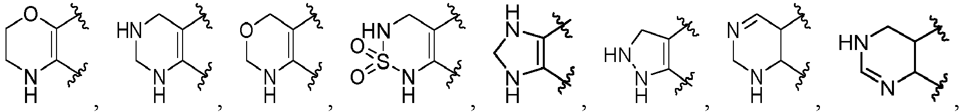 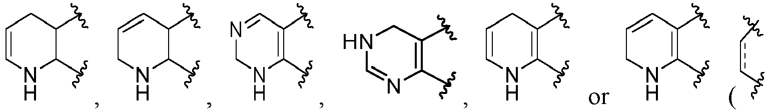 indicates a site for ortho-fusing to ring A¹, and 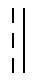 indicates a single bond or double bond).

In certain preferred embodiments of the present disclosure, certain groups in the heterocyclic compound represented by formula I are defined as follows (the groups not mentioned are as described in any one of the embodiments of the present application),
when ring A² is 5- to 6-membered heteroaryl, the 5- to 6-membered heteroaryl is

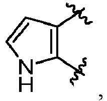

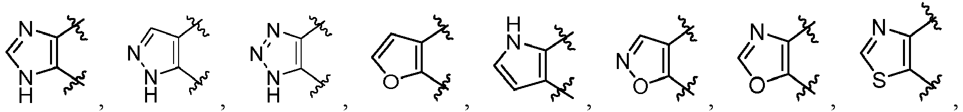

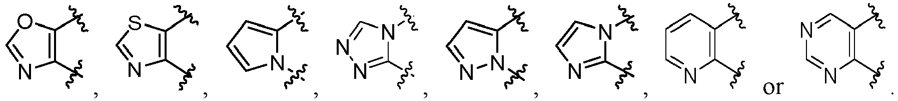

In certain preferred embodiments of the present disclosure, certain groups in the heterocyclic compound represented by formula I are defined as follows (the groups not mentioned are as described in any one of the embodiments of the present application),
when ring A³ is 5- to 6-membered heterocycloalkyl, the 5- to 6-membered heterocycloalkyl is 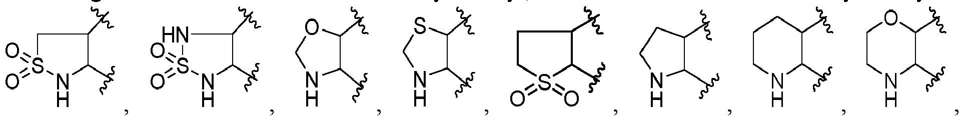 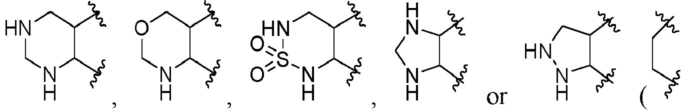 indicates a site for ortho-fusing to ring A¹).

In certain preferred embodiments of the present disclosure, certain groups in the heterocyclic compound represented by formula I are defined as follows (the groups not mentioned are as described in any one of the embodiments of the present application),
when ring A³ is 5- to 6-membered heterocycloalkenyl, the 5- to 6-membered heterocycloalkenyl is 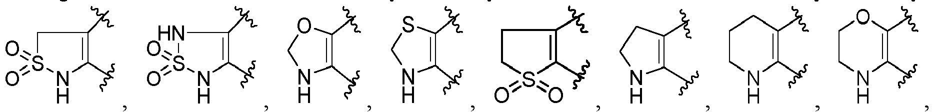 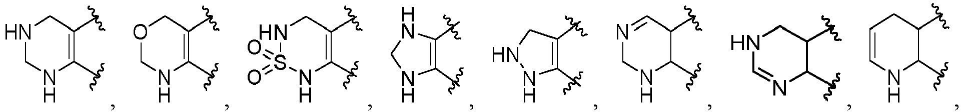 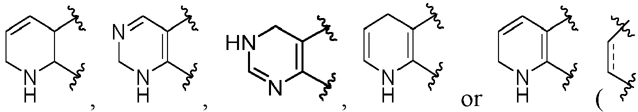 indicates a site for ortho-fusing to ring A¹, and 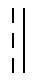 indicates a single bond or double bond).

In certain preferred embodiments of the present disclosure, certain groups in the heterocyclic compound represented by formula I are defined as follows (the groups not mentioned are as described in any one of the embodiments of the present application),
when ring A³ is 6-membered heteroaryl, the 6-membered heteroaryl is

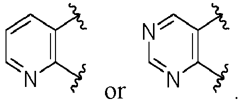

In certain preferred embodiments of the present disclosure, certain groups in the heterocyclic compound represented by formula I are defined as follows (the groups not mentioned are as described in any one of the embodiments of the present application),
when ring A³ is 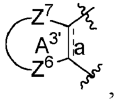 the A^{3'} is

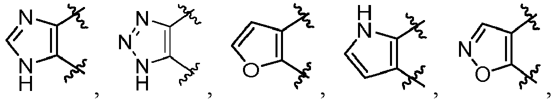

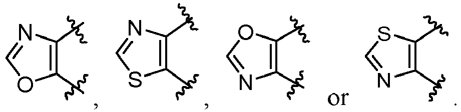

In certain preferred embodiments of the present disclosure, certain groups in the heterocyclic compound represented by formula I are defined as follows (the groups not mentioned are as described in any one of the embodiments of the present application),
when R^{a1} and R^{a2} are independently C₁-C₄ alkyl or C₁-C₄ alkyl-O-, the C₁-C₄ alkyl or the C₁-C₄ alkyl of the C₁-C₄ alkyl-O- is methyl, ethyl, *n*-propyl, isopropyl, *n*-butyl, isobutyl, *sec*-butyl or *tert*-butyl, e.g., methyl.

In certain preferred embodiments of the present disclosure, certain groups in the heterocyclic compound represented by formula I are defined as follows (the groups not mentioned are as described in any one of the embodiments of the present application),
when R¹¹, R³¹, R¹², R³², R¹³, R²³ and R³³ are independently C₁-C₄ alkyl or C₁-C₄ alkyl-O-, the C₁-C₄ alkyl and the C₁-C₄ alkyl of the C₁-C₄ alkyl-O- is methyl, ethyl, n-propyl, isopropyl, *n-*butyl, isobutyl, *sec*-butyl or *tert*-butyl, e.g., methyl.

In certain preferred embodiments of the present disclosure, certain groups in the heterocyclic compound represented by formula I are defined as follows (the groups not mentioned are as described in any one of the embodiments of the present application),
when R¹¹, R³¹, R¹², R³², R¹³, R²³ and R³³ are independently 3- to 6-membered cycloalkyl, the 3-to 6-membered cycloalkyl is cyclopropyl, cyclobutyl, cyclopentyl and cyclohexyl, e.g., cyclopropyl.

In certain preferred embodiments of the present disclosure, certain groups in the heterocyclic compound represented by formula I are defined as follows (the groups not mentioned are as described in any one of the embodiments of the present application),
when R¹⁴ is C₁-C₄ alkyl-O-, the C₁-C₄ alkyl of the C₁-C₄ alkyl-O- is methyl, ethyl, *n*-propyl, isopropyl, *n*-butyl, isobutyl, *sec*-butyl or *tert*-butyl, e.g., methyl.

In certain preferred embodiments of the present disclosure, certain groups in the heterocyclic compound represented by formula I are defined as follows (the groups not mentioned are as described in any one of the embodiments of the present application),
when R³⁴ is C₁-C₄ alkyl, the C₁-C₄ alkyl is methyl, ethyl, *n*-propyl, isopropyl, *n*-butyl, isobutyl, *sec*-butyl or *tert*-butyl, e.g., methyl.

In certain preferred embodiments of the present disclosure, certain groups in the heterocyclic compound represented by formula I are defined as follows (the groups not mentioned are as described in any one of the embodiments of the present application),
when R³⁴ is 3- to 6-membered cycloalkyl, the 3- to 6-membered cycloalkyl is cyclopropyl, cyclobutyl, cyclopentyl and cyclohexyl, e.g., cyclopropyl.

In certain preferred embodiments of the present disclosure, certain groups in the heterocyclic compound represented by formula I are defined as follows (the groups not mentioned are as described in any one of the embodiments of the present application),
R^{7'} and R^{7"} are independently hydrogen, F, methyl or ethyl-O-.

In certain preferred embodiments of the present disclosure, certain groups in the heterocyclic compound represented by formula I are defined as follows (the groups not mentioned are as described in any one of the embodiments of the present application),
R⁵ is 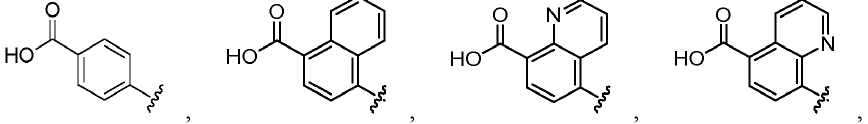 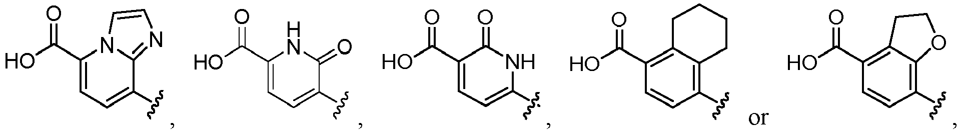 e.g.,

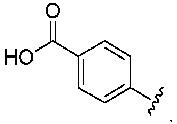

In certain preferred embodiments of the present disclosure, certain groups in the heterocyclic compound represented by formula I are defined as follows (the groups not mentioned are as described in any one of the embodiments of the present application),
R^{a1} and R^{a2} are independently hydroxy, =O or methyl.

In certain preferred embodiments of the present disclosure, certain groups in the heterocyclic compound represented by formula I are defined as follows (the groups not mentioned are as described in any one of the embodiments of the present application),
R¹¹, R³¹, R¹², R³², R¹³ and R³³ are independently methyl, methyl-O- or cyclopropyl;
for example, R¹¹, R¹² and R¹³ are independently methyl, methyl-O- or cyclopropyl;
R³¹, R³² and R³³ are independently methyl.

In certain preferred embodiments of the present disclosure, certain groups in the heterocyclic compound represented by formula I are defined as follows (the groups not mentioned are as described in any one of the embodiments of the present application),
R¹⁴ is methyl-O-.

In certain preferred embodiments of the present disclosure, certain groups in the heterocyclic compound represented by formula I are defined as follows (the groups not mentioned are as described in any one of the embodiments of the present application),
R³⁴ is methyl or cyclopropyl.

In certain preferred embodiments of the present disclosure, certain groups in the heterocyclic compound represented by formula I are defined as follows (the groups not mentioned are as described in any one of the embodiments of the present application),
Wis 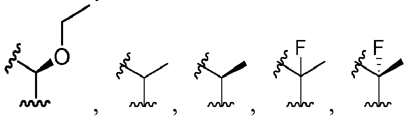 or methylene, e.g.,

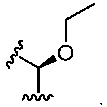

In certain preferred embodiments of the present disclosure, certain groups in the heterocyclic compound represented by formula I are defined as follows (the groups not mentioned are as described in any one of the embodiments of the present application), 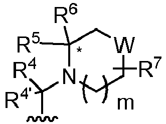 is

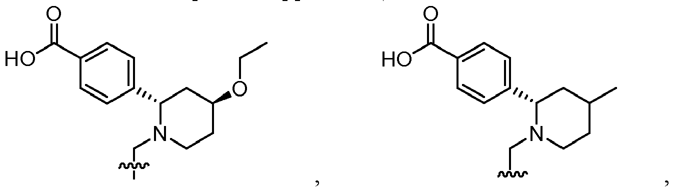

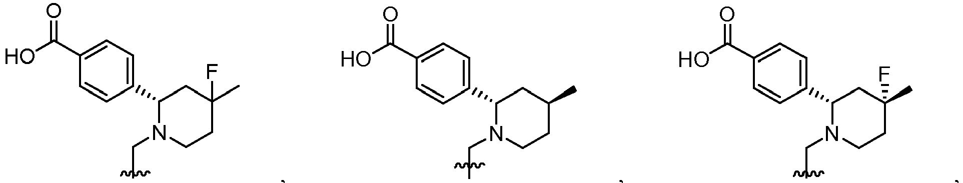

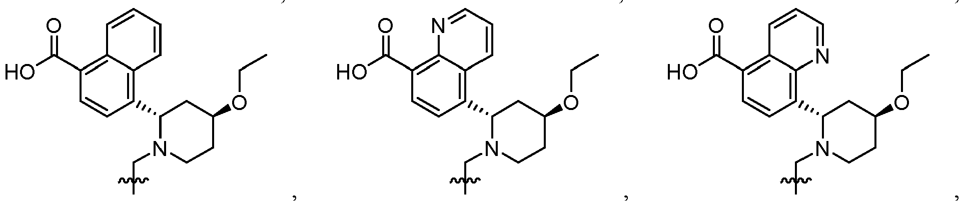

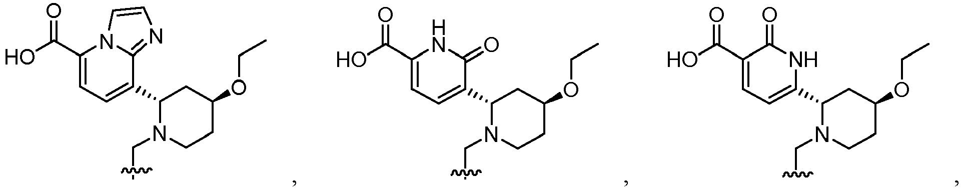

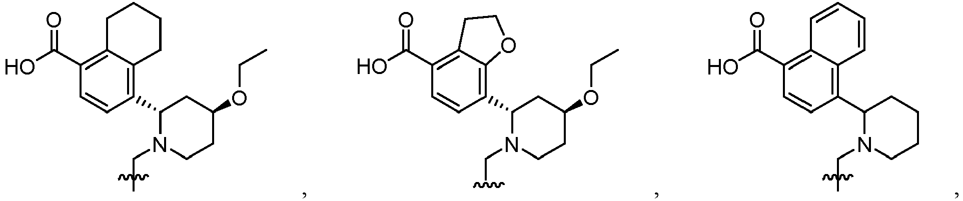

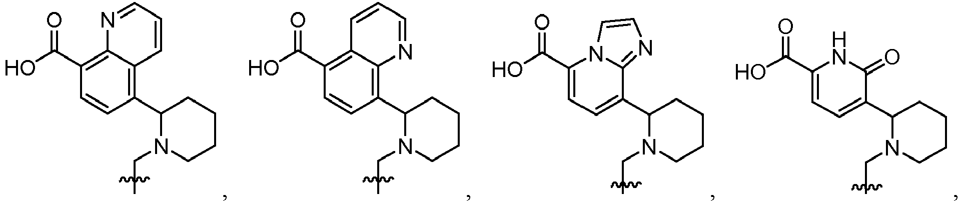

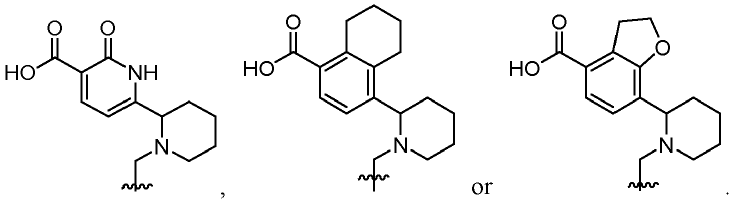

In certain preferred embodiments of the present disclosure, certain groups in the heterocyclic compound represented by formula I are defined as follows (the groups not mentioned are as described in any one of the embodiments of the present application).
when A is 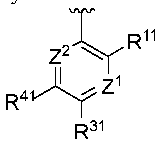 , e.g.,

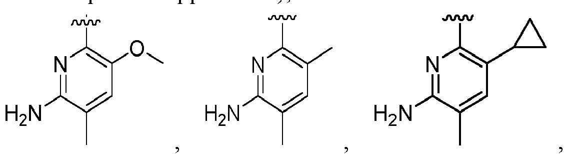

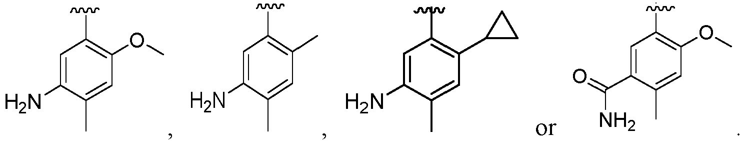

In certain preferred embodiments of the present disclosure, certain groups in the heterocyclic compound represented by formula I are defined as follows (the groups not mentioned are as described in any one of the embodiments of the present application),
when A is 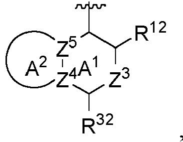 e.g., 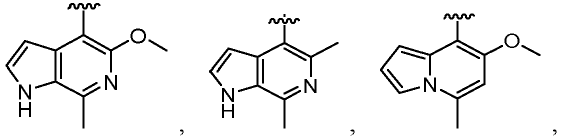 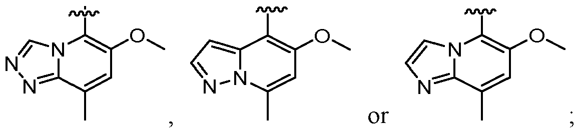 ; e.g.,

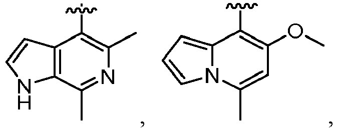

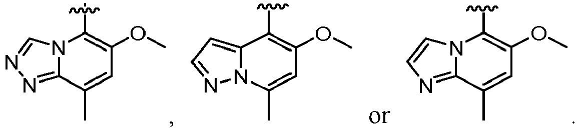

In certain preferred embodiments of the present disclosure, certain groups in the heterocyclic compound represented by formula I are defined as follows (the groups not mentioned are as described in any one of the embodiments of the present application),
when A is 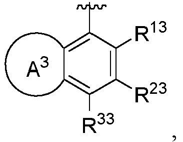 e.g., 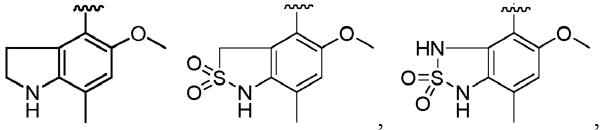 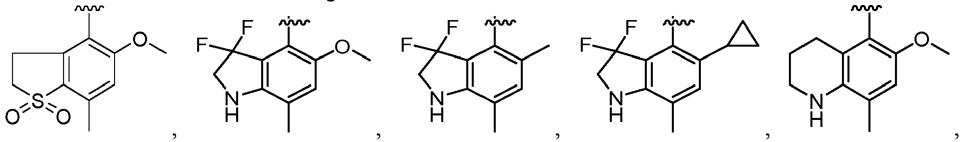 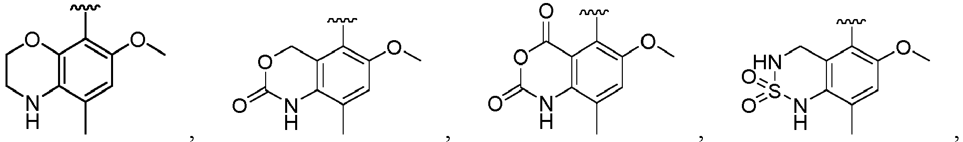 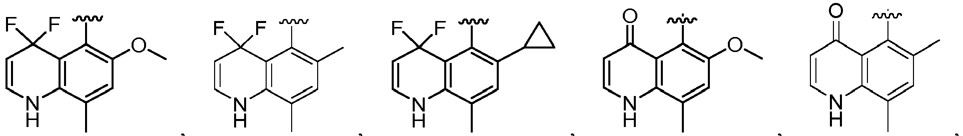 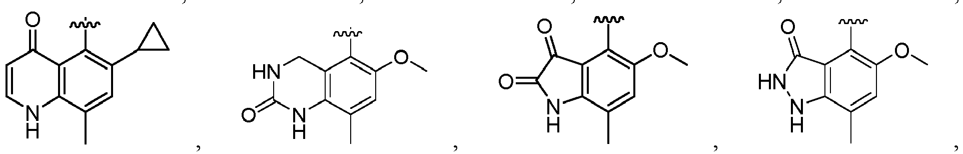 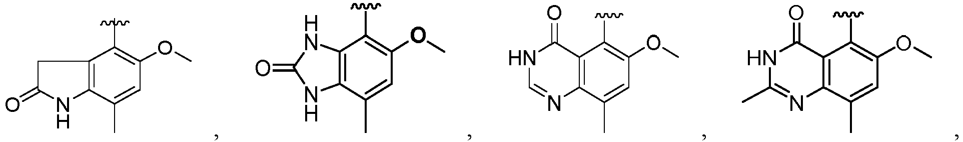 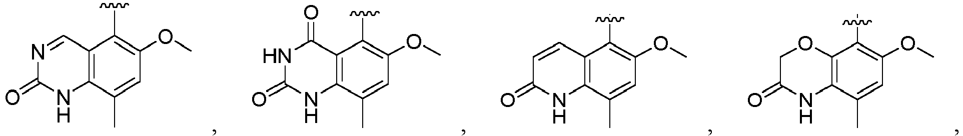 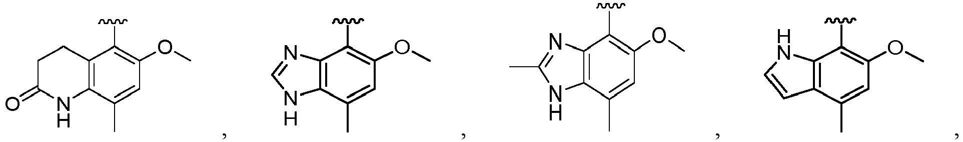 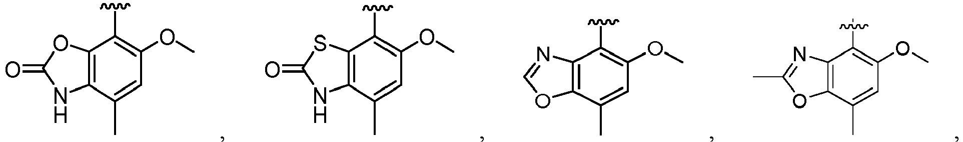 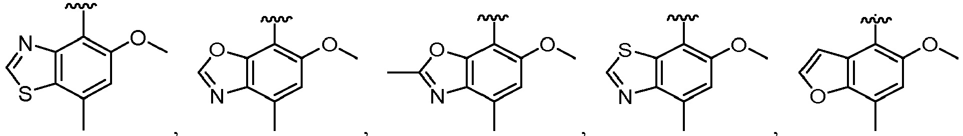 or 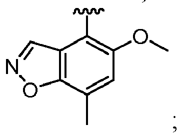 e.g.,

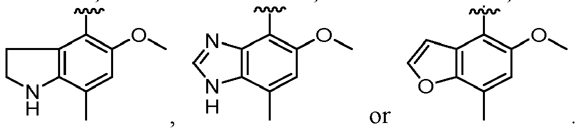

In certain preferred embodiments of the present disclosure, certain groups in the heterocyclic compound represented by formula I are defined as follows (the groups not mentioned are as described in any one of the embodiments of the present application),
when A is 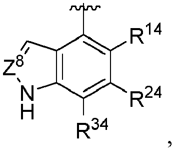 e.g.,

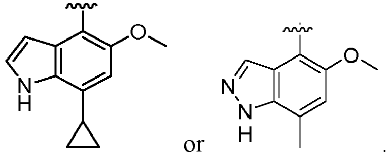

In certain preferred embodiments of the present disclosure, the heterocyclic compound represented by formula I is any one of the following structures,

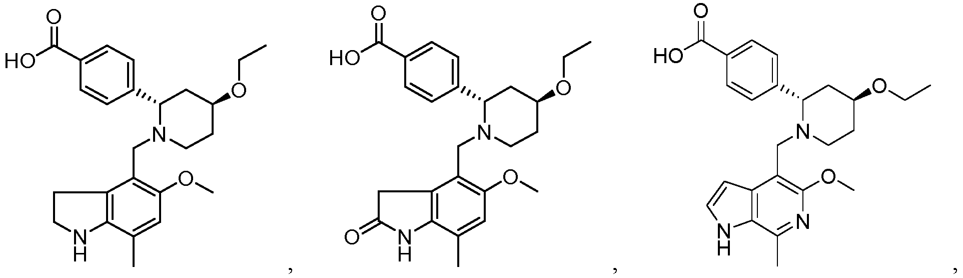

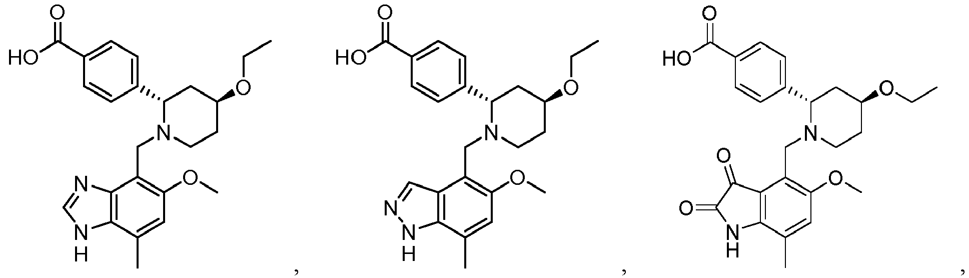

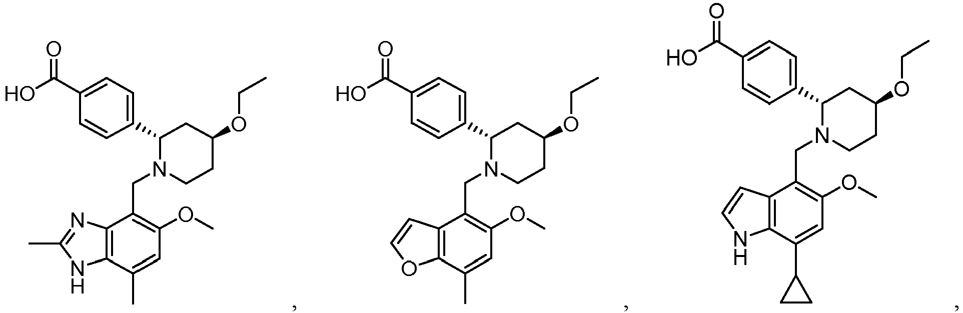

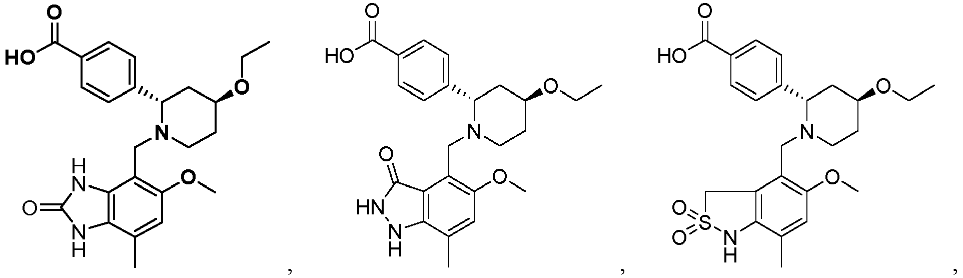

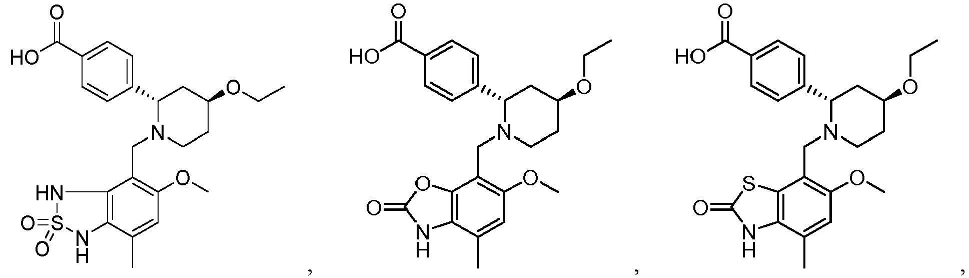

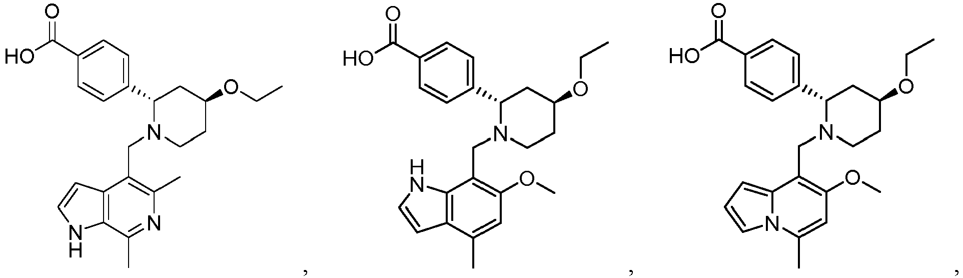

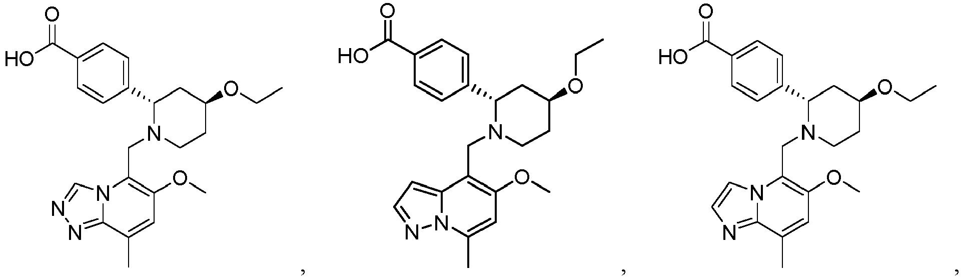

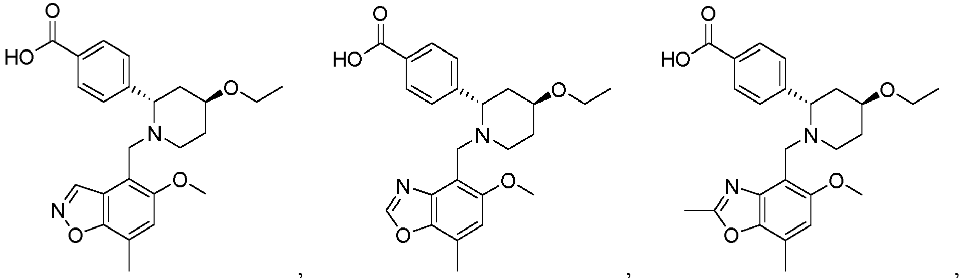

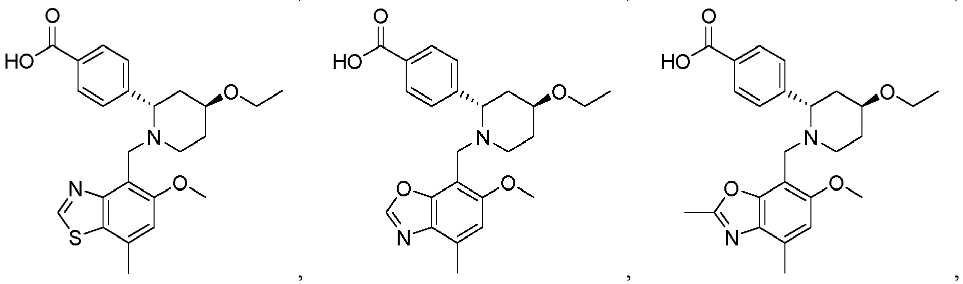

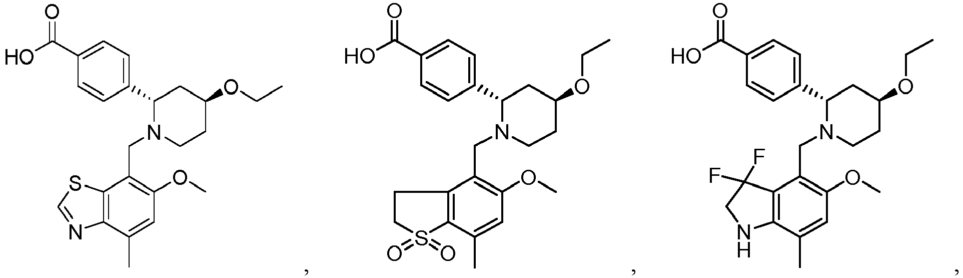

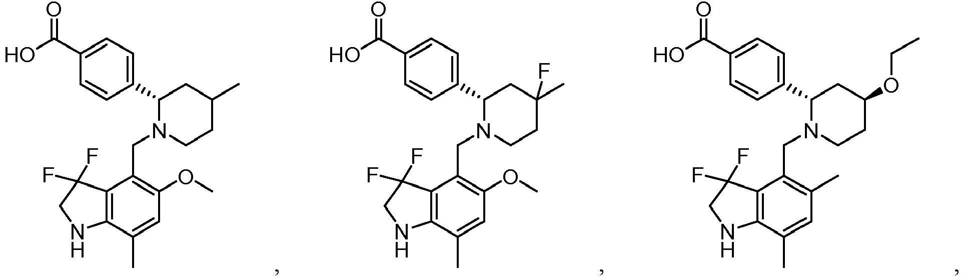

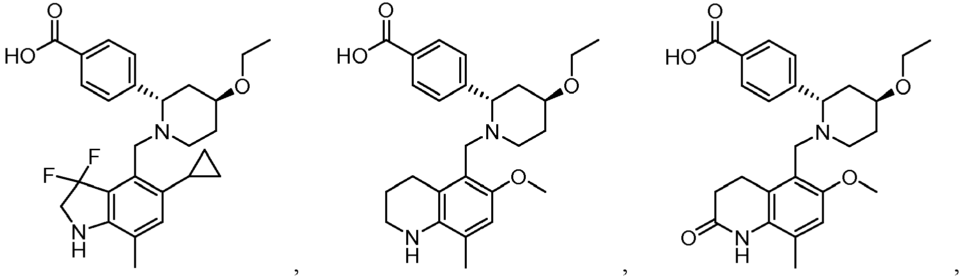

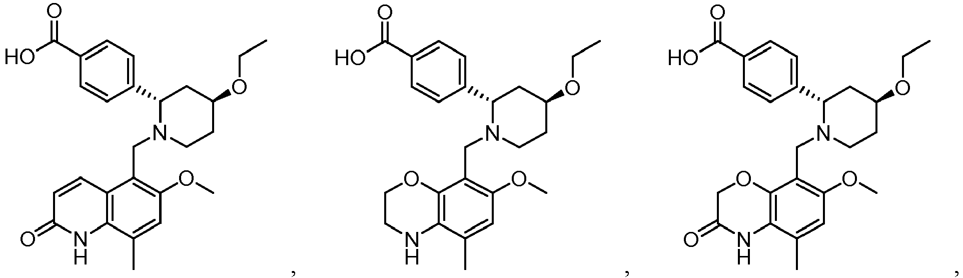

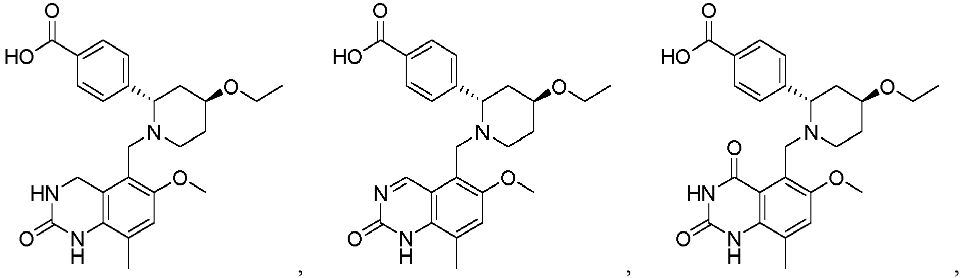

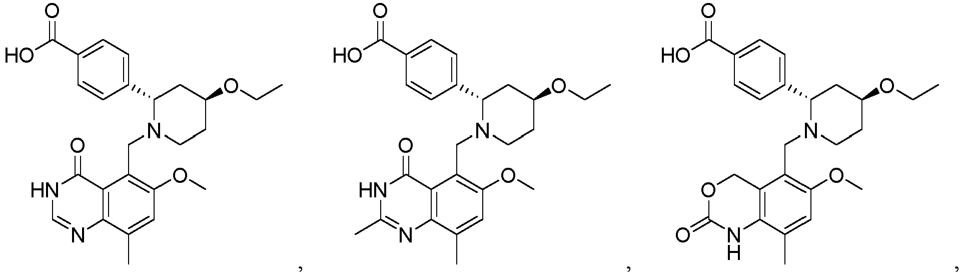

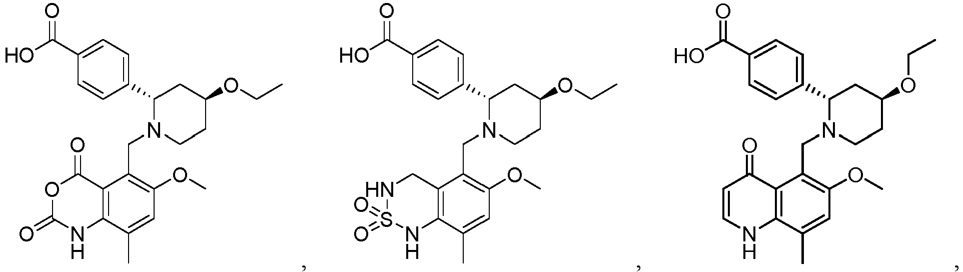

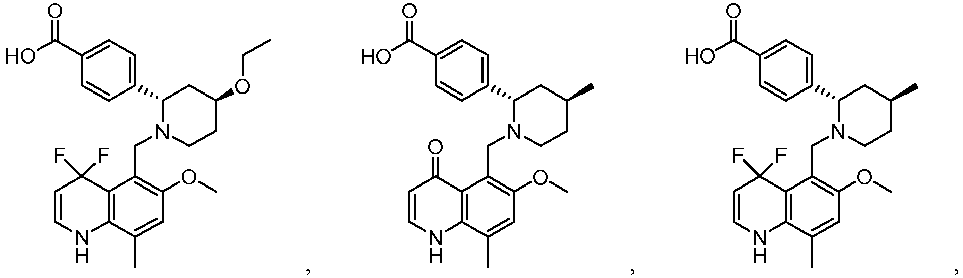

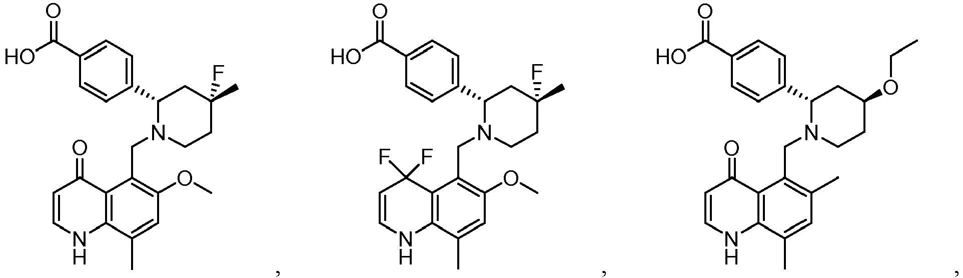

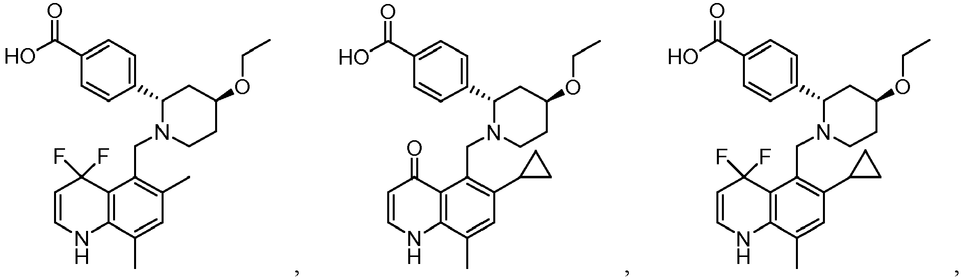

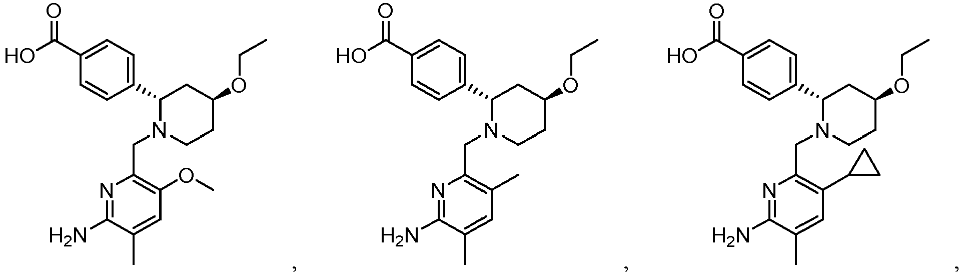

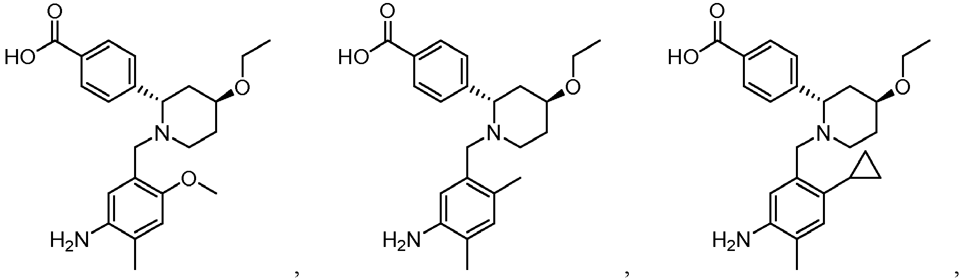

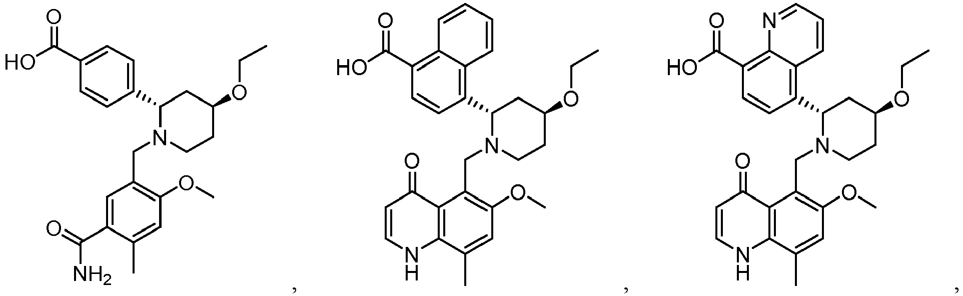

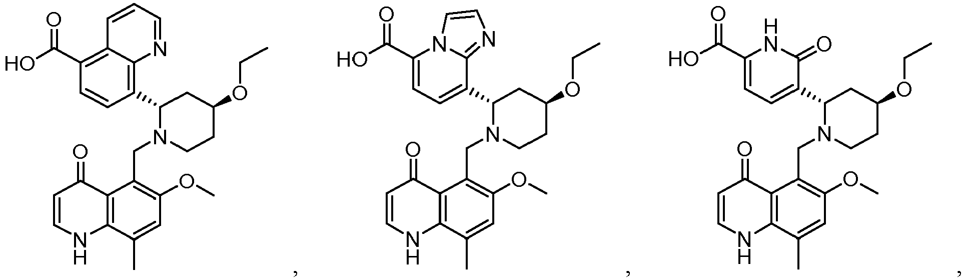

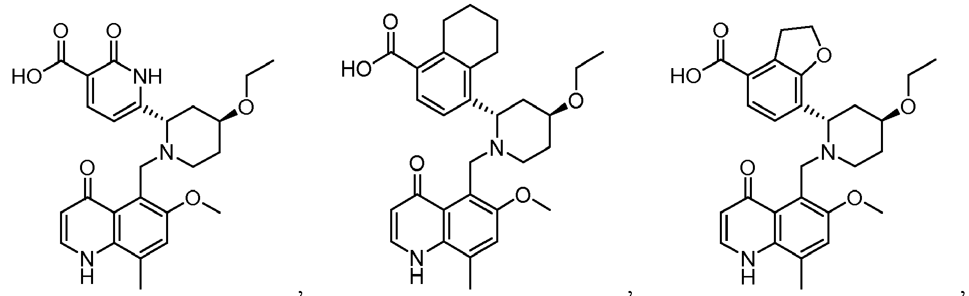

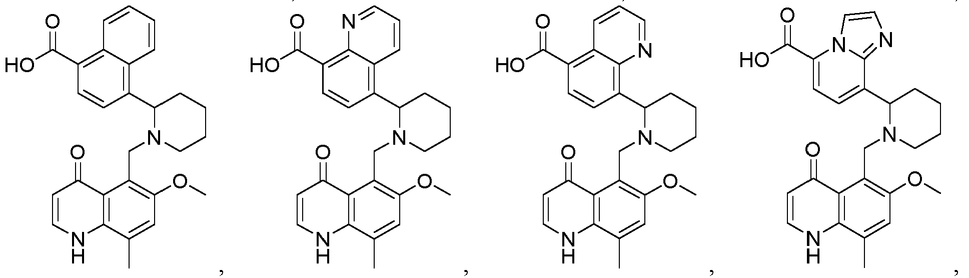

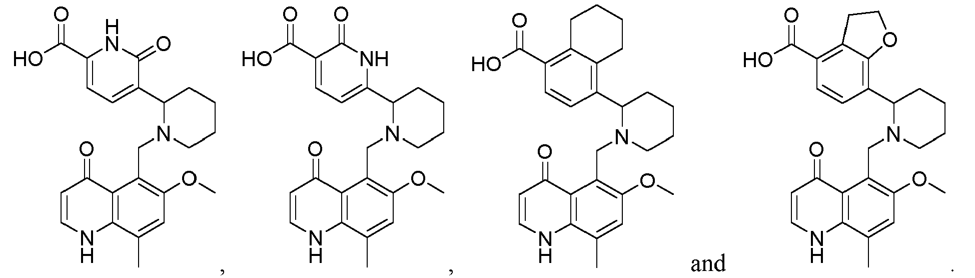

In certain preferred embodiments of the present disclosure, the pharmaceutically acceptable salt of the heterocyclic compound represented by formula I is any one of the following structures, 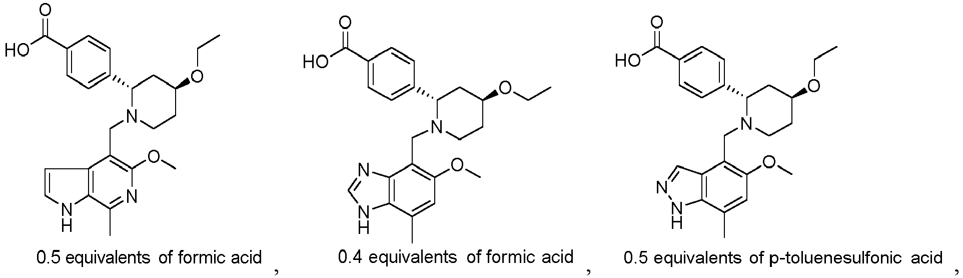 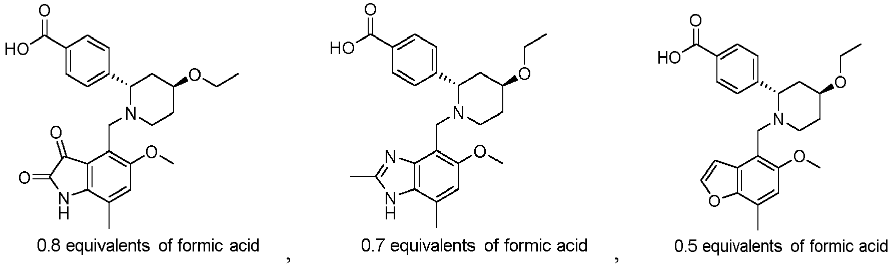 and

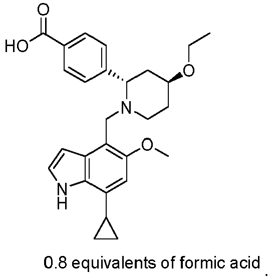

In the present disclosure, the heterocyclic compound represented by formula I or the pharmaceutically acceptable salt, the isotopic analog and the prodrug thereof have one or more chiral carbon atoms, so that optically pure isomers, such as pure enantiomers, racemates or mixed isomers, can be obtained by separation. Pure single isomers can be obtained using separation methods in the art, such as chiral crystallization to form salts, or chiral preparative column separation.

In the present disclosure, if the heterocyclic compound represented by formula I or the pharmaceutically acceptable salt, the isotopic analog or the prodrug thereof has stereoisomeric forms, it may be present in the form of single stereoisomeric forms or mixtures thereof (such as racemates). The term "stereoisomer" refers to a *cis-trans* isomer or an optical isomer. These stereoisomers can be separated, purified and enriched using asymmetric synthesis methods or chiral resolution (including, but not limited to, thin layer chromatography, rotary chromatography, column chromatography, gas chromatography, high pressure liquid chromatography, etc.), and can also be obtained by chiral resolution by bonding (chemical bonding, etc.) or salt formation (physical bonding, etc.) with other chiral compounds. The term "single stereoisomer" means that the content by mass of one stereoisomer of the compound of the present disclosure with respect to all stereoisomers of the compound is not less than 95%. In the present disclosure, if the heterocyclic compound represented by formula I or the pharmaceutically acceptable salt, the isotopic analog or the prodrug thereof has tautomeric forms, it may be present in the form of single tautomers or mixtures thereof, preferably be present mainly in the form of relatively stable tautomers. For example, when the following structural fragments are contained:

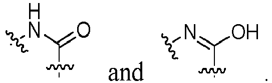

In the present disclosure, the heterocyclic compound represented by formula I or the pharmaceutically acceptable salt, the isotopic analog or the prodrug thereof can be synthesized using methods similar to those known in the chemical field in which reference can be made to the steps and conditions of similar reactions in the art, especially synthesized according to the description herein. The starting materials are generally from commercial sources (e.g., Aldrich) or can be readily prepared using methods well known to those skilled in the art (obtained by SciFinder and Reaxys online databases).

In the present disclosure, the other heterocyclic compounds represented by formula I or the pharmaceutically acceptable salts thereof can also be obtained using conventional methods in the art by peripherally modifying the prepared heterocyclic compound represented by formula I or the pharmaceutically acceptable salt, the isotopic analog or the prodrug thereof.

The necessary starting materials or reagents for preparing the heterocyclic compound represented by formula I or the pharmaceutically acceptable salt, the isotopic analog or the prodrug thereof are commercially available or can be prepared using synthesis methods known in the art. The compounds of the present disclosure, either as a free base or as its acid addition salt, can be prepared using the methods described in the experimental section below. The term pharmaceutically acceptable salt refers to a pharmaceutically acceptable salt defined herein and has all the effects of the parent compound. The pharmaceutically acceptable salt can be prepared by adding the corresponding acid to a suitable organic solvent of an organic base and treating according to conventional methods.

The present disclosure also provides a preparation method for the heterocyclic compound represented by formula I, which comprises the following step:
subjecting a compound represented by formula II to a de-esterification reaction as shown below in a solvent in the presence of a base to give the heterocyclic compound represented by formula I: 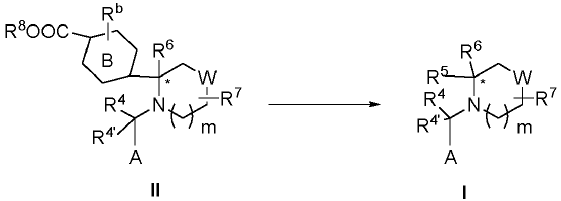 wherein R⁸ is C₁-C₄ alkyl (e.g., methyl, ethyl, *n*-propyl, isopropyl, *n*-butyl, isobutyl, *sec*-butyl or *tert*-butyl; e.g., methyl or ethyl); R^{b}, R⁴, R^{4'}, R⁶, R⁷, A, W, m and * are as defined above.

In the preparation method for the heterocyclic compound represented by formula I, the conditions and operation for the de-esterification reaction can be conventional conditions and operation for such reactions in the art, and, in the present disclosure, are preferably the following: the solvent can be water and/or an alcohol solvent (e.g., methanol and/or ethanol), e.g., water and an alcohol solvent.

The solvent is used in such an amount that the reaction is not affected. For example, the compound represented by formula II and the solvent are in a mass-volume ratio of 1 g/L to 20 g/L (e.g., 15 g/L to 20 g/L).

The base may be an alkali metal hydroxide, such as sodium hydroxide and/or potassium hydroxide.

The de-esterification reaction may be performed at a temperature of 0-100 °C (e.g., 70-80 °C). The progress of the de-esterification reaction may be monitored using conventional monitoring methods known in the art (e.g., TLC, HPLC or NMR). Generally, the reaction comes to an end when the compound represented by formula II disappears or is no longer reacted.

The present disclosure also provides a heterocyclic compound represented by formula II as described above, 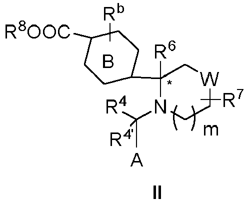 wherein R⁸ is C₁-C₄ alkyl (e.g., methyl, ethyl, *n*-propyl, isopropyl, *n*-butyl, isobutyl, *sec*-butyl or *tert*-butyl; e.g., methyl or ethyl); R^{b}, R^{4'}, R^{4'}, R⁶, R⁷, A, W, m and * are as defined above.

In a certain embodiment of the present disclosure, the heterocyclic compound represented by formula I has the following structure: 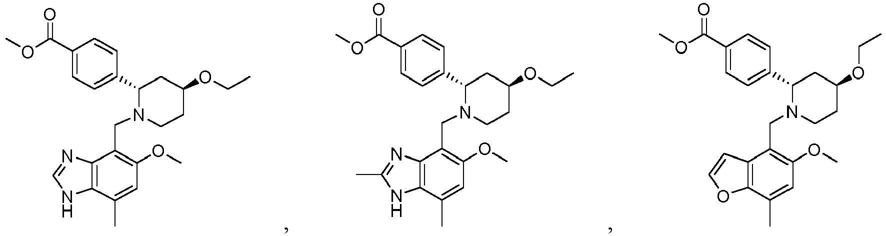 and

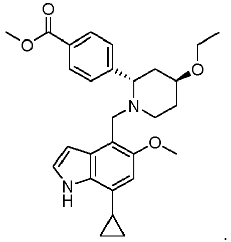

The present disclosure provides a pharmaceutical composition comprising the heterocyclic compound represented by formula I or the pharmaceutically acceptable salt, the isotopic analog or the prodrug thereof described above, and one or more pharmaceutically acceptable carriers. In the pharmaceutical composition, the amount of the heterocyclic compound represented by formula I or the pharmaceutically acceptable salt, the isotopic analog or the prodrug thereof can be a therapeutically effective amount.

The pharmaceutically acceptable carriers (pharmaceutically acceptable auxiliary materials) can be those excipients widely used in the field of pharmaceutical production. The auxiliary materials are primarily used to provide a safe, stable and functional pharmaceutical composition and may also provide a method for dissolving the active ingredients at a desired rate or for promoting effective absorption of the active ingredients after administration of the composition to a subject. The pharmaceutically acceptable auxiliary materials may be inert fillers or provide a function such as stabilizing the overall pH of the composition or preventing degradation of the active ingredients of the composition. The pharmaceutically acceptable auxiliary materials may comprise one or more of the following excipients: adhesives, suspending agents, emulsifiers, diluents, fillers, granulating agents, gluing agents, disintegrants, lubricants, anti-adherents, glidants, wetting agents, gelling agents, absorption retardants, dissolution inhibitors, reinforcing agents, adsorbents, buffering agents, chelating agents, preservatives, colorants, flavoring agents and sweeteners.

The pharmaceutical composition disclosed herein may be prepared in accordance with the disclosure using any method known to those skilled in the art, for example, conventional mixing, dissolving, granulating, emulsifying, levigating, encapsulating, embedding or lyophilizing processes.

The pharmaceutical composition of the present disclosure can be administered in any form, including injection (intravenous), mucosal, oral (solid and liquid formulations), inhalation, ocular, rectal, topical or parenteral (infusion, injection, implantation, subcutaneous, intravenous, intraarterial, intramuscular) administration. The pharmaceutical composition of the present disclosure can also be in controlled-release or delayed-release dosage form (e.g., liposomes or microspheres). Examples of solid oral formulations comprise, but are not limited to, powders, capsules, caplets, soft capsules and tablets. Examples of liquid formulations for oral or mucosal administration comprise, but are not limited to, suspensions, emulsions, elixirs and solutions. Examples of topical formulations comprise, but are not limited to, emulsions, gels, ointments, creams, patches, pastes, foams, lotions, drops or serum formulations. Examples of formulations for parenteral administration comprise, but are not limited to, solutions for injection, dry formulations that can be dissolved or suspended in a pharmaceutically acceptable carrier, suspensions for injection, and emulsions for injection. Examples of other suitable formulations of the pharmaceutical composition comprise, but are not limited to, eye drops and other ophthalmic formulations, aerosols such as nasal sprays or inhalants, liquid dosage forms suitable for parenteral administration, suppositories and lozenges.

An object of the present disclosure is to provide the heterocyclic compound represented by formula I or the pharmaceutically acceptable salt, the isotopic analog or the prodrug thereof described above for use as a complement factor B inhibitor.

In the use, the complement factor B inhibitor can be used in a mammalian organism; it can also be used *in vitro,* mainly for experimental purposes, for example: as a standard sample or a control sample for comparison, or be prepared into a kit according to conventional methods in the art for providing a quick assay for the inhibitory effect against complement factor B.

An object of the present disclosure is to provide use of the heterocyclic compound represented by formula I or the pharmaceutically acceptable salt, the isotopic analog or the prodrug thereof described above for the manufacturing of a medicament; the medicament can be used for treating a disease associated with abnormal activation of complement factor B or occur in normal functioning of complement factor B; or, the medicament can be used for treating blood, autoimmune, inflammatory and neurodegeneration diseases and the like. The heterocyclic compound represented by formula I or the pharmaceutically acceptable salt, the isotopic analog or the prodrug thereof can be used in a therapeutically effective amount.

An object of the present disclosure is to provide use of the heterocyclic compound represented by formula I or the pharmaceutically acceptable salt, the isotopic analog or the prodrug thereof described above for treating a disease associated with abnormal activation of the complement system or occur in normal functioning of the complement system.

An object of the present disclosure is to provide a method for treating a disease associated with abnormal activation of the complement system or occur in normal functioning of the complement system, comprising administering to a patient an effective dose of the heterocyclic compound represented by formula I or the pharmaceutically acceptable salt, the isotopic analog or the prodrug thereof described above.

The disease associated with abnormal activation of the complement system or occur in normal functioning of the complement system may be a disease selected from blood, autoimmune, inflammatory and neurodegeneration diseases and the like. The disease comprises, but are not limited to: paroxysmal nocturnal hemoglobinuria (PNH), primary glomerulonephritis (IgAN), membranous nephropathy (MN), C3 glomerulonephritis (C3G), age-related macular degeneration (AMD), geographic atrophy (GA), atypical hemolytic uremic syndrome (aHUS), hemolytic uremic syndrome (HUS), hemodialysis complications, hemolytic anemia or hemodialysis, neuromyelitis optica (NMO), arthritis, rheumatoid arthritis, liver-related inflammations, dermatomyositis and amyotrophic lateral sclerosis, myasthenia gravis (MG), respiratory disease, cardiovascular disease and the like.

The complement system is preferably one associated with or regulated by complement factor B. When used as a medicament, the heterocyclic compound represented by formula I or the pharmaceutically acceptable salt, the isotopic analog or the prodrug thereof can be administered in the form of a pharmaceutical composition. These compositions can be prepared using methods well known in the art of pharmacy and can be administered through a variety of routes, depending on the topical or systemic treatment desired and the area to be treated. Administration may be topical (including epidermal and transdermal administration, and ocular and mucosal administration, including intranasal, vaginal and rectal delivery), pulmonary (e.g., inhalation or insufflation of powders or aerosols, including using a nebulizer; intratracheal or intranasal administration), oral or parenteral. Oral administration may comprise dosage forms formulated for once-daily or twice-daily (BID) administration. Parenteral administration comprises intravenous administration, intraarterial administration, subcutaneous administration, intraperitoneal administration, intramuscular administration or injection or infusion; or intracranial administration, e.g., intrathecal or intraventricular administration. Parenteral administration may be in a single bolus dosage form, or may be performed using a continuous infusion pump. Pharmaceutical compositions and formulations for topical administration may comprise transdermal patches, salves, lotions, ointments, gels, drops, suppositories, sprays, liquids and powders. Conventional pharmaceutical carriers, water, powdered or oily bases and thickeners and the like may be necessary or desirable.

As used herein, the term "treatment" refers to a therapeutic or palliative measure. Beneficial or desired clinical outcomes comprise, but are not limited to: completely or partially alleviated symptoms associated with the disease or disorder or condition, reduced extent of the disease, a stable (not worsening) condition of illness, delayed or slowed progression of the disease, an ameliorated or alleviated condition of illness (e.g., one or more symptoms of the disease), and detectable or undetectable amelioration (whether partial or complete). "Treatment" may also refer to prolonging survival relative to the expected survival of not having treatment.

In certain embodiments, the heterocyclic compound represented by formula I or the pharmaceutically acceptable salt, the isotopic analog or the prodrug thereof or the pharmaceutical composition described above can be used to prevent the a disease or condition defined herein (e.g., blood, autoimmune, inflammatory, neurodegeneration disease and the like). As used herein, the term "prevention" means complete or partial prevention of the disease or condition defined herein or the occurrence, recurrence or spread of the symptoms thereof.

As used herein, the term "prodrug" represents a compound that is converted *in vivo* to the compound represented by formula I. Such conversion is affected by hydrolysis of the prodrug in the blood or by enzymatic conversion of the prodrug into the parent structure in the blood or tissue. The prodrugs disclosed herein can be esters, and in the prior art, the esters that can be used as prodrugs comprise phenyl esters, aliphatic (C1-24) esters, acyloxymethyl esters, carbonates, carbamates and amino acid esters. For example, a compound disclosed herein containing hydroxy can be acylated to give a prodrug. Other prodrugs comprise phosphate esters, and those phosphate esters are obtained by phosphorylating via the hydroxy on the parent structure. For a complete discussion of prodrugs, reference can be made to the following: T. Higuchiand V. Stella, Pro-drugs as Novel Delivery Systems, Vol. 14 of the A.C.S. Symposium Series*;* Edward B. Roche, ed., Bioreversible Carriersin Drug Design, American Pharmaceutical Associationand Pergamon Press, 1987; J. Rautioetal., Prodrugs: Designand Clinical Applications, Nature Review Drug Discovery, 2008, 7, 255-270; and S. J. Heckeretal., Prodrugs of Phosphate sand Phosphonates, Journal of Medicinal Chemistry, 2008, 51, 2328-2345.

The compound of formula I and the salt thereof are intended to encompass any isotopically labeled (or "radiolabeled") derivative of the compound of formula I and the salt thereof. Such derivatives are derivatives of a compound of formula I or a salt thereof shown below, wherein one or more atoms are replaced with an atom whose atomic mass or mass number is different from that usually found in nature. The radionuclide used will depend on the particular application of the radiolabeled derivative. For example, ³H or ¹⁴C is often useful for *in vitro* receptor labeling and competition assays. For radiographic application, ¹¹C or ¹⁸F is often useful.

Particular isotopic variants of the compound of the present disclosure, particularly those into which one or more radioactive isotopes have been incorporated, may be useful, for example, in investigating the mechanism of action or the distribution of the active component in the body; compounds labelled with ³H or ¹⁴C isotopes are particularly suitable for this purpose due to their relative ease of preparation and detectability. In addition, the incorporation of isotopes such as deuterium may afford particular therapeutic benefits as the compound has greater metabolic stability, for example, an increased *in vivo* half-life or a reduction in the effective dosage required; such modifications to the compound of the present disclosure may therefore also constitute preferred embodiments of the present disclosure in some cases. Isotopic variants of the compound of the present disclosure can be prepared using methods known to those skilled in the art, for example, using the methods described below and the methods described in the operation examples, or using the corresponding isotopically modified specific reagents and/or starting compounds.

The term "pharmaceutical auxiliary material" or "excipient" refers to a pharmaceutically acceptable chemical, e.g., an agent known to those of ordinary skill in the pharmaceutical art to aid in the administration of a drug. It is a compound that can be used to prepare pharmaceutical compositions. It is generally safe, non-toxic, and biologically or otherwise undesirable. It comprises excipients that are pharmaceutically acceptable for veterinary use and for human use. Typical excipients comprise adhesives, surfactants, diluents, disintegrants and lubricants. Unless otherwise stated, the following definitions as used herein should be applied. For the purpose of the present invention, the chemical elements are consistent with the Periodic Table of Elements (CAS version) and Handbook of Chemistry and Physics (75th Edition, 1994). In addition, general principles of organic chemistry can be found in Organic Chemistry, Thomas Sorrell, University Science Books, Sausalito: 1999, and March's Advanced Organic Chemistry by Michael B. Smith and Jerry March, John Wiley & Sons, New York: 2007, which are incorporated herein by reference in their entirety.

In the present specification, groups and substituents thereof can be selected by those skilled in the art to provide stable moieties and compounds. When a substituent is described by a general formula written from left to right, the substituent also comprises chemically equivalent substituents that are obtained when the structural formula is written from right to left.

Certain chemical groups defined herein are preceded by a shorthand notation to indicate the total number of carbon atoms present in the groups. For example, C₁-C₆ alkyl refers to an alkyl group as defined below containing a total of 1, 2, 3, 4, 5, or 6 carbon atoms. The total number of carbon atoms in the shorthand notation does not comprise carbon atoms that may be present in a substituent of the group.

Numerical ranges defined herein in substituents, such as 0 to 4, 1-4, 1 to 3, etc., indicate integers within the range; for example, 1-6 indicates 1, 2, 3, 4, 5 and 6.

In addition to the above description, when used in the specification and claims of the present application, the following terms have the meanings shown below, unless otherwise specified.

The term "one or more" or "one or two or more" means 1, 2, 3, 4, 5, 6, 7, 8, 9 or more.

The term "comprise", "comprises" or "including" is open-ended, i.e. including what is meant by the present disclosure, but not excluding other aspects.

The term "substituted" means that one or more hydrogen atoms on a specific atom are substituted by substituents, including deuterium and hydrogen variants, as long as the valence of the specific atom is normal and the substituted compound is stable.

In general, the term "substituted" means that one or more hydrogen atoms in a given structure are substituted with a particular substituent. Further, when the group is substituted with one or more substituents described above, the substituents are independent of each other, that is, the one or more substituents may be different or the same. Unless otherwise indicated, the substitution of a substituent may occur at various substitutable positions of the substituted group. When more than one position in a given structural formula can be substituted with one or more substituents selected from particular groups, the substitution of the substituents may occur at various positions, identically or differently.

In each part of this specification, substituents for the disclosed compounds are disclosed according to group types or ranges. It is specifically noted that each separate sub-combination of the various members of these group types and ranges is encompassed in the present disclosure. The term "Cₓ-C_{y} alkyl" refers to a linear or branched saturated hydrocarbon containing x to y carbon atoms. For example, the term "C₁-C₆ alkyl" or "C₁₋₆ alkyl" specifically refers to the independently disclosed methyl, ethyl, C₃ alkyl, C₄ alkyl, C₅ alkyl, and C₆ alkyl; "C₁₋₄ alkyl" specifically refers to the independently disclosed methyl, ethyl, C₃ alkyl (i.e., propyl, including *n*-propyl and isopropyl), and C₄ alkyl (i.e., butyl, including *n*-butyl, isobutyl, *sec*-butyl, and *tert-*butyl).

The term "halogen" is selected from F, Cl, Br and I, and particularly refers to F or Cl.

The term "alkoxy" refers to the group -O-R^{X}, wherein R^{X} is alkyl as defined above.

The terms "moiety", "structural moiety", "chemical moiety", "group" and "chemical group", as used herein, refer to a particular fragment or functional group in a molecule. A chemical moiety is generally considered to be a chemical entity that is embedded in or attached to a molecule.

When the substituents listed are not indicated by which atom they are attached to the compounds comprised in the general chemical structural formula but not specifically mentioned, such substituents may be bonded through any atom thereof. A combination of a substituent and/or a variant thereof is permissible only if the combination can result in a stable compound.

When no substituent is explicitly indicated in the listed groups, such groups are simply unsubstituted. For example, when "C₁-C₄ alkyl" is not defined as "substituted or unsubstituted", it refers only to the "C₁-C₄ alkyl" itself or "unsubstituted C₁-C₄ alkyl".

In each part of the present disclosure, connecting substituents are described. When a linking group is clearly required to a structure, the Markush variables listed for the group should be understood as a linking group. For example, if a linking group is required to the structure and "alkyl" are listed for the Markush group definition of the variable, it should be understood that the "alkyl" represents a linked alkylene group.

In some specific structures, when an alkyl group is explicitly indicated as a linking group, the alkyl group represents a linked alkylene group, e.g., C₁-C₆ alkyl in the group "halogenated C₁-C₆ alkyl" should be considered as C₁-C₆ alkylene.

The term "alkylene" refers to a saturated divalent hydrocarbon radical resulting from the removal of two hydrogen atoms from a linear or branched saturated hydrocarbon radical. Examples of alkylene groups comprise methylene (-CH₂-), ethylidene (including -CH₂CH₂- or -CH(CH₃)-), isopropylene (including -CH(CH₃)CH₂- or -C(CH₃)₂-), and the like.

In the present application, as a group or part of another group (e.g., used in groups such as halogen-substituted alkyl and the like), the term "alkyl" is intended to comprise branched and linear saturated aliphatic hydrocarbon groups containing a specified number of carbon atoms, for example, a linear or branched saturated hydrocarbon chain containing 1 to 20 carbon atoms, or C₁-C₆ alkyl, as another example. For example, "C₁-C₆ alkyl" is defined to comprise groups containing 1, 2, 3, 4, 5 or 6 carbon atoms in a linear or branched structure. Propyl is a C₃ alkyl group (including isomers such as *n*-propyl or isopropyl). Butyl is a C₄ alkyl group (including isomers such as *n*-butyl, *sec*-butyl, isobutyl or *tert*-butyl). Pentyl is a C₅ alkyl group (including isomers such as *n*-pentyl, 1-methyl-butyl, 1-ethyl-propyl, 2-methyl-1-butyl, 3-methyl-1-butyl, isopentyl, *tert*-pentyl or neopentyl). Hexyl is a C₆ alkyl group (including isomers such as *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl or 2,3-dimethylbutyl).

The term "cycloalkyl" refers to a saturated monocyclic or polycyclic carbocyclic substituent consisting of only carbon atoms and hydrogen atoms, which can be linked to the rest of the molecule by single bonds via any suitable carbon atom; when it is polycyclic, it may be a ortho-fused system, bridged ring system or spiro ring system with ortho-fusing, ring bridging or spiro ring linking (i.e., two geminal hydrogen atoms on a carbon atom are substituted with alkylene). In a certain embodiment, typical monocyclic cycloalkyl is, for example, cyclopropyl, cyclobutyl, cyclopentyl, cyclohexyl or cycloheptyl.

In the present application, the term "cycloalkenyl" by itself or as part of another substituent refers to a monocyclic, polycyclic or bridged carbocyclic substituent containing a partially unsaturated double bond, which may be linked to the rest of the molecule by single bonds via any suitable carbon atom; when it is polycyclic, it may be a bridged ring system or a spiro ring system with ortho-fusing or spiro ring linking (i.e., two geminal hydrogen atoms on a carbon atom are substituted with alkylene). In some embodiments, "cycloalkenyl" is a monocyclic unsaturated carbocyclic alkenyl group containing 5 to 6 ring atoms ("5- to 6-membered cycloalkenyl"). The term comprises, but is not limited to, cyclopentenyl (e.g., 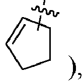 cyclopentadienyl (e.g., 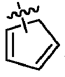 ), cyclohexenyl (e.g., 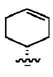 ) or cyclohexadienyl, as well as stereoisomers thereof.

The term "heterocycloalkyl" refers to a heteroatom-containing saturated cyclic group, a stable 3- to 10-membered saturated heterocyclic ring system containing 1 or more heteroatoms independently selected from N, O, S, S(=O) and S(=O)₂ with the remainder being carbon atoms. Unless otherwise specified in the specification, a heterocycloalkyl group may be a monocyclic system ("monocyclic heterocycloalkyl"), or a bicyclic or tricyclic system, or a ring system containing more rings, which may comprise ortho-fused (fused), bridged (of bridged rings) or spiro ring systems (e.g., bicyclic systems ("bicyclic heterocycloalkyl")). The bicyclic heterocycloalkyl system may comprise one or more heteroatoms in one or two rings, and is saturated. In some embodiments, "heterocycloalkyl" is 5- to 6-membered heterocycloalkyl. Exemplary 3-membered heterocyclyl groups comprise, but are not limited to, aziridinyl, oxiranyl and thiiranyl, or stereoisomers thereof. Exemplary 4-membered heterocyclyl groups comprise, but are not limited to, azetidinyl, oxetanyl, thietanyl, or isomers and stereoisomers thereof. Exemplary 5-membered heterocyclyl groups comprise, but are not limited to, tetrahydrofuranyl, tetrahydrothienyl, pyrrolidinyl, thiazolidinyl, isothiazolidinyl, oxazolidinyl, isoxazolidinyl, imidazolidinyl, pyrazolidinyl, dioxolanyl, oxathiafuranyl, dithiofuranyl, or isomers and stereoisomers thereof. Exemplary 6-membered heterocyclyl groups comprise, but are not limited to, piperidyl, tetrahydropyranyl, sulfocyclopentyl, morpholinyl, thiomorpholinyl, dithianyl, dioxanyl, piperazinyl, triazinyl, or isomers and stereoisomers thereof.

In the present application, the term "heterocycloalkenyl" by itself or as part of another substituent refers to a cyclic alkenyl group linked via a heteroatom or heteroatom group, unless otherwise specified. Thus, "heterocycloalkenyl" encompasses the definitions of "hetero" and cycloalkenyl described above. In some embodiments, in one certain embodiment, the "heterocycloalkenyl" is a stable, 3- to 10-membered, unsaturated, double bond-containing, heterocyclic ring system group consisting of 2-9 carbon atoms and 1, 2, 3 or 4 heteroatoms selected from N, O, S, S(=O) and S(=O)₂ or heteroatom-containing groups. Unless otherwise specified in the specification, a heterocycloalkenyl group may be a monocyclic system ("monocyclic heterocycloalkenyl"), or a bicyclic or tricyclic system, or a ring system containing more rings, which may comprise fused, bridged or spiro ring systems (e.g., bicyclic systems ("bicyclic heterocycloalkenyl"). The bicyclic heterocycloalkenyl system may comprise one or more heteroatoms in one or two rings.) In addition, it contains an unsaturated double bond. Heterocycloalkenyl can be linked to the rest of the molecule via a carbon atom and by a single bond. In a heterocycloalkenyl group containing one or more nitrogen atoms, the linking site may be a carbon or nitrogen atom; or it is ortho-fused to the rest of the molecule, so long as the valency permits.

The term "aryl" refers to an all-carbon aromatic group containing a fully conjugated π-electron system, which may be monocyclic or fused cyclic, and generally contains 6-14 carbon atoms, preferably 6-12 carbon atoms, and most preferably 6 carbon atoms. Examples of aryl comprise, but are not limited to, phenyl, naphthyl and anthracenyl.

The term "heteroaryl" refers to a heteroatom-containing aromatic group that may be monocyclic or fused cyclic, preferably 5- to 12-membered heteroaryl containing 1-4 heteroatoms independently selected from N, O and S, including but not limited to pyrrolyl, furanyl, thienyl, indolyl, imidazolyl, oxazolyl, isoxazolyl, pyrazolyl, pyridinyl, pyrimidinyl, pyrazinyl, pyridazinyl, quinolyl, isoquinolyl, (benzo)oxazolyl, (benzo)furanyl, (benzo)thienyl, (benzo)thiazolyl and triazolyl. In a certain embodiment, it is typically 5- to 6-membered monocyclic heteroaryl containing 1 or more heteroatoms independently selected from N, O and S. In a certain embodiment, "heteroaryl" is 5- to 6-membered heteroaryl comprising 1, 2 or 3 heteroatoms selected from N, O and S.

Unless otherwise stated, all the technical and scientific terms used herein have the standard meaning in the art to which the claimed subject matter belongs. If there are multiple definitions for a term, the definition given herein prevails.

It should be understood that the singular forms used herein such as "a" and "an" encompass plural references unless otherwise stated. In addition, the term "comprise", "comprises" or "including" is open-ended instead of closed-ended, i.e. including what is meant by the present disclosure, but not excluding other aspects.

Unless otherwise stated, the conventional methods-mass spectrometry and elemental analysis-are employed in the present disclosure. For steps and conditions, reference can be made to those conventional in the art.

Unless otherwise indicated, standard nomenclature for analytical chemistry, organic synthetic chemistry, and optics, and standard laboratory procedures and techniques are employed in the present disclosure. In some cases, standard techniques are used for chemical synthesis, chemical analysis and light-emitting device performance testing.

In addition, it should be noted that, unless otherwise explicitly indicated, the description of "... is independently" used herein is to be understood broadly and means that each individual group described is independent from the others and may be independently the same or different specific groups. In more detail, the description of "... is independently" can mean that the specific options expressed by the same symbols in different groups do not affect each other; it can also mean that the specific options expressed by the same symbols in the same group do not affect each other. As will be understood by those skilled in the art, " 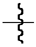 " used in the structural formulas describing groups herein means that the corresponding groups are connected with other fragments and groups in the compound through this site, according to conventions used in the art.

It will be understood by those skilled in the art that the "-̅ -̅ -̅" used in the structural formulas describing groups herein represents a single or double bond, according to conventions used in the art.

The preferred conditions described above may be combined arbitrarily to obtain preferred embodiments of the present disclosure without departing from the general knowledge in the art. The reagents and starting materials used in the present disclosure are commercially available. The positive progress effect of the present disclosure is that: the compound of the present disclosure has inhibitory activity against complement factor B (in the complement hemolytic activity experiment, the IC₅₀ values of the inhibitory activity against complement factor B are all less than 5 **µ**M, most of the IC₅₀ values are less than 1.5 **µ**M, and some of the IC₅₀ values are less than 1 **µ**M).

### DETAILED DESCRIPTION

The present disclosure is further illustrated by the following examples; however, these examples should not be construed as limiting the present disclosure. Experimental procedures without specified conditions in the following examples are conducted in accordance with conventional procedures and conditions, or in accordance with the manufacturer's manual.

The structures of the compounds were determined by nuclear magnetic resonance (NMR) and/or mass spectrometry (MS). The NMR shifts (δ) were given in 10⁻⁶ (ppm). The NMR measurement was performed using Bruker ASCEND^{™}-400 NMR spectrometer, with deuterated dimethyl sulfoxide (DMSO-d6), deuterated chloroform (CDCl₃) and deuterated methanol (CD₃OD) as solvents and tetramethylsilane (TMS) as an internal standard.

The MS measurement was performed using Agilent 6110, Agilent 1100, Agilent 6120 and AgilentG6125B liquid chromatography-mass spectrometers.

The HPLC measurement was performed using Shimadzu HPLC-2010C high-pressure liquid chromatograph (XBRIDGE 2.1× 50 mm, 3.5 µm column).

The chiral HPLC analysis was performed using THARSFC X5.

Yantai Qingdao GF254 silica gel plates were used as thin-layer chromatography silica gel plates: 0.15 mm-0.2 mm silica gel plates were used for thin-layer chromatography (TLC) analysis, and 0.4 mm-0.5 mm silica gel plates for thin-layer chromatography product separation and purification.

Qingdao Haiyang 200-300 mesh silica gel was generally used as the carrier in column chromatography.

Waters 2767, Waters 2545 and a Chuangxintongheng LC3000 preparative chromatograph were used in preparative high performance liquid chromatography.

Shimadzu LC-20AP and THARSFC PREP 80 were used in chiral preparative column chromatography.

A Beijing Jiawei Kechuang Technology GCD-500G hydrogen generator was used in pressurized hydrogenation reactions.

A Biotage initiator+ microwave reactor was used in microwave reactions.

In the examples, the reactions were all carried out under argon atmosphere or nitrogen atmosphere unless otherwise specified.

The argon atmosphere or nitrogen atmosphere means that the reaction flask was connected to a balloon with about 1 liter of argon or nitrogen gas.

The hydrogen atmosphere means that the reaction flask was connected to a balloon with about 1 liter of hydrogen gas.

In the experimental examples, the reaction temperature was at room temperature and ranged from 20 °C to 30 °C unless otherwise specified.

### Reagent Names Corresponding to English Abbreviations of the Reagents

| **English Abbreviations of Reagents** | **Reagent Names** |
|---|---|
| Na (AcO)₃BH | Sodium triacetoxyborohydride |
| DCE | 1,2-Dichloroethane |
| NaCNBH₃ | Sodium cyanoborohydride |
| AcOH | Acetic acid |
| NaOH | Sodium hydroxide |
| MeOH | Methanol |
| NBS | Bromosuccinimide |
| Cs₂CO₃ | Cesium carbonate |
| dioxane | Dioxane |
| DCM | Dichloromethane |
| THF | Tetrahydrofuran |
| LiOH | Lithium hydroxide |
| FA | Formic acid |
| CCl₄ | Tetrachloromethane |
| DIBAL | Diisobutylaluminum hydride |
| Ruphos | 2-Dicyclohexylphosphonium-2',6'-diisopropoxy-1,1'-biphenyl |
| DMF | *N*,*N*-dimethylformamide |
| 50% HBr | 50% aqueous hydrogen bromide solution |
| Ac₂O | Acetic anhydride |
| AcOH | Acetic acid |
| HNO₃ | Nitric acid |
| TFA | Trifluoroacetic acid |
| Urotropine | Urotropin |
| ACN | Acetonitrile |
| EtOH | Ethanol |
| Cu₂O | Cuprous oxide |
| t-BuONO | *tert*-Butyl nitrite |
| CeCl₃.7H₂O | Cerous chloride heptahydrate |
| IBX | 2-Iodoxybenzoic acid |
| 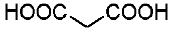 | Malonic acid |
| Pd/C | Palladium on carbon |
| Piperidine | Piperidine |
| C₅H₅N | Pyridine |
| Ti(OEt)₄ | Tetraethyl titanate |
| POCl₃ | Phosphorus oxychloride |
| TfOH | Triflic acid |
| AlCl₃ | Aluminum trichloride |
| LiAlH₄ | Lithium aluminum hydride |
| Et₂O | Diethyl ether |
| H₂O | Water |
| EA | Ethyl acetate |
| mCPBA | *m*-chloroperoxybenzoic acid |
| Et₃N | Triethylamine |
| DMAP | 4-Dimethylaminopyridine |
| t-BuLi | *tert*-Butyl lithium |
| KNO₃ | Potassium nitrate |
| H₂SO₄ | Sulfuric acid |
| Boc₂O | Di-*tert*-butyl dicarbonate |
| 1,2-Dichloroethane | 1,2-Dichloroethane |
| NaBH₄ | Sodium borohydride |
| Zn(CN)₂ | Zinc cyanide |
| Pd(PPh₃)₄ | Tetrakis(triphenylphosphine)palladium(0) |
| Na₂CO₃ | Sodium carbonate |
| Pd(dppf)Cl₂ | [1,1'-bis(diphenylphosphino)ferrocene]dichloropalladium(II) |
| HPO(OEt)₂ | Diethylphosphite |
| DIEA | *N*,*N*-diisopropylethylamine |
| PhMe | Toluene |
| PtO₂ | Platinum dioxide |
| TMSBr | Trimethylbromosilane |
| Ph₂NH | Diphenylamine |
| Ph₂SiH₂ | Diphenylsilane |
| B(C₆F₅)₃ | Tris(pentafluorophenyl)borane |
| B₂(OH)₄ | Tetrahydroxydiborane |
| X-Phos Pd G 2 | Chloro(2-dicyclohexylphosphino-2',4',6'-triisopropyl-1,1'-biphenyl)[2-(2'-amino-1,1'-biphenyl)]palladium(II) |
| X-Phos | 2-Dicyclohexylphosphino-2,4,6-triisopropylbiphenyl |
| KOAc | Potassium acetate |
| PhI (OAc)₂ | Phenyliodine diacetate |
| HO-NH₂ HCl | Hydroxylamine hydrochloride |
| HATU | 2-(7-Azabenzotriazol)-*N*,*N*,*N*',*N*'-tetramethyluronium hexafluorophosphate |
| CH₃I | Iodomethane |
| NaH | Sodium hydride |

### Example 1:

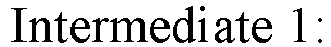

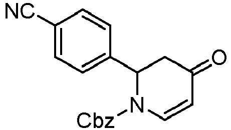

To a 3 L three-necked flask were successively added tetrahydrofuran (150 mL) and 4-bromoxynil (50 g). Isopropylmagnesium chloride lithium chloride coordination complex (1.3 M, 210 mL) was slowly added to the reaction system under nitrogen atmosphere. After the reaction was carried out at room temperature for 2 h, the reaction system was diluted with anhydrous tetrahydrofuran (500 mL) for dilution. The reaction system was cooled to -5 °C, and 4-methoxypyridine (25 mL) was added, followed by slowly dropwise addition of benzyl chloroformate (35 mL) (the system temperature was maintained below 0 °C). After the dropwise addition was completed, the reaction system was successively reacted at 0 °C for 2 h, and then warmed to room temperature and reacted at that temperature for 16 h. After the reaction was completed, hydrochloric acid solution (6 M, 150 mL) was added. The mixture was stirred at room temperature for half an hour, added with water (1000 mL) for dilution, and extracted twice with ethyl acetate (500 mL). The extract phase was washed with saturated brine (50 mL), then dried over anhydrous sodium sulfate and filtered. The filtrate was concentrated, and the resulting crude product was separated and purified by a silica gel column (petroleum ether:ethyl acetate = 3:1 to 1:1) to give intermediate 1 (23 g, yield: 23%). MS m/z (ESI): 333.0[M+H].

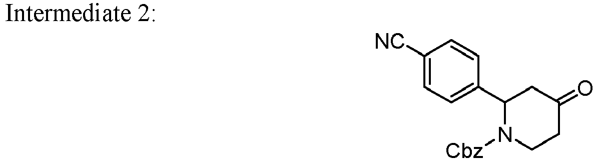

To a 500 mL single-neck flask were successively added intermediate 1 (28 g), zinc powder (55 g) and acetic acid (200 mL). The reaction mixture was heated to 100 °C and reacted at that temperature for 16 h. After the reaction was completed, the reaction mixture was filtered. The filtrate was added with water (500 mL) for dilution and extracted with ethyl acetate (500 mL). The extract phase was washed twice with saturated aqueous sodium bicarbonate solution (500 mL), washed once with saturated brine (100 mL), dried over anhydrous sodium sulfate and filtered. The filtrate was concentrated under reduced pressure to give intermediate 2 (26 g, yield: 73%). MS m/z (ESI): 334.8 [M+H].

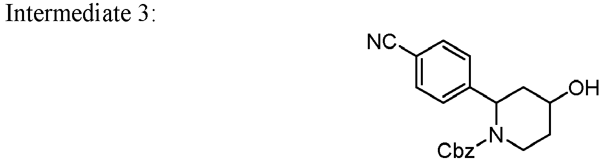

To a 1 L single-neck flask were successively added tetrahydrofuran (100 mL), ethanol (100 mL) and intermediate 2 (26 g), and sodium borohydride (2 g) was added in batches. The mixture was reacted at room temperature for 2 h. After the reaction was completed, the system was cooled to 0 °C, and saturated aqueous ammonium chloride solution (100 mL) was added until the temperature did not increase any more. Water (300 mL) was added for dilution, followed by extraction with ethyl acetate (200 mL × 2). The extract phase was washed with saturated brine (500 mL), dried over anhydrous sodium sulfate and filtered. The filtrate was concentrated under reduced pressure to give intermediate 3 (25 g, yield: 76%). MS m/z (ESI): 336.9[M+H].

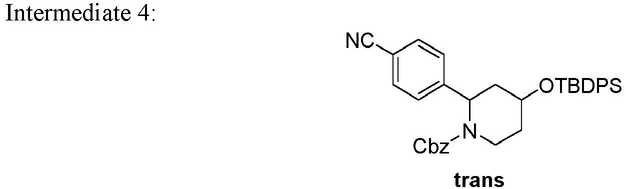

Dichloromethane (200 mL) was added to a 500 mL single-neck flask, and then intermediate 3 (25 g), imidazole (6.6 g) and tert-butyldiphenylchlorosilane (25 g) were successively added. The mixture was reacted at room temperature for 2 h. After the reaction was completed, water (500 mL) was added for dilution, followed by the extraction with dichloromethane (200 mL). The extract phase was washed with water (50 mL), dried over anhydrous sodium sulfate and filtered. The filtrate was concentrated under reduced pressure. The residue was separated and purified by a silica gel column (petroleum ether:ethyl acetate = 10:1) to give intermediate 4 (5.7 g, yield: 13%, R_{f} = 0.55; isomer R_{f} = 0.50). MS m/z (ESI):597.0[M+23].

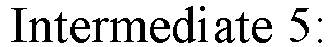

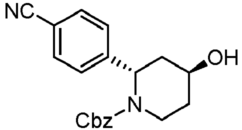

To a 250 mL single-neck flask were successively added a solution of tetrabutylammonium fluoride in tetrahydrofuran (1 M, 30 mL) and intermediate 4 (5 g). The mixture was reacted at room temperature for 2 h. After the reaction was completed, water (100 mL) was added for dilution, followed by the extraction with ethyl acetate (50 mL × 3). The extract phase was washed with saturated brine (100 mL), dried over anhydrous sodium sulfate and filtered. The filtrate was concentrated under reduced pressure. The residue was separated and purified by a silica gel column (petroleum ether: ethyl acetate = 3:1 to 0:1) to give a racemic intermediate. The intermediate was subjected to SFC chiral resolution (apparatus: SFC Thar prep 80; column: CHIRALPAK AD-H, 250 mm × 20 mm, 5 µm; modifier: 35% methanol (0.2% aqueous ammonia); column temperature: 40 °C; column pressure: 60 bar; wavelength: 214/254 nm; flow rate: 40 g/min; Rt = 4.78 min) to give intermediate 5 (1.2 g, yield: 41%). MS m/z (ESI): 358.8[M+23].

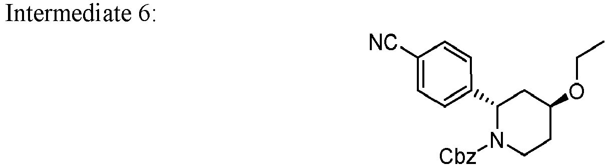

To a 100 mL single-neck flask were successively added *N*,*N*-dimethylformamide (15 mL) as a solvent, intermediate 5 (1.2 g) and iodoethane (1.1 g). After the reaction system was cooled to 0 °C, sodium hydrogen (60%, 243 mg) was added. Then the system was warmed to room temperature and reacted at that temperature for 2 h. After the reaction was completed, water (30 mL) was added for dilution, followed by the extraction with ethyl acetate (50 mL). The extract phase was washed with saturated brine (10 mL), dried over anhydrous sodium sulfate and filtered. The filtrate was concentrated under reduced pressure to give intermediate 6 (1.2 g, yield: 83%). MS m/z (ESI): 386.9[M+23].

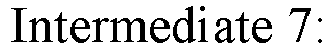

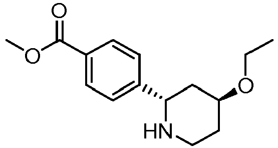

To a 100 mL single-neck flask were successively added methanol (10 mL), water (10 mL), concentrated sulfuric acid (10 mL) and intermediate 6 (1.2 g). The mixture was heated to 80 °C and reacted at that temperature for 48 h. After the reaction was completed, the reaction mixture was concentrated to remove methanol. The residue was made neutral with saturated aqueous sodium hydroxide solution and extracted three times with ethyl acetate (10 mL). The extract phase was washed with saturated brine (5 mL), dried over anhydrous sodium sulfate and filtered. The filtrate was concentrated under reduced pressure to give intermediate 7 (850 mg, yield: 81%). MS m/z (ESI): 264.1[M+H]. ¹H NMR (400 MHz, CDCl₃) *δ* 8.01 (d, *J* = 8.3 Hz, 2H), 7.49 (d, *J* = 8.3 Hz, 2H), 4.13 (dd, *J* = 11.7, 2.4 Hz, 1H), 3.92 (s, 3H), 3.82 - 3.70 (m, 1H), 3.62 - 3.47 (m, 2H), 3.27 - 3.10 (m, 1H), 3.02 - 2.88 (m, 1H), 2.07 - 1.97 (m, 1H), 1.95 - 1.85 (m, 1H), 1.82 - 1.62 (m, 2H), 1.27 (t, *J* = 7.0 Hz, 3H).

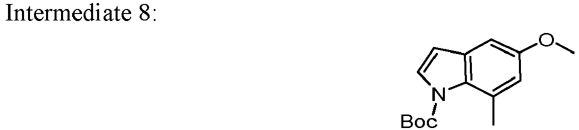

To a 250 mL single-neck flask were successively added dichloromethane (50 mL), 5-methoxy-7-methyl-1*H*-indole (3 g), BOC anhydride (5.68 g), 4-dimethylaminopyridine (227 mg) and triethylamine (2.26 g). The mixture was reacted at room temperature for 16 h. After the reaction was completed, the reaction mixture was quenched by adding saturated ammonium chloride solution (5 mL) and extracted three times with dichloromethane (20 mL). The combined organic phases were washed with water (5 mL), dried over anhydrous sodium sulfate and filtered. The filtrate was concentrated. The residue was purified by column chromatography on silica gel (petroleum ether:ethyl acetate = 10:1) to give intermediate 8 (4.6 g, yield: 94%). MS m/z (ESI): 262.0[M+H].

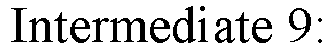

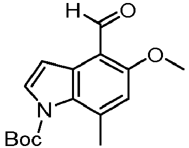

To a 250 mL single-neck flask were successively added dichloromethane (80 mL), *N-*methylformanilide (3.8 g) and oxalyl chloride (3.6 g). The mixture was stirred at room temperature for 3 h. Then the reaction temperature was lowered to -14 °C, and intermediate 8 (2.5 g) was added. The reaction system was naturally warmed to room temperature and stirred for 1 h. After the reaction was completed, the reaction liquid was poured into ice water and extracted three times with dichloromethane (100 mL). The combined extract phases were washed twice with water (10 mL), dried over anhydrous sodium sulfate and filtered. The filtrate was concentrated. The residue was separated and purified by a silica gel column (petroleum ether: ethyl acetate = 20:1) to give intermediate 9 (1.3 g, yield: 47%). MS m/z (ESI): 290.0[M+H]. ¹H NMR (400 MHz, CDCl₃) *δ* 10.65 (s, 1H), 7.65 (d, *J* = 3.4 Hz, 1H), 7.49 (d, *J* = 3.4 Hz, 1H), 6.76 (s, 1H), 3.98 (s, 3H), 2.70 (s, 3H), 1.65 (s, 9H).

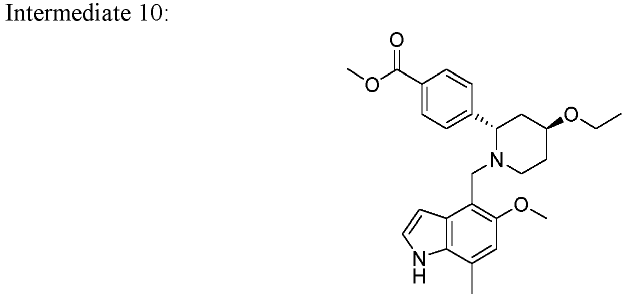

To a 50 mL three-necked flask were successively added 1,2-dichloroethane (5 mL), intermediate 7 (127 mg) and intermediate 9 (130 mg). The mixture was reacted at room temperature for 18 h. Then sodium triacetoxyborohydride (438.72 mg) was added, and the system was successively reacted at room temperature for 18 h. After the reaction was completed, dichloromethane (10 mL) was added for dilution, followed by a wash with 10 mL of water. The organic phase was dried over anhydrous sodium sulfate and filtered. The filtrate was concentrated. The residue was separated and purified by a silica gel column (methanol:dichloromethane = 1:10) to give intermediate 10 (50 mg, yield: 14.58%). MS m/z(ESI): 437.3 [M+H], RT = 1.142 min.

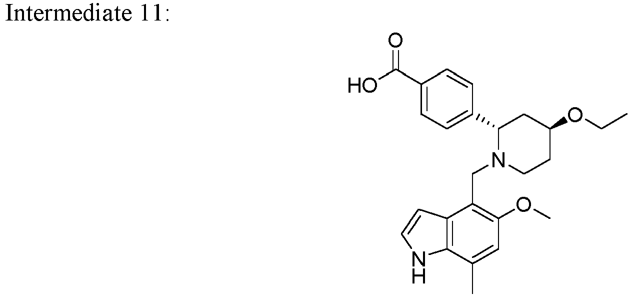

To a 50 mL three-necked flask were successively added tetrahydrofuran (0.5 mL), methanol (0.5 mL), water (0.5 mL), sodium hydroxide (44 mg) and intermediate 10 (50 mg). The mixture was reacted at room temperature for 18 h. After the reaction was completed, the reaction liquid was directly concentrated under reduced pressure and lyophilized to give intermediate 11 (50 mg, yield: 92%). MS m/z(ESI): 423.1 [M+H].

Intermediate 7 can be obtained through another solution:

### Intermediate 12:

To a 2 L three-necked flask were successively added a solution of tetrabutylammonium fluoride in tetrahydrofuran (1 M, 840 mL) and intermediate 4 (140 g). The mixture was reacted at room temperature for 2 h. After the reaction was completed, water (600 mL) was added for dilution, followed by the extraction with ethyl acetate (700 mL × 3). The extract phase was washed with saturated brine (500 mL), dried over anhydrous sodium sulfate and filtered. The filtrate was concentrated under reduced pressure. The residue was separated and purified by a silica gel column (petroleum ether:ethyl acetate = 3:1 to 1:1) to give intermediate 12 (77 g, yield: 95%), MS M/z (ESI): 358.8[M+23].

### Intermediate 13:

To a 2 L three-necked flask were successively added the solvents *N*,*N*-dimethylformamide (700 mL), intermediate 12 (77 g) and iodoethane (56 g). After the reaction system was cooled to 0 °C, sodium hydrogen (60%, 14.61 g) was added. Then the system was warmed to room temperature and reacted at that temperature for 2 h. After the reaction was completed, the temperature was lowered to 0 °C, and aqueous ammonium chloride solution was added until the temperature of the reaction mixture did not increase. Ethyl acetate (500 mL) was added for extraction. The extract phase was washed with saturated brine (300 mL), dried over anhydrous sodium sulfate and filtered. The filtrate was concentrated under reduced pressure to give intermediate 13 (75 g, yield: 89%). MS m/z (ESI): 386.9[M+23].

### Intermediate 14:

To a 2 L three-necked flask were successively added isopropanol (300 mL), water (800 mL), intermediate 3 (75 g) and Ba(OH)₂.8H₂O (233 g). The mixture was heated to 100 °C and reacted at that temperature for 20 h. After the reaction was completed, the reaction liquid was concentrated to remove isopropanol. The residue was adjusted to pH 2-3 with saturated aqueous sodium hydroxide solution and extracted three times with dichloromethane (300 mL). The extract phase was washed with saturated brine (200 mL), dried over anhydrous sodium sulfate and filtered. The filtrate was concentrated under reduced pressure to give intermediate 14 (67 g, yield: 85%). MS m/z (ESI): 384.1[M+H].

### Intermediate 15:

To a 2 L three-necked flask were successively added *N*,*N*-dimethylformamide (670 mL), potassium carbonate (96.6 g), iodomethane (37.3 g) and intermediate 14 (67 g). The mixture was reacted at room temperature for 2 h. After the reaction was completed, 300 mL of water was added to quench the reaction, followed by the extraction with methyl tert-butyl ether (300 mL × 2). The extract phase was washed with saturated brine (5 mL), dried over anhydrous sodium sulfate and filtered. The filtrate was concentrated under reduced pressure and purified by column chromatography (petroleum ether:ethyl acetate = 10:1 to 3:1) to give intermediate 15 (54 g, yield: 78%). MS m/z (ESI): 394.1[M+H].

### Intermediate 7:

To a 1 L single-neck flask were successively added ethyl acetate (500 mL), palladium on carbon (5.4 g, 10% loading) and intermediate 15 (54 g). The mixture was reacted under one pressure of hydrogen at room temperature for 16 h. After the reaction was completed, the reaction liquid was added to celite for filtration. The filtrate was concentrated under reduced pressure to give a racemic intermediate. The intermediate was subjected to chiral resolution (apparatus: Shimadzu LC-20AD; column: CHIRALPAK AD-H(ADH0CD-SK003), 0.46 cm I.D. × 25 cm L; modifier: (methanol/diethylamine 0.1%)/CO₂ = 25/75 (V/V); flow rate: 2.0 mL/min; Rt = 3.58 min) to give intermediate 7 (15.7 g, yield: 43%). 1H NMR (400 MHz, DMSO- *d₆*) δ 7.89 (d, J = 8.26 Hz, 2H), 7.50 (d, J = 8.26 Hz, 2H), 3.92 (dd, J = 11.36, 2.32 Hz, 1H), 3.84 (s, 3H), 3.69-3.64 (m, 1H), 3.51-3.42 (m, 2H), 2.94 (dt, J = 12.15, 2.56 Hz, 1H), 2.76 (ddd, J = 11.62, 4.24, 2.62 Hz, 1H), 1.85 (dd, J = 13.23, 2.16 Hz, 1H), 1.73 (d, J = 13.47 Hz, 1H), 1.59-1.41 (m, 2H), 1.16 (t, J = 6.98 Hz, 3H), MS m/z (ESI): 264.0[M+H].

### Target compound:

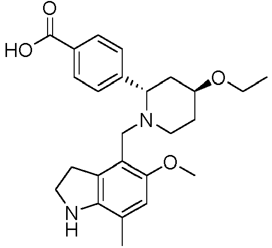

To a 25 mL three-necked flask were successively added acetic acid (1 mL), intermediate 11 (50 mg) and sodium cyanoborohydride (23 mg). The mixture was reacted at room temperature for 18 h. After the reaction was completed, the reaction mixture was added with ethyl acetate (5 mL) for dilution, washed once with saturated brine (5 mL), dried over anhydrous sodium sulfate and filtered. The filtrate was concentrated under reduced pressure. The residue was purified by Prep-HPLC (apparatus: Shimadzu LC-20AD; column: CHIRALPAK AD-H(ADH0CD-SK003), 0.46 cm I.D. × 25 cm L; modifier: (methanol/diethylamine 0.1%)/CO₂ = 25/75 (V/V); flow rate: 2.0 mL/min; Rt = 3.58 min) to give the target compound (10 mg, yield: 18.67%). MS m/z(ESI): 425.1 [M+H]. ¹H NMR (400 MHz, DMSO- *d₆*) δ 8.19 (d, *J* = 8.4 Hz, 2H), 7.69 (d, *J* = 8.4 Hz, 2H), 6.63 (s, 1H), 4.83 - 4.68 (m, 1H), 4.15 - 4.01 (m, 1H), 4.00 - 3.85 (m, 2H), 3.72 (s, 3H), 3.67 - 3.57 (m, 2H), 3.55 - 3.35 (m, 4H), 3.10 - 2.85 (m, 2H), 2.35 - 2.22 (m, 2H), 2.18 (s, 3H), 2.17 - 2.04 (m, 2H), 1.31 (t, *J* = 6.8 Hz, 3H).

### Example 2:

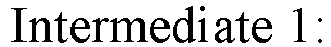

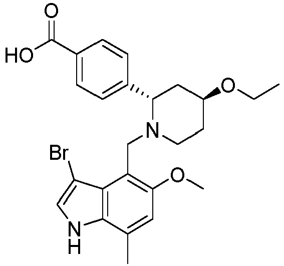

N-bromosuccinimide (22.4 mg) was added in batches to the reaction liquid of intermediate 10 (50 mg) of Example 1 in *N*,*N*-dimethylformamide (1 mL). After the reaction was carried out at room temperature for 18 h, the reaction liquid was directly purified by Prep-HPLC (column: C18 spherical, 100A, 20 g, 20-35 µm; acetonitrile-water = 10-70%, UV: 214 nm) to give intermediate 1 (120 mg, yield: 48.38%), MS m/z(ESI): 501.1[M+H].

### Target compound:

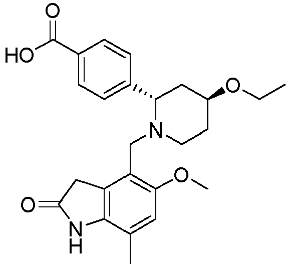

To a 10 mL small flask were successively added tetrahydrofuran (1 mL), intermediate 1 (10 mg) and 50% hydrobromic acid solution (0.5 mL). The mixture was warmed to 70 °C and reacted at that temperature for 5 h. After the reaction was completed, the reaction mixture was concentrated under reduced pressure. The resulting residue was purified by Prep-HPLC (column: C18 spherical, 100A, 20 g, 20-35 µm; acetonitrile-water = 10-70%; UV: 214 nm.) to give the target compound (51.5 mg, yield: 55.8%). MS m/z (ESI): 439.3[M+H]. ¹H NMR (400 MHz, CD₃OD) δ 8.20 (d, *J* = 8.0 Hz, 2H), 7.72 (d, J = 8.0 Hz, 2H), 6.76 (s, 1H), 4.77 (dd, J = 12.0 Hz, 3.6 Hz, 1H), 4.08 - 3.97 (m, 2H), 3.89 - 3.82 (m, 1H), 3.66 - 3.55 (m, 4H), 3.53 - 3.39 (m, 3H), 2.36 - 2.21 (m, 5H), 2.18 - 2.09 (m, 2H), 1.95 - 1.85 (m, 1H), 1.70 - 1.60 (m, 1H), 1.30 (t, J = 7.0 Hz, 3H).

### Example 3

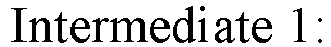

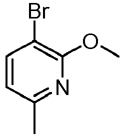

To a 250 mL single-neck flask were successively added methanol (100 mL) and 3-bromo-2-fluoro-6-methylpyridine (10 g), and then potassium tert-butoxide (11 g) was added in batches to the reaction system. The mixture was heated to 75 °C and stirred at that temperature for 3 h. After the reaction was completed, the reaction mixture was directly concentrated under reduced pressure and separated and purified by a silica gel column (petroleum ether:ethyl acetate = 10:1) to give intermediate 1 (10 g, yield: 89%). MS m/z (ESI): 201.9[M+H].

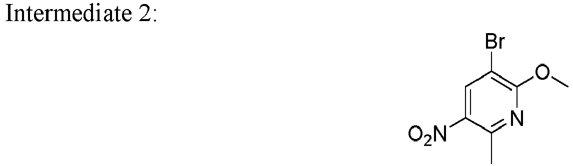

To a 250 mL single-neck flask were successively added concentrated sulfuric acid (50 mL) and intermediate 1 (10 g), and then nitric acid (20 mL) was slowly added dropwise. After the dropwise addition was completed, the mixture was reacted at room temperature for 1 h. After the reaction was completed, the reaction liquid was poured into ice water and extracted twice with ethyl acetate (500 mL). The organic phase was washed with saturated brine (300 mL), dried over anhydrous sodium sulfate and filtered. The filtrate was concentrated under reduced pressure to give intermediate 2 (7.5 g, yield: 75%). ¹H NMR (400 MHz, DMSO-*d₆*) *δ* 8.65 (s, 1H), 4.04 (s, 3H), 2.69 (s, 3H).

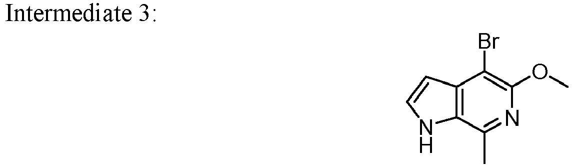

To a 500 mL single-neck flask were successively added the solvent tetrahydrofuran (100 mL) and intermediate 2 (5 g). Vinylmagnesium chloride (1 M in THF, 100 mL) was slowly added dropwise to the reaction system at -40 °C under nitrogen atmosphere. The mixture was slowly warmed to -20 °C and reacted at that temperature for 2 h. After the reaction was completed, the reaction mixture was quenched by adding aqueous ammonium chloride solution (100 mL) and extracted twice with ethyl acetate (200 mL). The combined organic phases were washed with saturated brine (100 mL), dried over anhydrous sodium sulfate and filtered. The filtrate was concentrated under reduced pressure. The residue was separated and purified by a silica gel column (dichloromethane:methanol = 30:1) to give intermediate 3 (4 g, yield: 75%). MS m/z (ESI): 240.8[M+H].

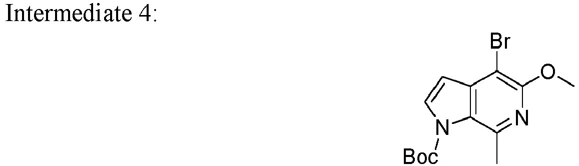

To a 500 mL single-neck flask were successively added dichloromethane (100 mL), intermediate 3 (4 g), Boc anhydride (6 g) and 4-dimethylaminopyridine (3 g). The mixture was reacted at room temperature for 2 h. After the reaction was completed, water (100 mL) was added for dilution, followed by the extraction with dichloromethane (100 mL). The organic phase was washed with water (30 mL), dried over anhydrous sodium sulfate and filtered. The filtrate was concentrated under reduced pressure. The residue was separated and purified by a silica gel column (petroleum ether:ethyl acetate = 10: 1) to give intermediate 4 (2.7 g, yield: 44%). MS m/z (ESI): 340.8[M+H].

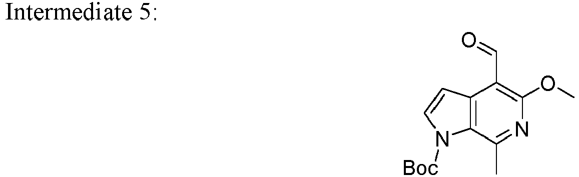

To a 50 mL three-necked flask were successively added tetrahydrofuran (10 mL) as a solvent and intermediate 4 (500 mg). The mixture was cooled to -78 °C with dry ice, and then *n-*butyllithium (0.75 mL) was slowly added dropwise under nitrogen atmosphere. After the reaction was carried out at -78 °C for 1 h, anhydrous DMF (131 mg) was slowly added to the reaction system, and the reaction system was successively reacted at that temperature for 2 h. The reaction system was naturally warmed to 0 °C, quenched by adding saturated ammonium chloride (20 mL) and extracted twice with ethyl acetate (50 mL). The organic phase was washed with saturated brine (50 mL), dried over anhydrous sodium sulfate and filtered. The filtrate was concentrated under reduced pressure. The residue was separated and purified by a silica gel column (petroleum ether:ethyl acetate = 10:1) to give intermediate 5 (160 mg, yield: 16%). MS m/z (ESI): 291.1[M+H].

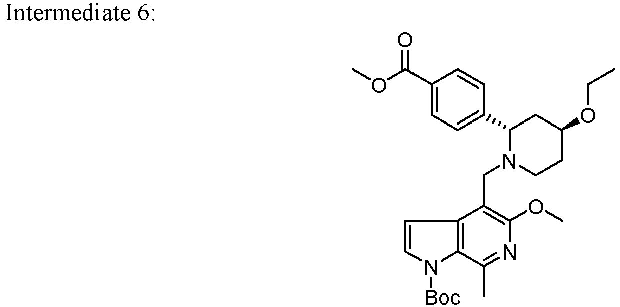

To a 100 mL single-neck flask were successively added 1,2-dichloroethane (3 mL) as a solvent, intermediate 5 (60 mg) and intermediate 7 (55 mg) of Example 1. After the reaction was carried out at 25 °C for 8 h, sodium triacetoxyborohydride (120 mg) was added, and the mixture was successively reacted at 25 °C for 16 h. After the reaction was completed, silica gel was directly added to the reaction liquid and stirred, and the mixture was separated and purified by a silica gel column (methanol:dichloromethane = 1:20) to give intermediate 6 (75 mg, 60%). MS m/z (ESI): 537.8[M+H].

### Target compound:

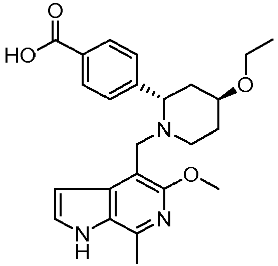

To a 25 mL single-neck flask were successively added methanol (2 mL), water (2 mL), intermediate 6 (75 mg) and sodium hydroxide (50 mg). The mixture was heated to 75 °C and reacted at that temperature for 3 h. After the reaction was completed, the reaction mixture was directly purified by Prep-HPLC (column: Gemini-C18, 150 × 21.2 mm, 5 µm; mobile phase: acetonitrile-water (0.1% formic acid); gradient: 15-35%). The resulting solution was concentrated. The remaining small amount of aqueous solution was lyophilized to give the target compound (33.0 mg, yield: 46%, containing 0.5 equivalents of formic acid). MS m/z (ESI): 423.9[M+H]. ¹H NMR (400 MHz, CD₃OD) δ 8.43 (s, 0.5H), 8.16 (d, *J* = 8.4 Hz, 2H), 7.65 (d, J = 8.4 Hz, 2H), 7.51 (d, *J* = 3.2 Hz, 1H), 6.34 (s, 1H), 4.76 - 4.66 (m, 1H), 4.33 - 4.13 (m, 2H), 3.89 (s, 3H), 3.88 - 3.82 (m, 1H), 3.66 - 3.58 (m, 2H), 3.57 - 3.49 (m, 1H), 3.41 - 3.34 (m, 1H), 2.64 (s, 3H), 2.32 - 2.22 (m, 2H), 2.14 - 2.04 (m, 2H), 1.32 (t, *J* = 6.8 Hz, 3H).

### Example 4:

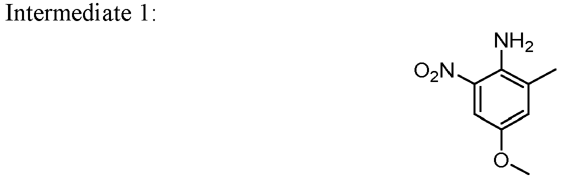

An aqueous solution of sodium hydroxide (15%, 10 mL) was added to a solution of N-(4-methoxy-2-methyl-6-nitrophenyl)acetamide (500 mg) in ethanol (10 mL) at room temperature. The mixture was heated to 90 °C and reacted at that temperature for 16 h. After the reaction was completed, the reaction system was cooled to room temperature, concentrated under reduced pressure to remove ethanol, added with water (20 mL) for dilution, adjusted to pH 5-6 with 5 M hydrochloric acid and extracted five times with ethyl acetate (20 mL). The combined extract phases were washed with saturated brine (20 mL), dried over anhydrous sodium sulfate and filtered. The filtrate was concentrated under reduced pressure. The residue was separated and purified by a silica gel column (petroleum ether:ethyl acetate = 5:1) to give intermediate 1 (180 mg, yield: 44%). MS m/z (ESI): 183.0 [M+H].

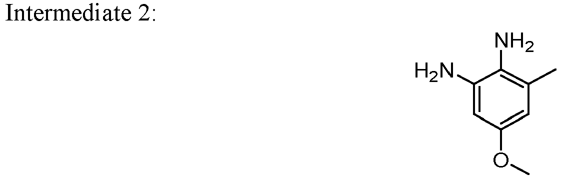

10% Pd/C (54 mg, 30% wt/wt) was added to a solution of intermediate 1 (180 mg) in methanol (15 mL) at room temperature. A hydrogenation reaction was carried out at room temperature for 16 h. After the reaction was completed, the reaction mixture was filtered. The filtrate was directly concentrated under reduced pressure to give intermediate 2 (120 mg, yield: 80%). MS m/z (ESI):
153.1 [M+H].

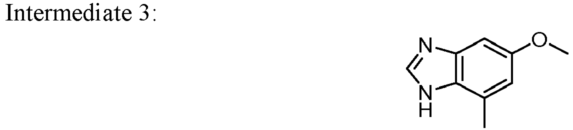

Formic acid (681 mg) was added to a solution of intermediate 2 (450 mg) in hydrochloric acid (4 M, 20 mL) at room temperature. The reaction system was heated to 100 °C and reacted at that temperature for 3 h. After the reaction was completed, the reaction mixture was poured into water (20 mL). The mixture was adjusted to pH 8-9 with 5 M sodium hydroxide solution and extracted three times with ethyl acetate (30 mL). The combined organic phases were washed twice with saturated brine (20 mL), dried over anhydrous sodium sulfate and filtered. The filtrate was concentrated under reduced pressure. The residue was separated and purified by a silica gel column (dichloromethane:methanol = 10:1) to give intermediate 3 (320 mg, yield: 66%). MS m/z (ESI): 163.1 [M+H].

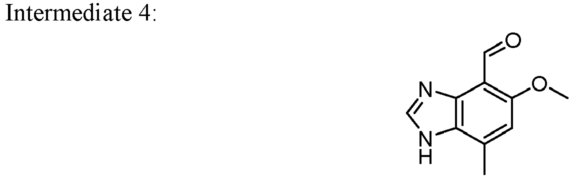

Urotropin (220 mg) was added to a solution of intermediate 3 (170 mg) in trifluoroacetic acid (10 mL) at 5 °C. The mixture was heated to 80 °C and reacted at that temperature for 3 h. After the reaction was completed, the reaction mixture was concentrated under reduced pressure and separated and purified by a silica gel column (dichloromethane:methanol = 10:1) to give intermediate 4 (170 mg, yield: 85%). MS m/z (ESI): 191.0 [M+H].

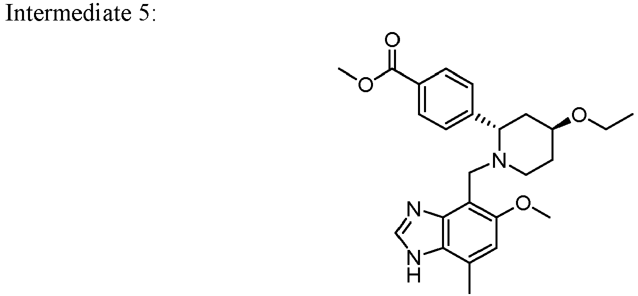

Sodium triacetoxyborohydride (381.60 mg) was added to a solution of intermediate 4 (171 mg) and intermediate 7 (157 mg) of Example 1 in 1,2-dichloroethane (10 mL) at room temperature. The mixture was reacted at 25 °C for 16 h. After the reaction was completed, the reaction mixture was concentrated under reduced pressure. The crude product was separated and purified by a silica gel column (dichloromethane:methanol = 20: 1) to give intermediate 5 (47 mg, yield: 18%). MS m/z (ESI): 438.0 [M+H].

### Target compound:

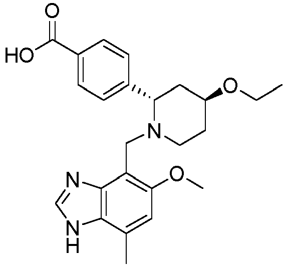

A solution of sodium hydroxide (60 mg) in water (2 mL) was added to a solution of intermediate 5 (67 mg) in methanol (6 mL) at room temperature. The mixture was reacted at room temperature for 16 h. After the reaction was completed, the methanol solvent was removed by concentration under reduced pressure. The aqueous phase was adjusted to pH 5-6 with hydrochloric acid (5 M) solution and then concentrated under reduced pressure. The crude product was separated and purified by Prep-HPLC (column: Gemini-C18, 150 × 21.2 mm, 5 µm; mobile phase: acetonitrile-water (0.1% formic acid); gradient: 5-30%) and then purified by preparative TLC (dichloromethane/methanol = 5:1) to give the target compound (13 mg, yield: 20%, containing 0.4 equivalents of formic acid). MS m/z (ESI): 423.8 [M+H].¹H NMR (400 MHz,CD₃OD) δ 8.54 (s, 0.4H), 8.13 (s, 1H), 8.02 (d, *J* = 8.2 Hz, 2H), 7.58 (d, *J=* 8.2 Hz, 2H), 6.82 (s, 1H), 4.26 - 4.16 (m, 1H), 4.11 (d, *J* = 13.6 Hz, 1H), 3.83 (d, *J* = 13.6 Hz, 1H), 3.76 (s, 4H), 3.56 (q, *J* = 6.8 Hz, 2H), 3.15 - 2.91 (m, 2H), 2.55 (s, 3H), 2.17 - 2.07 (m, 2H), 1.98 - 1.92 (m, 2H), 1.27 (t, *J* = 6.8 Hz, 3H).

### Example 5:

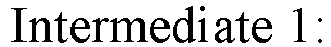

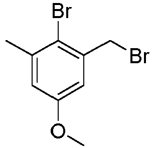

To a solution of 2-bromo-5-methoxy-1,3-xylene (10 g) in carbon tetrachloride (100 mL) were added N-bromosuccinimide (8 g) and azobisisobutyronitrile (AIBN) (1 g). The mixture was heated to 80 °C and stirred at that temperature overnight. After the reaction was completed, the reaction mixture was poured into dichloromethane (300 mL) for dilution, followed by two washes with aqueous sodium bicarbonate solution (30 mL) and then one wash with water (30 mL). The mixture was dried over anhydrous sodium sulfate and filtered. The filtrate was concentrated under reduced pressure. The residue was separated and purified by a silica gel column (petroleum ether:ethyl acetate = 10:1) to give crude intermediate 1 (11 g, yield: 64%).

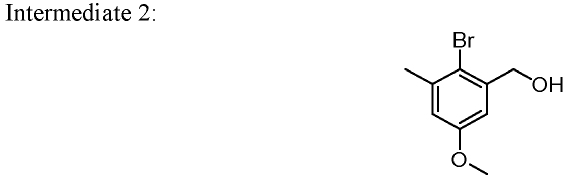

To a solution of intermediate 1 (15 g) in dioxane (250 mL) were added cesium carbonate (30 g) and water (250 mL). The reaction system was heated to 110 °C and stirred at that temperature for 16 h. After the reaction was completed, the reaction mixture was concentrated under reduced pressure. The residue was separated and purified by a silica gel column (petroleum ether:ethyl acetate = 10:1) to give intermediate 2 (6.5 g, 48%). MS m/z (ESI): 252.9 [M+Na].

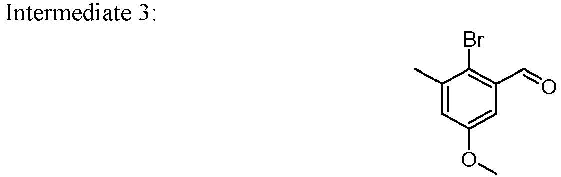

To a solution of intermediate 2 (1.8 g) in dichloromethane (50 mL) was added manganese dioxide (3 g). The mixture was stirred at 25 °C overnight. After the reaction was completed, the reaction system was filtered. The filtrate was concentrated under reduced pressure. The residue was purified by silica gel column chromatography (petroleum ether:ethyl acetate = 10:1) to give intermediate 3 (0.72 g, yield: 44%). MS m/z (ESI): 228.8[M+H].

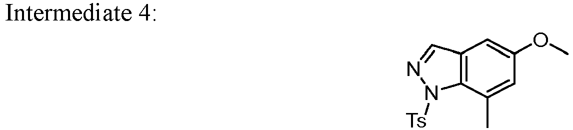

Intermediate 3 (680 mg) was added to a solution of *p*-toluenesulfonyl hydrazide (550 mg) in methanol (10 mL). The mixture was heated at reflux for 3 h. Then the solvent was removed by concentration under reduced pressure. The resulting product was added to a mixture of cuprous oxide (2 g) in mesitylene (10 mL). The mixture was heated to 130 °C and successively reacted at that temperature for 16 h. After the reaction was completed, the reaction mixture was directly concentrated to remove the solvent. The residue was separated and purified by a silica gel column (petroleum ether:ethyl acetate = 10:1) to give intermediate 4 (400 mg, yield: 42%). MS m/z (ESI): 317.0 [M+H].

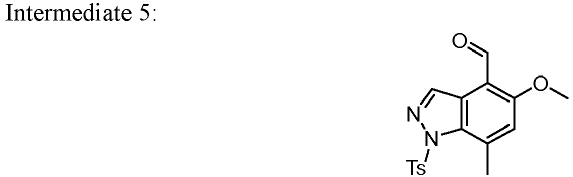

After intermediate 4 (800 mg) was added to dichloromethane solution (6 mL), the temperature was lowered to -78 °C, and titanium tetrachloride (1.8 g) was slowly added dropwise at that temperature. After the mixture was stirred for five minutes, dichloromethyl methyl ether (1.2 g) was added. The resulting reaction mixture was slowly warmed to 0 °C and reacted for 6 h. After the reaction was completed, the reaction system was cooled to -40 °C, quenched by slowly adding water (10 mL) and diluted with ethyl acetate (30 mL) and water (10 mL). After being separated, the organic phase was washed once with aqueous sodium bicarbonate solution (5 mL), washed with saturated brine (5 mL), dried over anhydrous sodium sulfate and filtered. The filtrate was concentrated under reduced pressure. The residue was separated and purified by a silica gel column (petroleum ether:ethyl acetate = 10:1) to give intermediate 5 (400 mg, yield: 38%). ¹H NMR (400 MHz, CDCl₃) *δ* 10.53 (s, 1H), 8.94 (s, 1H), 7.71 (d, *J* = 8.4 Hz, 2H), 7.24 (d, *J* = 8.4 Hz, 2H), 7.04 (s, 1H), 4.00 (s, 3H), 2.93 (s, 3H), 2.38 (s, 3H).

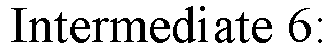

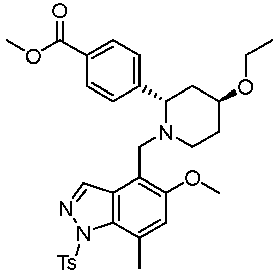

A solution of intermediate 5 (214 mg) and intermediate 7 (140 mg) of Example 1 in 1,2-dichloroethane (2 mL) was stirred at room temperature under nitrogen atmosphere for 6 h, and then sodium borohydride triacetate (340 mg) was added. The mixture was stirred at room temperature under nitrogen atmosphere overnight. After the reaction was completed, dichloroethane (10 mL) and water (10 mL) were added to dilute the reaction mixture. After being separated, the organic phase was dried over anhydrous sodium sulfate and filtered. The filtrate was concentrated under reduced pressure. The residue was separated and purified by a silica gel column (petroleum ether:ethyl acetate = 5:1) to give intermediate 6 (170 mg, yield: 54%). MS m/z (ESI): 592.0[M+H].

### Target compound:

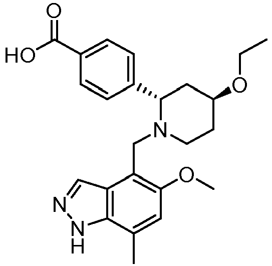

A solution of lithium hydroxide (90 mg) in water (1 mL) was added to a solution of intermediate 6 (170 mg) in tetrahydrofuran (2 mL) and methanol (2 mL). The mixture was stirred at room temperature overnight. After the reaction was completed, the reaction mixture was adjusted to pH 3-4 with 1 M hydrochloric acid and concentrated under reduced pressure to remove the solvent. The residue was separated and purified by Prep-HPLC (column: Gemini-C18, 150 × 21.2 mm, 5 µm; mobile phase: acetonitrile-water (0.1% formic acid); gradient: 10-40%). The resulting solution was concentrated. The remaining small amount of aqueous solution was lyophilized to give the target compound (49 mg, yield: 38%, containing 0.5 equivalents of *p-*toluenesulfonic acid). MS m/z (ESI): 424.3[M+H]. ¹H NMR (400 MHz,CD₃OD) δ 8.18 (d, *J* = 8.0 Hz, 2H), 7.95 (s, 1H), 7.75 - 7.65 (m, 3H), 7.21 (d, J = 8.0 Hz, 1H), 7.10 (s, 1H), 4.80 - 4.65 (m, 1H), 4.40 - 4.20 (m, 2H), 3.90 - 3.77 (m, 4H), 3.65 - 3.50 (m, 3H), 3.58 (s, 3H), 2.36 (s, 1.5H), 2.32 - 2.20 (m, 2H), 2.15 - 1.95 (m, 2H), 1.30 (t, *J* = 6.8 Hz, 3H).

### Example 6:

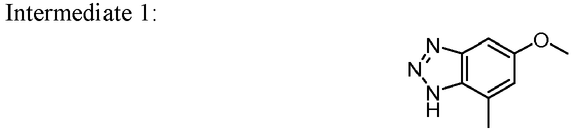

*tert*-Butyl nitrite (499 mg) was added to a solution of 5-methoxy-3-methylbenzene-1,2-diamine (500 mg) in acetonitrile (20 mL) at room temperature. The reaction mixture was stirred at room temperature for 16 h. After the reaction was completed, the reaction mixture was concentrated under reduced pressure. The residue was separated and purified by a silica gel column (dichloromethane:methanol = 20:1) to give intermediate 1 (360 mg, yield: 67%). MS m/z (ESI): 164.1 [M+H].

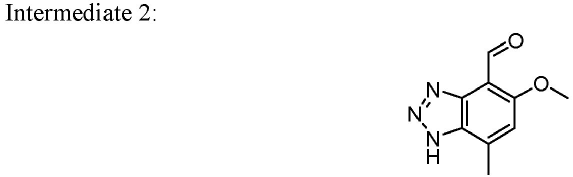

Urotropin (582 mg) was added to a solution of intermediate 1 (340 mg) in trifluoroacetic acid (10 mL) at room temperature. The mixture was heated to 80 °C and stirred at that temperature for 3 h. After the reaction was completed, the reaction system was cooled to room temperature. The reaction mixture was directly concentrated under reduced pressure. The resulting residue was separated and purified by a silica gel column (dichloromethane:methanol = 10:1) to give intermediate 2 (200 mg, yield: 50%). MS m/z (ESI): 192.0 [M+H].

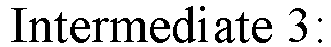

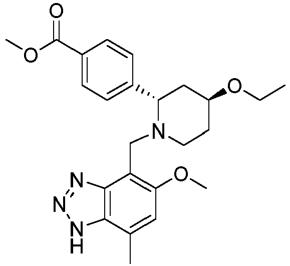

Sodium triacetoxyborohydride (419.76 mg) was added to a solution of intermediate 2 (189.27 mg) and intermediate 7 (174 mg) of Example 1 in 1,2-dichloroethane (20 mL) at room temperature. The mixture was stirred at room temperature for 16 h. After the reaction was completed, the reaction mixture was directly concentrated under reduced pressure. The resulting residue was separated and purified by a silica gel column (dichloromethane:methanol = 50:1) to give intermediate 3 (180 mg, yield: 62%). MS m/z (ESI): 438.8 [M+H].

### Target compound:

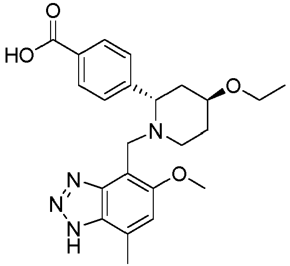

A solution of sodium hydroxide (164 mg) in water (2 mL) was added to a solution of intermediate 3 (180 mg) in methanol (6 mL) at room temperature. The mixture was stirred at room temperature for 16 h. After the reaction was completed, methanol was removed by concentration under reduced pressure. The aqueous phase was adjusted to pH 5-6 with 5 M hydrochloric acid solution and directly concentrated under reduced pressure. The resulting residue was separated and purified by Prep-HPLC (column: Gemini-C18, 150 × 21.2 mm, 5 µm; mobile phase: acetonitrile-water (0.1% formic acid); gradient: 10-30%) to give the target compound (65 mg, yield: 37%, containing 0.5 equivalents of formic acid). MS m/z (ESI): 424.8 [M+H].¹H NMR (400 MHz,CD₃OD) δ 8.37 (s, 0.5H), 8.11 (d, *J* = 8.0 Hz, 2H), 7.68 (d, *J* = 8.0 Hz, 2H), 7.10 (s, 1H), 4.54 - 4.44 (m, 1H), 4.34 - 4.26 (d, J = 13.2 Hz, 1H), 4.14 - 4.04 (m, 1H), 3.87-3.80 (m, 4H), 3.65 - 3.55 (m, 2H), 3.43 - 3.32 (m, 1H), 3.17 - 3.08 (m, 1H), 2.69 (s, 3H), 2.28-2.15 (m, 2H), 2.08-1.99 (m, 2H), 1.30 (t, *J=* 7.0 Hz, 3H).

### Example 7:

### Target compound:

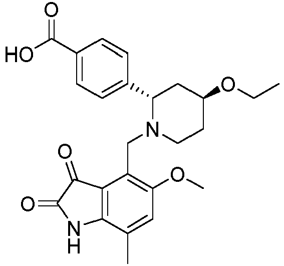

To a 50 mL single-neck flask were successively added acetonitrile (20 mL), water (2 mL), intermediate 11 (150 mg) of Example 1, cerium chloride heptahydrate (8.1 mg) and 2-iodoxybenzoic acid (252 mg). The reaction system was heated to 80 °C and stirred at that temperature for 3.5 h. After the reaction was completed, the reaction mixture was directly concentrated under reduced pressure. The resulting residue was purified by Prep-HPLC (column: C18 spherical, 100A, 20 g, 20-35 µm; acetonitrile-water = 10-70%, UV: 214 nm) to give the target compound (5.9 mg; yield: 55.8%, containing 0.8 equivalents of formic acid). MS m/z (ESI): 453.1[M+H]. ¹H NMR (400 MHz,CD₃OD) δ 8.43(s, 0.8H), 8.04 (d, *J* = 8.0 Hz, 2H), 7.58 (d, *J* = 8.0 Hz, 2H), 6.72 (s, 1H), 3.76 - 3.71 (m, 4H), 3.60 - 3.40 (m, 4H), 3.20 - 3.00 (m, 1H), 2.55 - 2.35 (m, 2H), 2.20 (s, 3H), 2.10 - 1.98 (m, 2H), 1.93 - 1.83 (m, 1H), 1.70 - 1.56 (m, 1H), 1.23 (t, *J* = 6.8 Hz, 3H).

### Example 8:

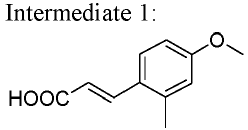

To a 250 mL single-neck flask were added pyridine (70 mL), 4-methoxy-2-methylbenzaldehyde (10 g), malonic acid (13.87 g) and piperidine (568 mg). The reaction system was heated to 90 °C and stirred at that temperature for 16 h. After the reaction was completed, the reaction mixture was poured into ice water. The pH was adjusted to 3-4 with diluted hydrochloric acid, and a solid precipitated. The solid was collected by filtration and dried to give intermediate 1 (12 g, yield: 86%). MS m/z (ESI): 193.1[M+H].

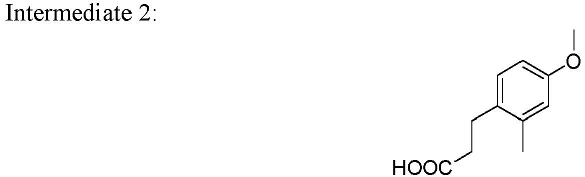

To a 250 mL single-neck flask were added ethanol (100 mL), intermediate 1 (5 g) and Pd/C (1 g). A catalytic hydrogenation reaction was carried out under hydrogen atmosphere at room temperature for 16 h. After the reaction was completed, the reaction mixture was filtered. The filtrate was concentrated under reduced pressure to remove the solvent (water bath: 40 °C) to give intermediate 2 (4.9 g, yield: 97%). MS m/z (ESI): 195.0[M+H].

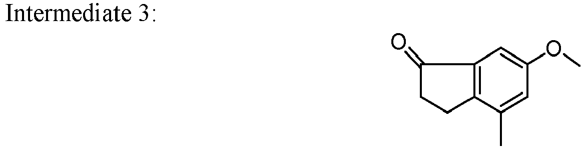

To a closed container were added dichloromethane (20 mL), intermediate 2 (2 g) and trifluoromethanesulfonic acid (4.6 g). The mixture was heated to 80 °C and stirred at that temperature for 16 h. After the reaction was completed, the reaction mixture was poured into ice water and extracted three times with dichloromethane (30 mL). The combined organic phases were dried over anhydrous sodium sulfate and filtered. The filtrate was concentrated under reduced pressure (water bath: 40 °C). The resulting residue was separated and purified by preparative TLC (petroleum ether:ethyl acetate = 10:1) to give intermediate 3 (300 mg, yield: 16%). MS m/z (ESI): 177.0 [M+H].

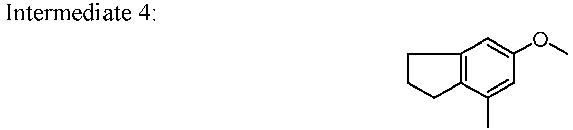

To a 50 mL single-neck flask were added diethyl ether (30 mL), aluminum chloride (1.7 g) and lithium aluminum hydride (494 mg). The temperature was lowered to 0 °C, and intermediate 3 (130 mg) was added. The mixture was naturally warmed to room temperature and stirred at that temperature for 16 h. After the reaction was completed, the reaction mixture was quenched by slowly adding 5 M sodium hydroxide solution and extracted three times with ethyl acetate (50 mL). The combined organic phases were washed with saturated brine, dried over anhydrous sodium sulfate and filtered. The filtrate was concentrated under reduced pressure to give intermediate 4 (yield: 47%). ¹H NMR (400 MHz, CDCl₃) *δ* 6.67 - 6.57 (m, 2H), 3.85 (s, 3H), 2.92 - 2.77 (m, 4H), 2.29 (s, 3H), 2.11 - 2.06 (m, 2H).

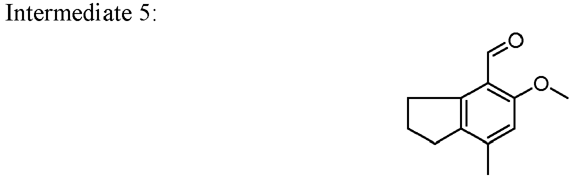

To a 50 mL single-neck flask was added a mixed solution of phosphorus oxychloride/*N*,*N-*dimethylformamide (9:10, 6 mL). The solution was stirred at room temperature under nitrogen atmosphere for 15 min, and then intermediate 4 (350 mg) was added. The mixture was heated to 60 °C and stirred at that temperature for 1 h. After the reaction was completed, the reaction mixture was poured into ice water and extracted three times with ethyl acetate (50 mL). The combined organic phases were dried over anhydrous sodium sulfate and filtered. The filtrate was concentrated under reduced pressure to remove the solvent (water bath: 40 °C). The residue was separated and purified by a silica gel column (petroleum ether:ethyl acetate = 10:1). The filtrate was concentrated under reduced pressure and separated and purified by Prep-HPLC (column: Gemini-C18 150 × 21.2 mm, 5 µm, mobile phase: acetonitrile-water (0.1% formic acid), gradient: 60-65%) to give intermediate 5 (20 mg, yield: 5%). MS m/z (ESI): 191.1 [M+H].

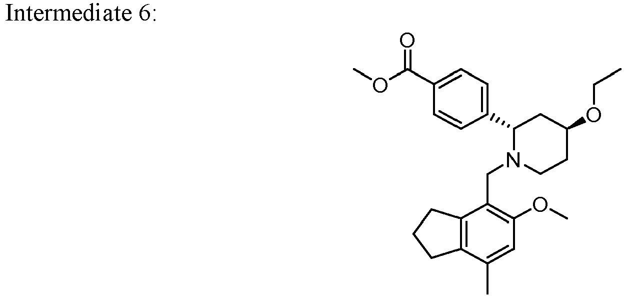

To a 25 mL microwave tube was added tetrahydrofuran (4 mL), intermediate 7 (20 mg) of Example 1, intermediate 5 (20 mg) and tetraethyl titanate (16 mg). The reaction system was heated to 70 °C under nitrogen atmosphere and reacted at that temperature for 16 h. Then the reaction system was cooled to room temperature. Sodium triacetoxyborohydride (44 mg) was added, and the reaction system was successively reacted for 1 h. After the reaction was completed, methanol was added until the solution became clear. The solution was purified by preparative TLC (petroleum ether:ethyl acetate = 2:1) to give intermediate 6 (30 mg, yield: 85%). MS m/z (ESI): 438.1 [M+H].

### Target compound:

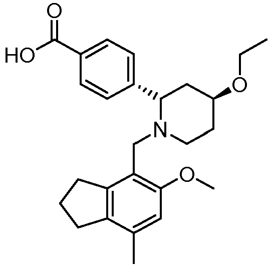

To a 50 mL single-neck flask were successively added a mixed solution of methanol/water (3:1, 4 mL), intermediate 6 (40 mg) and sodium hydroxide (73 mg). The mixture was reacted at room temperature for 16 h. After the reaction was completed, the reaction mixture was poured into water, adjusted to pH 7-8 with diluted hydrochloric acid and concentrated under reduced pressure (45 °C) to remove the organic solvent. The remaining aqueous phase was washed five times with a mixed solution of dichloromethane/methanol (10:1) (20 mL). Then the aqueous phase residue was separated and purified by Prep-HPLC (column: Gemini-C18 150 × 21.2 mm, 5 µm; mobile phase: acetonitrile-water (0.1% formic acid); gradient: 20-50%, UV: 214 nm) to give the target compound (5.3 mg, yield: 14%). MS m/z (ESI): 424.1 [M+H]. ¹H NMR (400 MHz, CD₃OD) δ 8.10 (d, *J* = 8.0 Hz, 2H), 7.57 (d, *J* = 8.0 Hz, 2H), 6.65 (s, 1H), 4.70 - 4.55 (m, 1H), 4.12 - 3.98 (m, 1H), 3.94 - 3.80 (m, 2H), 3.74 (s, 3H), 3.63 - 3.54 (m, 2H), 3.46 - 3.38 (m, 1H), 2.94 - 2.65 (m, 5H), 2.30 - 1.95 (m, 9H), 1.29 (t, J = 7.0 Hz, 3H).

### Example 9:

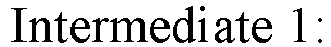

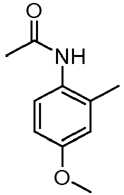

Acetic anhydride (8.9 g) was added to a solution of 4-methoxy-2-methylaniline (10 g) in dichloromethane (200 mL) at 0 °C. The mixture was reacted at room temperature under nitrogen atmosphere for 3 h. After the reaction was completed, the reaction mixture was washed successively with saturated sodium bicarbonate solution (100 mL) and saturated brine (100 mL). The organic phase was dried over anhydrous sodium sulfate and filtered. The filtrate was concentrated under reduced pressure to give intermediate 1 (13.3 g, yield: 91%). MS m/z (ESI): 180.1 [M+H].

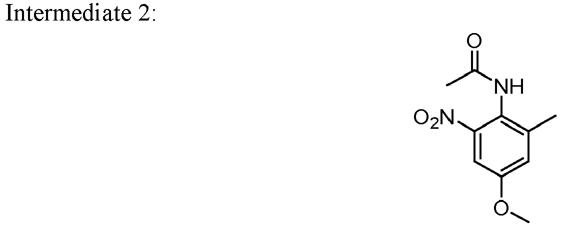

Concentrated nitric acid (8.2 g, 0.14 mol) was added dropwise to a solution of intermediate 1 (11.7 g) in acetic acid (300 mL) at 0 °C. The mixture was stirred at 20 °C for 3 h. After the reaction was completed, the reaction mixture was poured into water (500 mL) and extracted three times with ethyl acetate (300 mL). The combined organic phases were washed twice with saturated brine (300 mL), dried over anhydrous sodium sulfate and filtered. The filtrate was concentrated under reduced pressure. The resulting crude product was separated and purified by a silica gel column (petroleum ether:ethyl acetate = 1:1) to give intermediate 2 (5.2 g, yield: 33%). MS m/z (ESI): 225.0 [M+H]. 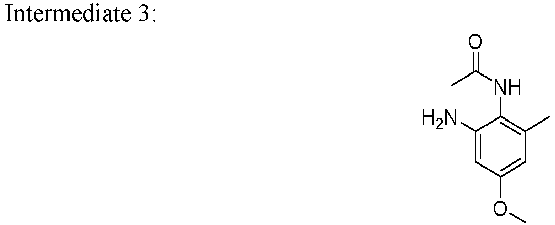 10% Pd/C (150 mg, 30% WT/WT) was added to a solution of intermediate 2 (480 mg) in methanol (15 mL) at room temperature. A catalytic hydrogenation reaction was carried out under hydrogen atmosphere at room temperature for 16 h. After the reaction was completed, the reaction mixture was filtered. The filtrate was concentrated under reduced pressure to give intermediate 3 (430 mg, purity: 80%, yield: 83%). MS m/z (ESI): 195.1 [M+H].

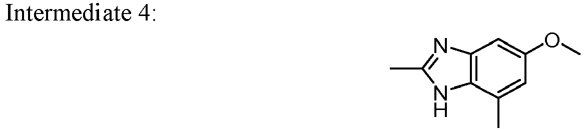

An aqueous solution of sodium hydroxide (15%, 10 mL) was added to a solution of intermediate 3 (430 mg) in ethanol (10 mL) at room temperature. The reaction system was stirred at 90 °C for 16 h. After the reaction was completed, the reaction mixture was directly concentrated under reduced pressure to remove ethanol. Water (20 mL) was added to the residual phase. The mixture was adjusted to pH 5-6 with 5 M hydrochloric acid and extracted five times with ethyl acetate (20 mL). The combined organic phases were washed with saturated brine (20 mL), dried over anhydrous sodium sulfate and filtered. The filtrate was concentrated under reduced pressure to give intermediate 4 (370 mg, yield: 94%). MS m/z (ESI): 177.1 [M+H].

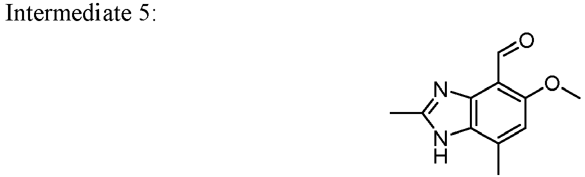

Urotropin (271 mg) was added to a solution of intermediate 4 (310 mg) in trifluoroacetic acid (10 mL) at room temperature. The reaction system was stirred at 80 °C for 3 h. After the reaction was completed, the reaction mixture was directly concentrated under reduced pressure. The crude product was separated and purified by a silica gel column (dichloromethane:methanol = 20:1) to give intermediate 5 (170 mg, yield: 47%). MS m/z (ESI): 205.1 [M+H].

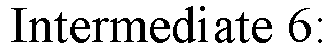

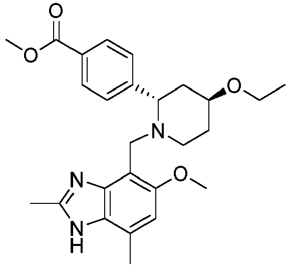

Sodium triacetoxyborohydride (330 mg) was added to a solution of intermediate 5 (159.30 mg) and intermediate 7 (137 mg) of Example 1 in 1,2-dichloroethane (20 mL) at room temperature. The mixture was stirred at room temperature for 16 h. After the reaction was completed, the reaction mixture was directly concentrated under reduced pressure. The residue was separated and purified by a silica gel column (dichloromethane:methanol = 20: 1) to give intermediate 6 (200 mg, yield: 85%). MS m/z (ESI): 451.8 [M+H].

### Target compound:

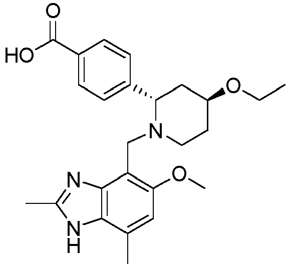

A solution of sodium hydroxide (176 mg) in water (2 mL) was added to a solution of intermediate 6 (200 mg) in methanol (6 mL) at room temperature. The reaction system was reacted at room temperature for 16 h. After the reaction was completed, methanol was removed by concentration under reduced pressure. The aqueous phase was adjusted to pH 5-6 with 5 M hydrochloric acid solution and then concentrated under reduced pressure. The residue was separated and purified by Prep-HPLC (column: Gemini-C18, 150 × 21.2 mm, 5 µm; mobile phase: acetonitrile-water (0.1% formic acid); gradient: 0-30%) to give the target compound (32 mg, yield: 17%, containing 0.7 equivalents of formic acid). MS m/z (ESI): 437.8 [M+H]. ¹H NMR (400 MHz, CD₃OD) δ 8.35 (bs, 0.7H), 8.15 (d, *J* = 8.0 Hz, 2H), 7.69 (d, *J* = 8.0 Hz, 2H), 6.80 (d, *J* = 4.2 Hz, 1H), 4.78 - 4.65 (m, 1H), 4.38 - 4.32 (m, 1H), 4.21 (d, *J* = 12.8 Hz, 1H), 3.89 - 3.82 (m, 1H), 3.77 (s, 3H), 3.67 - 3.55 (m, 3H), 3.32 - 3.22 (m, 1H), 2.62 (s, 3H), 2.52 (s, 3H), 2.29 - 2.27 (m, 2H), 2.08 - 2.00 (m, 2H), 1.32 (t, *J=* 7.0 Hz, 3H).

### Example 10:

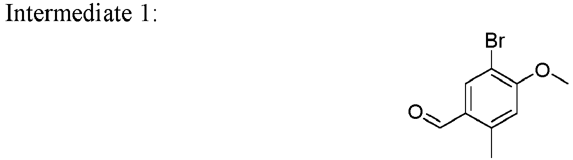

N-Bromosuccinimide (15.6 g) was added in batches to a solution of 2-methyl-4-methoxybenzaldehyde (12 g) in *N*,*N*-dimethylformamide (200 mL) at room temperature (0.5 h). The reaction system was stirred at room temperature overnight. After the reaction was completed, the reaction mixture was added with ethyl acetate (120 mL) and aqueous sodium bicarbonate solution (120 mL) for dilution. The organic phase was separated. The aqueous phase was extracted once with ethyl acetate (120 mL). The combined organic phases were washed with saturated brine (30 mL), dried over anhydrous sodium sulfate and filtered. The filtrate was concentrated under reduced pressure. The residue was separated and purified by silica gel column chromatography (petroleum ether/ethyl acetate = 20:1 to 5:1) to give intermediate 1 (13 g, yield: 71%). MS m/z (ESI): 228.9 [M+H].

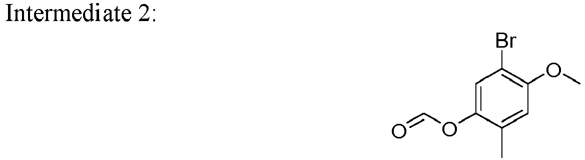

m-Chloroperoxybenzoic acid (16 g) was added in batches to a solution of intermediate 1 (10.7 g) in dichloromethane (150 mL) at room temperature. The reaction mixture was heated to 40 °C and stirred at that temperature overnight. After the reaction was completed, the reaction mixture was added with dichloromethane (100 mL) for dilution and washed three times with aqueous sodium bicarbonate solution (30 mL) and once with water (30 mL). The organic phase was dried over anhydrous sodium sulfate and filtered. The filtrate was concentrated under reduced pressure to give intermediate 2 (10 g, yield: 8%). MS m/z (ESI): 244.9 [M+H].

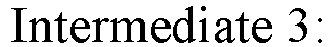

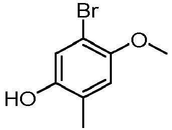

To a solution of intermediate 2 (10 g) in acetonitrile (150 mL) was added triethylamine (10 g). The reaction system was heated to 50 °C and stirred at that temperature for 6 h. After the reaction was completed, the reaction mixture was directly concentrated. The residue was separated and purified by silica gel column chromatography (petroleum ether:ethyl acetate = 10:1) to give intermediate 3 (6 g, yield: 80%). MS m/z (ESI): 216.9 [M+H].

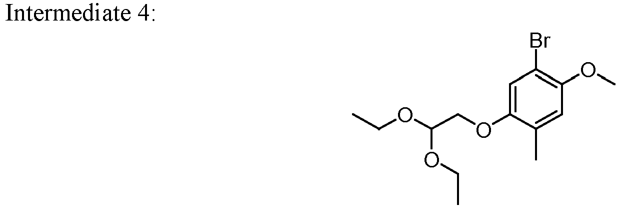

To a solution of intermediate 3 (6 g) and bromoacetaldehyde diethyl acetal (9.5 g) in *N,N-*dimethylformamide (80 mL) were added sodium iodide (0.5 g) and potassium carbonate (15 g). The mixture was heated to 100 °C and stirred at that temperature for 10 h. After the reaction was completed, the reaction mixture was added with ethyl acetate (200 mL) and water (200 mL) for dilution. The aqueous phase was extracted twice with ethyl acetate (100 mL). The combined organic phases were washed with saturated brine (50 mL), dried over anhydrous sodium sulfate and filtered. The filtrate was concentrated under reduced pressure. The residue was separated and purified by silica gel column chromatography (petroleum ether:ethyl acetate = 10:1) to give intermediate 4 (2.8 g, yield: 30%). MS m/z (ESI): 354.9 [M+Na].

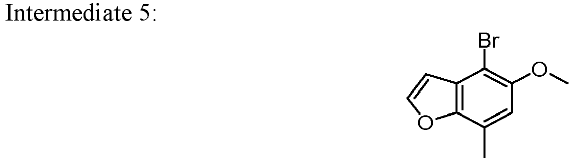

Polyphosphoric acid (4 g) was added to a solution of intermediate 4 (2.8 g) in toluene (50 mL). The reaction mixture was heated to 100 °C and stirred at that temperature for 6 h. After the reaction was completed, the reaction mixture was added with ethyl acetate (100 mL) and water (50 mL) for dilution. The aqueous phase was extracted once with ethyl acetate (50 mL). The combined organic phases were made neutral with aqueous sodium carbonate solution, dried over anhydrous sodium sulfate and filtered. The filtrate was concentrated under reduced pressure. The residue was separated and purified by silica gel column chromatography (petroleum ether:ethyl acetate = 15:1) to give intermediate 5 (1.5 g, yield: 74%). ¹H NMR (400 MHz, CDCl₃) *δ* 7.64 (d, *J* = 2.0 Hz, 1H), 6.75 - 6.78 (m, 2H), 3.92 (s, 3H), 3.49 (s, 3H).

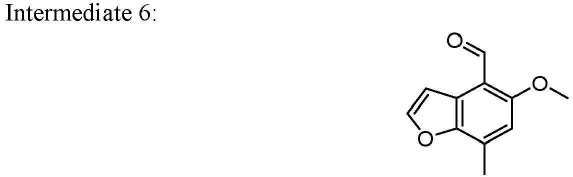

A solution of intermediate 5 (1.07 g) in tetrahydrofuran (10 mL) was cooled to -78 °C under nitrogen atmosphere, and *tert*-butyllithium solution (1.3 M, 7.5 mL) was slowly added dropwise. After the dropwise addition was completed, the mixture was stirred at -78 °C for 1 h. *N,N-*Dimethylformamide (1 g) was added. Then the reaction mixture was naturally warmed and stirred at room temperature overnight. After the reaction was completed, the reaction mixture was added with aqueous ammonium chloride solution (10 mL) and ethyl acetate (20 mL) for dilution, and the phases were separated. The organic phase was dried over anhydrous sodium sulfate and filtered. The filtrate was concentrated under reduced pressure. The residue was separated and purified by silica gel column chromatography (petroleum ether:ethyl acetate = 10:1) to give intermediate 6 (300 mg, yield: 36%). ¹H NMR (400 MHz, CDCl₃) *δ* 10.58 (s, 1H), 7.72 (d, *J* = 2.0 Hz, 1H), 7.53 (d, *J* = 2.0 Hz, 1H), 6.75 (s, 1H), 3.95 (s, 3H), 2.58 (s, 3H).

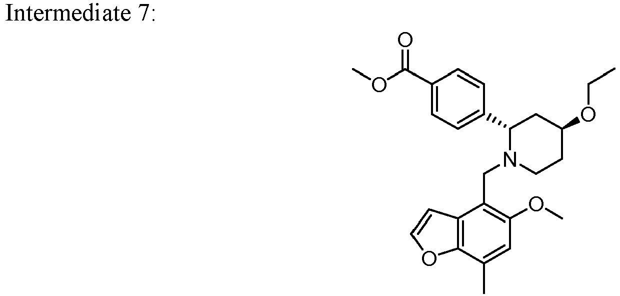

To a solution of 1,2-dichloroethane (2 mL) were successively added intermediate 6 (150 mg) and intermediate 7 (150 mg) of Example 1. The reaction system was stirred at room temperature under nitrogen atmosphere for 8 h. Then sodium triacetoxyborohydride (360 mg) was added, and the mixture was successively reacted at room temperature overnight. After the reaction was completed, the reaction mixture was quenched by adding water (10 mL) and added with ethyl acetate (20 mL) for dilution. The organic phase was dried over anhydrous sodium sulfate and filtered. The filtrate was concentrated under reduced pressure. The residual phase was separated and purified by silica gel column chromatography (petroleum ether:ethyl acetate = 5:1) to give intermediate 7 (100 mg, yield: 40%). MS m/z (ESI): 438 [M+H].

### Target compound:

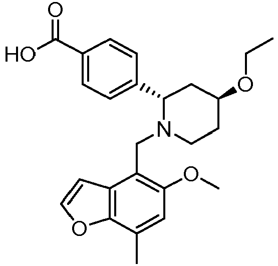

To a mixed solution of intermediate 7 (100 mg) in methanol/tetrahydrofuran/water (3 mL, 1:1:1) was added lithium hydroxide (100 mg). The mixture was stirred at room temperature overnight. After the reaction was completed, the reaction mixture was directly concentrated. The residue was purified by Prep-HPLC (column: Gemini-C18, 150 × 21.2 mm, 5 µm; mobile phase: acetonitrile-water (0.1% formic acid); gradient: 25-40%). The resulting product solution was concentrated to remove the solvent. The remaining small amount of aqueous solution was lyophilized to give the target compound (50 mg, yield: 51%, containing 0.5 equivalents of formic acid). MS m/z (ESI): 424.1 [M+H]. ¹H NMR (400 MHz,CD₃OD) δ 8.41 (s, 0.5H), 8.16 (d, *J* = 8.4 Hz, 2H), 7.82 (d, *J* = 2.0 Hz, 1H), 7.67 (d, *J* = 8.4 Hz, 2H), 6.89 (s, 1H), 6.79 (s, 1H), 4.60 - 4.72 (m, 1H), 4.26 (d, J = 12.8 Hz, 1H), 4.10 (d, *J* = 12.8 Hz, 1H), 3.87 - 3.82 (m, 1H), 3.79 (s, 3H), 3.66 - 3.56 (m, 2H), 3.54 - 3.42 (m, 1H), 3.31 - 3.22 (m, 1H), 2.51 (s, 3H), 2.32 - 2.20 (m, 2H), 2.15 - 1.97 (m, 2H), 1.32 (t, *J* = 6.8 Hz, 3H).

### Example 11:

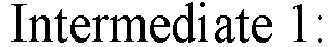

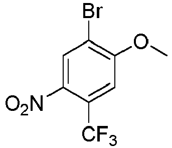

Potassium nitrate (4.35 g) was added to a solution of 1-bromo-2-methoxy-4-(trifluoromethyl)benzene (10 g) in sulfuric acid (40 mL) at 0 °C. Then the reaction system was naturally warmed to room temperature and stirred at that temperature for 1 h. After the reaction was completed, the reaction mixture was poured into ice-water (200 mL) and extracted three times with ethyl acetate (200 mL). The combined organic phases were washed three times with saturated brine (50 mL) and once with saturated aqueous sodium bicarbonate solution (50 mL), dried over anhydrous sodium sulfate and filtered. The filtrate was concentrated under reduced pressure to give intermediate 1 (10 g, yield: 90%). The crude product was directly used in the next step.

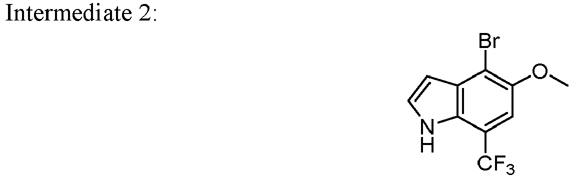

Vinylmagnesium bromide (150 mL) was slowly added dropwise to a solution of intermediate 1 (10 g) in tetrahydrofuran (400 mL) at -78 °C. The mixture was reacted at -78 °C for 2 h. After the reaction was completed, the reaction system was quenched by adding saturated aqueous ammonium chloride solution (100 mL) and extracted three times with ethyl acetate (800 mL). The combined organic phases were washed once with saturated brine (200 mL), dried over anhydrous sodium sulfate and filtered. The filtrate was concentrated under reduced pressure. The residue was separated and purified by a silica gel column (petroleum ether:ethyl acetate = 10: 1) to give intermediate 2 (1.5 g, yield: 15%). MS m/z (ESI): 293.9 [M+H].

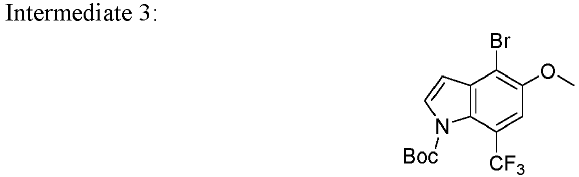

4-Dimethylaminopyridine (372 mg) was added to a solution of intermediate 2 (600 mg) and Boc₂O anhydride (664 mg) in dichloromethane (20 mL). The mixture was stirred at room temperature for 2 h. After the reaction was completed, the reaction mixture was directly concentrated under reduced pressure. The residue was separated and purified by a silica gel column (petroleum ether:ethyl acetate = 10:1) to give intermediate 3 (700 mg, yield: 85%). ¹H NMR (400 MHz, DMSO- *d₆*) *δ* 7.86 (d, *J* = 3.8 Hz, 1H), 7.40 (s, 1H), 6.73 (d, *J* = 3.8 Hz, 1H), 3.97 (s, 3H), 1.58 (s, 9H).

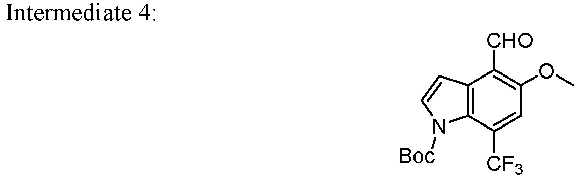

tert-Butyllithium (1.68 mL) was slowly added dropwise to a solution of intermediate 3 (400 mg) in tetrahydrofuran (5 mL) at -78 °C. After the mixture was stirred at -78 °C for 1.5 h, a solution of *N*,*N*--dimethylformamide (110 mg) in tetrahydrofuran (15 mL) was added dropwise. After the dropwise addition was completed, the reaction system was slowly warmed to room temperature and stirred at that temperature for 1.5 h. After the reaction was completed, the reaction mixture was quenched by adding saturated aqueous ammonium chloride solution (20 mL) and extracted three times with ethyl acetate (50 mL). The combined organic phases were washed with saturated brine (50 mL), dried over anhydrous sodium sulfate and filtered. The filtrate was concentrated under reduced pressure. The residue was separated and purified by a silica gel column (petroleum ether:ethyl acetate = 5:1) to give intermediate 4 (30 mg, yield: 8%). ¹H NMR (400 MHz, CDCl₃) *δ* 10.71 (s, 1H), 7.66 (d, *J* = 3.6 Hz, 1H), 7.51 (d, *J* = 3.6 Hz, 1H), 7.24 (s, 1H), 4.02 (s, 3H), 1.63 (s, 9H).

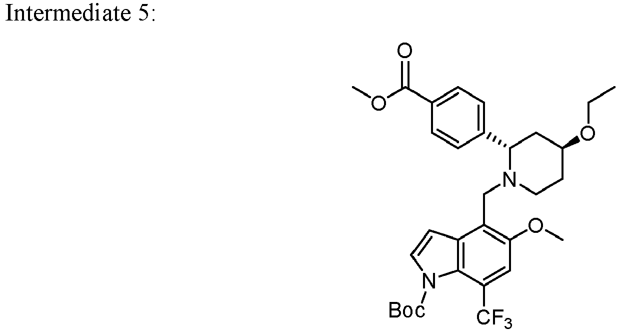

Intermediate 7 (100 mg) and intermediate 4 (131.21 mg) of Example 1 were successively added to a solution of 1,2-dichloroethane (5 mL) at room temperature. The mixture was reacted at room temperature for 16 h. Then borohydride triacetate (43.12 mg) was added, and the mixture was successively stirred at room temperature for 16 h. After the reaction was completed, the reaction mixture was directly concentrated under reduced pressure and separated and purified by preparative TLC (dichloromethane:methanol = 5:1) to give intermediate 5 (30 mg, yield: 12.8%). MS m/z (ESI): 591.1 [M+H].

### Target compound:

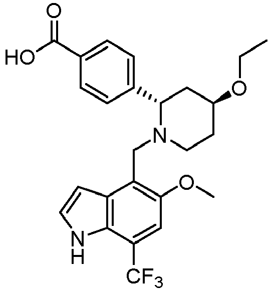

Sodium hydroxide (11.99 mg) was added to a solution of intermediate 5 (60 mg) in methanol (1 mL) and water (1 mL). The reaction system was heated to 75 °C and stirred at that temperature for 3 h. After the reaction was completed, the reaction mixture was directly concentrated under reduced pressure. The residue was separated and purified by Prep-HPLC (column: Xbridge-C18 150 × 19 mm, 5 µm; mobile phase: acetonitrile-water (0.1% formic acid); gradient: 20-40%) to give the target compound (18.8 mg, yield: 31.3%). MS m/z (ESI): 477.1 [M+H]. ¹H NMR (400 MHz,CD₃OD) δ 8.15 (d, *J* = 8.0 Hz, 2H), 7.66 (d, *J=* 8.0 Hz, 2H), 7.44 (d, *J* = 3.2 Hz, 1H), 7.17 (s, 1H), 6.52 (s, 1H), 4.67 - 4.56 (m, 1H), 4.36 - 4.15 (m, 2H), 3.87 - 3.77 (m, 4H), 3.64 - 3.55 (m, 2H), 3.50 - 3.40 (m, 1H), 3.24 - 3.17 (m, 1H), 2.28 - 2.19 (m, 2H), 2.04 - 2.00 (m, 2H), 1.30 (t, *J* = 6.8 Hz, 3H).

### Example 12:

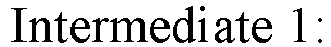

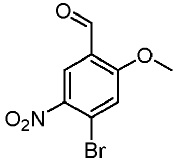

4-Bromo-2-methoxy-benzaldehyde (11 g) was added to a 250 mL single-neck flask, and sulfuric acid (100 mL) was added. Potassium nitrate (5.5 g) was slowly added to the reaction system with cooling in an ice bath. The ice bath was removed, and the mixture was successively reacted for 1 h. After the reaction was completed, the reaction mixture was slowly poured into ice water and extracted twice with ethyl acetate (200 mL). The organic phase was washed with saturated aqueous sodium bicarbonate solution (100 mL), washed three times with saturated brine (50 mL), dried over anhydrous sodium sulfate and filtered. The filtrate was concentrated under reduced pressure to give intermediate 1 (12 g, yield: 86%). ¹H NMR (400 MHz, CD₃OD) *δ* 10.24 (s, 1H), 8.29 (s, 1H), 7.78 (s, 1H), 4.07 (s, 3H).

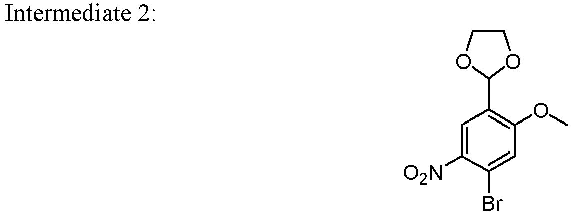

To a 500 mL single-neck flask were successively added toluene (200 mL), ethylene glycol (30 g), intermediate 1 (10 g) andp-toluenesulfonic acid (0.76 g). The reaction system was heated to 120 °C and reacted at that temperature for 4 h. After the reaction was completed, the solvent was removed by concentration. Water (500 mL) and ethyl acetate (500 mL) were added for dilution, and the phases were separated. The organic phase was dried over anhydrous sodium sulfate and filtered. The filtrate was concentrated under reduced pressure. The residual phase was separated and purified by a silica gel column (petroleum ether:ethyl acetate = 3:1) to give intermediate 2 (10 g, 81%). MS m/z (ESI): 304.0 [M+H].

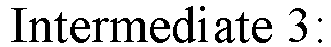

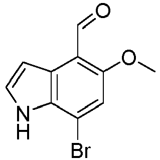

A solution of intermediate 2 (3 g) in tetrahydrofuran (100 mL) was cooled to -30 °C, and vinylmagnesium bromide (1 M, 60 mL) was slowly added dropwise to the reaction system. The mixture was stirred at that temperature for 3 h and then naturally warmed to 0 °C. After the reaction was completed, the reaction was quenched by adding saturated aqueous ammonium chloride solution (200 mL). The reaction mixture was extracted twice with ethyl acetate (500 mL). The combined organic phases were washed with saturated brine (100 mL), dried over anhydrous sodium sulfate and filtered. The filtrate was concentrated. The residual phase was separated and purified by a silica gel column (dichloromethane) to give intermediate 3 (1.2 g, yield: 45%). MS m/z (ESI): 253.8 [M+H].

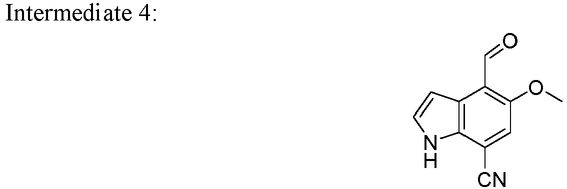

To a 10 mL microwave tube were successively added *N,N*-dimethylformamide (4 mL), intermediate 3 (340 mg), zinc cyanide (230 mg) and tetrakis(triphenylphosphine)palladium(0) (115 mg). The reaction system was heated to 130 °C under nitrogen atmosphere and reacted under microwaves for 1.5 h. After the reaction was completed, the reaction mixture was directly separated and purified by a silica gel column (petroleum ether:ethyl acetate = 10:1) to give intermediate 4 (220 mg, yield: 78%). MS m/z (ESI): 201.1 [M+H].

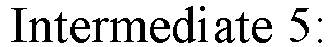

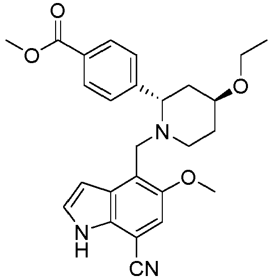

Intermediate 4 (130 mg) and intermediate 7 (110 mg) of Example 1 were successively added to 1,2-dichloroethane (3 mL). The mixture was stirred at room temperature for 8 h. Then sodium triacetoxyborohydride (440 mg) was added to the reaction system, and the mixture was successively reacted at room temperature for 16 h. After the reaction was completed, the reaction mixture was directly separated and purified by a silica gel column (methanol:dichloromethane = 1:20) to give intermediate 5 (200 mg, 41%). MS m/z (ESI): 447.9 [M+H].

### Target compound:

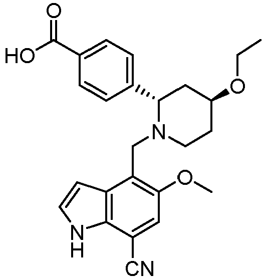

To a 25 mL single-neck flask were successively added methanol (3 mL), water (3 mL), intermediate 5 (100 mg) and sodium hydroxide (40 mg). The reaction system was heated to 75 °C and reacted at that temperature for 3 h. After the reaction was completed, the reaction mixture was directly separated and purified by Prep-HPLC (column: Gemini-C18, 150 × 21.2 mm, 5 µm; mobile phase: acetonitrile-water (0.1% formic acid); gradient: 15-45%). The resulting solution was concentrated. The remaining small amount of aqueous solution was lyophilized to give the target compound (60.2 mg, yield: 62.5%, containing 0.3 equivalents of formic acid). MS m/z (ESI): 434.1 [M+H]. ¹H NMR (400 MHz, CD₃OD) δ 8.42 (s, 0.3H), 8.11 (d, *J* = 8.0 Hz, 2H), 7.63 (d, *J* = 8.0 Hz, 2H), 7.46 (d, *J* = 3.2 Hz, 1H), 7.28 (s, 1H), 6.57 (s, 1H), 4.53 - 4.38 (m, 1H), 4.25 - 4.02 (m, 2H), 3.82 - 3.76 (m, 4H), 3.62 - 3.54 (m, 2H), 3.30 - 3.23 (m, 1H), 3.14 - 3.04 (m, 1H), 2.23 - 1.87 (m, 4H), 1.29 (t, *J* = 7.0 Hz, 3H).

### Example 13:

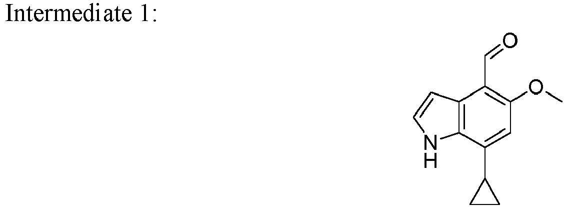

To a solution of intermediate 3 (500 mg) of Example 13 in dioxane (9 mL) were successively added water (1 mL), cyclopropylboronic acid (200 mg), sodium carbonate (500 mg) and Pd(dppf)Cl₂ dichloromethane (80 mg). The mixture was heated to 90 °C and stirred at that temperature for 16 h. After the reaction was completed, ethyl acetate (20 mL) was directly added for dilution. Silica gel was added and stirred, and then the mixture was separated and purified by a silica gel column (ethyl acetate:petroleum ether = 1: 10) to give intermediate 1 (340 mg, yield: 74%). MS m/z (ESI): 215.9 [M+H].

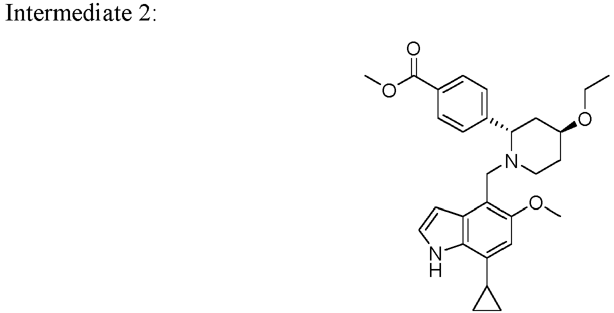

To a 25 mL single-neck flask were successively added 1,2-dichloroethane (3 mL) as a solvent, intermediate 1 (150 mg) and intermediate 7 (130 mg) of Example 1 at room temperature. After the reaction was carried out at room temperature for 8 h, sodium triacetoxyborohydride (450 mg) was added, and the mixture was successively reacted at room temperature for 16 h. After the reaction was completed, silica gel was directly added to the reaction mixture and stirred, and the mixture was separated and purified by a silica gel column (methanol:dichloromethane = 1:20) to give intermediate 2 (100 mg, 28%). MS m/z (ESI): 462.8 [M+H].

### Target compound:

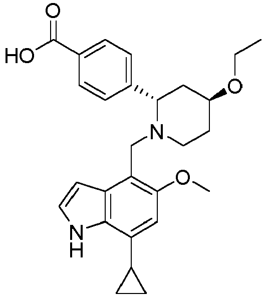

To a 25 mL single-neck flask were successively added methanol (2 mL), water (2 mL), intermediate 2 (100 mg) and sodium hydroxide (50 mg) at room temperature. The reaction system was heated to 40 °C and stirred at that temperature for 3 h. After the reaction was completed, the reaction mixture was directly purified by Prep-HPLC (column: Gemini-C18, 150 × 21.2 mm, 5 µm; mobile phase: acetonitrile-water (0.1% formic acid); gradient: 15-35%). The resulting solution was concentrated. The remaining small amount of aqueous solution was lyophilized to give the target compound (20.1 mg, yield: 16%, containing 0.8 equivalents of formic acid). MS m/z (ESI): 448.9 [M+H]. ¹H NMR (400 MHz, CD₃OD) δ 8.43 (bs, 0.8H), 8.17 (d, *J* = 8.0 Hz, 2H), 7.67 (d, *J* = 8.0 Hz, 2H), 7.36 (d, *J* = 2.8 Hz, 1H), 6.54 (s, 1H), 6.37 (d, *J* = 2.8 Hz, 1H), 4.80 - 4.71 (m, 1H), 4.37 - 4.19 (m, 2H), 3.87 - 3.81 (m, 1H), 3.76 (s, 3H), 3.65 - 3.48 (m, 3H), 3.42 - 3.36 (m, 1H), 2.30 - 1.94 (m, 5H), 1.33 (t, *J* = 7.2 Hz, 3H), 1.11 - 1.03 (m, 2H), 0.82 - 0.75 (m, 2H).

### Example 14:

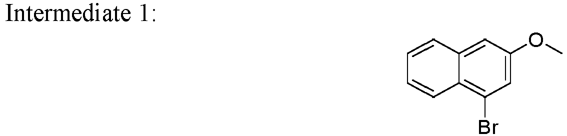

To a solution of 4-bromonaphthalen-2-ol (970 mg) and iodomethane (1.23 g) in *N,N-*dimethylformamide (10 mL) was slowly added sodium hydride (349 mg) at room temperature. The reaction system was stirred under nitrogen atmosphere at room temperature for 2 h. After the reaction was completed, the reaction mixture was poured into water (5 mL) to quench the reaction and extracted three times with ethyl acetate (100 mL). The combined organic phases were washed once with saturated brine (20 mL), dried over anhydrous sodium sulfate and filtered. The filtrate was concentrated under reduced pressure. The residue was purified by column chromatography (petroleum ether:ethyl acetate = 100:1) to give intermediate 1 (920 mg, yield: 89%). MS m/z (ESI): 236.9 [M+H]

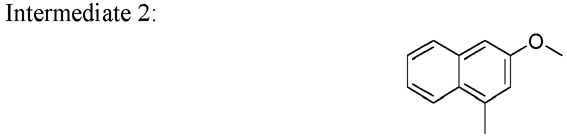

To a 100 mL single-neck flask were successively added *N,N*-dimethylformamide (12 mL), intermediate 1 (1.05 g), trimethylcyclotriboroxane (3.3 g), cesium carbonate (2.87 g) and tetrakis(triphenylphosphine)palladium(0) (254 mg). The reaction system was heated to 90 °C under nitrogen atmosphere and stirred at that temperature for 16 h. After the reaction was completed, the reaction mixture was cooled to room temperature, poured into water (50 mL) and extracted three times with ethyl acetate (100 mL). The combined organic phases were washed with saturated brine, dried over anhydrous sodium sulfate and filtered. The filtrate was concentrated under reduced pressure. The residue was separated and purified by a silica gel column (petroleum ether:ethyl acetate = 5:1) to give intermediate 2 (680 mg, yield: 80%). MS m/z (ESI): 173.1 [M+H].

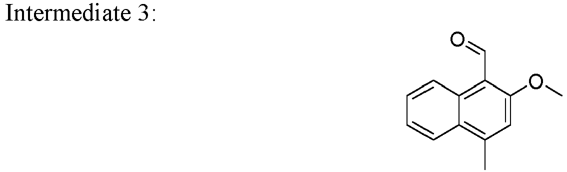

To a 100 mL single-neck flask were successively added *N,N*-dimethylformamide (4.2 mL) and phosphorus oxychloride (3.8 mL). The reaction system was stirred under nitrogen atmosphere at room temperature for 15 min. Then intermediate 2 (660 mg) was added, and the mixture was heated to 60 °C and successively stirred at that temperature for 1 h. After the reaction was completed, the reaction mixture was poured into ice water (30 mL) and extracted three times with ethyl acetate (100 mL). The combined organic phases were washed with saturated brine, dried over anhydrous sodium sulfate and filtered. The filtrate was concentrated under reduced pressure. The residue was separated and purified by a silica gel column (petroleum ether:ethyl acetate = 10:1) to give intermediate 3 (430 mg, yield: 56%). MS m/z (ESI): 201.1 [M+H].

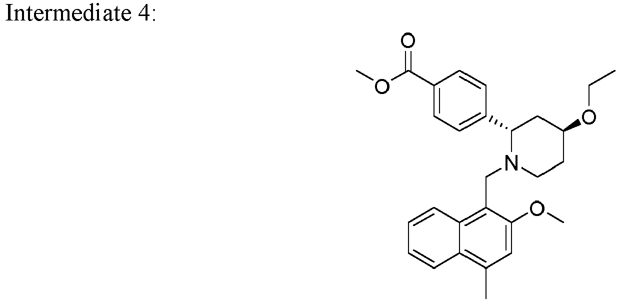

To a 50 mL single-neck flask were successively added 1,2-dichloroethane (4 mL), intermediate 3 (100 mg), intermediate 7 (197 mg) of Example 1 and sodium triacetoxyborohydride (318 mg). The reaction system was stirred under nitrogen atmosphere at room temperature for 16 h. After the reaction was completed, the reaction mixture was directly concentrated under reduced pressure. The residue was separated and purified by a silica gel column (petroleum ether:ethyl acetate = 10:1) to give intermediate 4 (120 mg, yield: 53%). MS m/z (ESI): 448.1 [M+H].

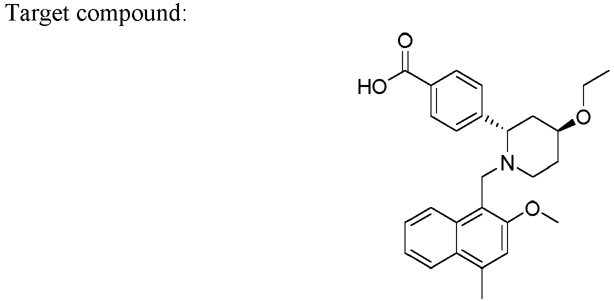

To a 50 mL single-neck flask were successively added methanol (3 mL), water (1 mL), intermediate 4 (120 mg) and sodium hydroxide (214 mg). The reaction system was stirred at room temperature for 16 h. After the reaction was completed, the reaction mixture was added with water (5 mL) for dilution and adjusted to pH 7-8 with 1 M hydrochloric acid. Then the mixture was directly concentrated under reduced pressure. The residue was separated and purified by Prep-HPLC (column: Gemini-C18 150 × 21.2 mm, 5 µM; mobile phase: acetonitrile-water (0.1% formic acid); gradient: 20-40%; UV: 214 nm) to give the target compound (93.5 mg, yield: 79%). MS m/z (ESI): 434.0 [M+H]. ¹H NMR (400 MHz, DMSO- *d₆*) δ 12.89 (s, 1H), 8.00 (d, *J* = 8.0 Hz, 2H), 7.96 (d, *J* = 8.4 Hz, 1H), 7.89 (d, *J* = 8.4 Hz, 1H), 7.69 (d, *J* = 8.0 Hz, 2H), 7.45 (t, *J* = 7.2 Hz, 1H), 7.35 (t, *J* = 7.2 Hz, 1H), 7.26 (s, 1H), 3.87 (s, 3H), 3.63 - 3.50 (m, 4H), 3.43 (q, *J* = 7.2 Hz, 2H), 2.63 (s, 3H), 2.44 - 2.30 (m, 2H), 1.87 - 1.77 (m, 2H), 1.70 - 1.62 (m, 1H), 1.47 - 1.37 (m, 1H), 1.68 (t, *J* = 7.0 Hz, 3H).

### Example 15:

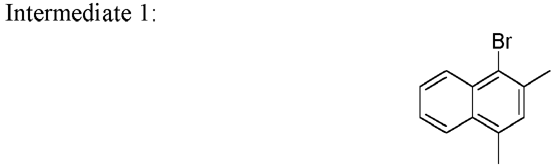

N-Bromosuccinimide (125 mg) was added to a solution of 1,3-dimethylnaphthalene (100 mg) in acetonitrile (6 mL). The reaction system was stirred at room temperature for 16 h. After the reaction was completed, the reaction mixture was directly concentrated under reduced pressure. The residue was separated and purified by a silica gel column (petroleum ether:ethyl acetate = 20:1) to give intermediate 1 (160 mg, yield: 85%). GC-MS: 234.0, 236.0 (MS).

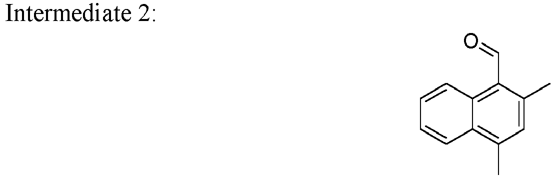

n-Butyllithium (0.37 mL) was added slowly dropwise to a solution of intermediate 1 (160 mg) in tetrahydrofuran (5 mL) at -78 °C. After the mixture was stirred at -78 °C for 1 h, *N,N-*dimethylformamide (99 mg) was slowly added dropwise to the reaction mixture. Then the reaction mixture was naturally warmed to room temperature and stirred at that temperature for 16 h. After the reaction was completed, the reaction mixture was poured into water (20 mL) and extracted twice with ethyl acetate (15 mL). The combined organic phases were washed three times with saturated brine (15 mL), dried over anhydrous sodium sulfate and filtered. The filtrate was concentrated under reduced pressure to give intermediate 2 (120 mg, yield: 95%). MS m/z (ESI): 185.1 [M+H].

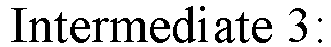

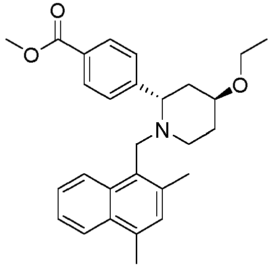

Tetraethyl titanate (118 mg) was added slowly to a solution of intermediate 2 (100 mg) and intermediate 7 (142 mg) of Example 1 in tetrahydrofuran (10 mL) at room temperature. The mixture was heated to 70 °C and stirred at that temperature for 16 h. After the reaction was naturally cooled to room temperature, sodium triacetoxyborohydride (343 mg) was added. The mixture was heated to 70 °C and successively stirred at that temperature for 1 h. After the reaction was completed, the reaction mixture was directly concentrated under reduced pressure. The residue was separated and purified by a silica gel column (petroleum ether:ethyl acetate = 2:1) to give intermediate 3 (30 mg, yield: 13%). MS m/z (ESI): 431.9 [M+H].

### Target compound:

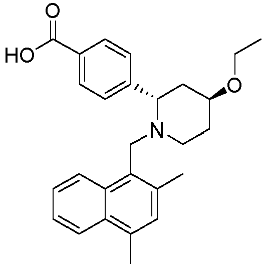

To a solution of intermediate 3 (30 mg) in methanol (4 mL) was added a solution of sodium hydroxide (32 mg) in water (1 mL). The reaction system was stirred at room temperature for 16 h. After the reaction was completed, methanol was removed by concentration under reduced pressure. The aqueous phase was adjusted to pH 5-6 with 5 M hydrochloric acid solution. Then the mixture was concentrated under reduced pressure. The residue was separated and purified by Prep-HPLC (column: Gemini-C18 150 × 21.2 mm, 5 µm; mobile phase: acetonitrile-water (0.1% formic acid); gradient: 5-30%) to give the target compound (15 mg, yield: 50%). MS m/z (ESI): 417.8 [M+H]. ¹H NMR (400 MHz, CD₃OD) δ 8.27 (d, *J* = 7.8 Hz, 2H), 8.08 - 8.02 (m, 1H), 7.87 - 7.80 (m, 2H), 7.58 - 7.50 (m, 3H), 7.29 (s, 1H), 4.80 - 4.65 (m, 1H), 4.55 - 4.38 (m, 1H), 3.84 (s, 1H), 3.71 - 3.49 (m, 3H), 3.17 - 3.09 (s, 1H), 2.65 (s, 3H), 2.54 - 2.22 (m, 6H), 2.10 - 1.85 (m, 2H), 1.30 (t, *J* = 7.0 Hz, 3H).

### Example 16:

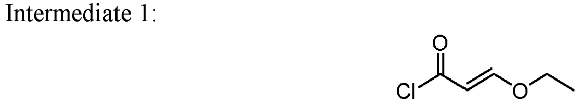

To a solution of ethyl 3,3-diethoxypropionate (20 g) in water (50 mL) was added sodium hydroxide (5 g) at room temperature. The mixture was heated to 110 °C and stirred at that temperature for 1 h. Then the reaction system was naturally cooled to room temperature, added with water (2 L) for dilution and extracted with ethyl acetate (1 L). The extract phase was washed with saturated brine (1 L), dried over anhydrous sodium sulfate and filtered. The filtrate was concentrated, and thionyl chloride (30 mL) was directly added to the resulting crude product. The mixture was heated to 70 °C and successively stirred at that temperature for one hour. After the reaction was completed, the reaction mixture was directly concentrated under reduced pressure to give intermediate 1 as a brown solid (14 g, yield: 90%).

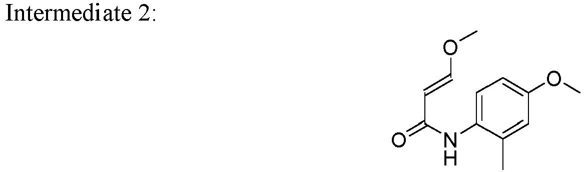

A solution of intermediate 1 (14 g) in dichloromethane (200 mL) was slowly added to a solution of 2-methyl-4-methoxyaniline (14 g) and pyridine (16 g) in dichloromethane (200 mL) under nitrogen atmosphere with cooling in an ice bath. The mixture was stirred at room temperature for 16 h. After the reaction was completed, water (500 mL) was added to quench the reaction, followed by the extraction with dichloromethane (500 mL). The extract phase was washed with water (500 mL), dried over anhydrous sodium sulfate and filtered. The filtrate was concentrated and purified by a silica gel column (dichloromethane:methanol = 10:1) to give intermediate 2 as a yellow oil (12 g, yield: 50%). MS m/z (ESI): 236.1 [M+H].

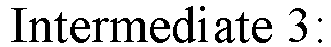

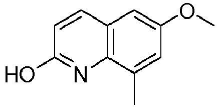

Intermediate 2 (12 g) was added to a solution of sulfuric acid (50 mL) with cooling in an ice bath. The mixture was stirred at room temperature for 1 h. After the reaction was completed, the reaction mixture was poured into ice water (200 mL), and the solid that precipitated was collected by filtration, washed with water and dried to give intermediate 3 as a brown solid (3 g, yield: 30%). MS m/z (ESI): 190.1 [M+H].

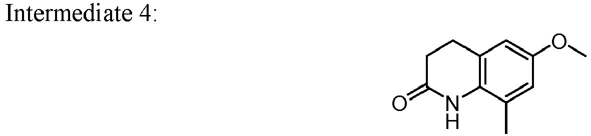

To a solution of intermediate 3 (600 mg) in acetic acid (10 mL) was added Pd/C (34 mg). A catalytic hydrogenation reaction was carried out at 90 °C under a balloon with hydrogen for 32 h. After the reaction was completed and cooled to room temperature, the reaction mixture was filtered. The filtrate was concentrated under reduced pressure. The residue was purified by a silica gel column (petroleum ether:ethyl acetate = 5:1) to give intermediate 4 as a yellow solid (100 mg, yield: 16%). MS m/z (ESI): 192.0 [M+H].

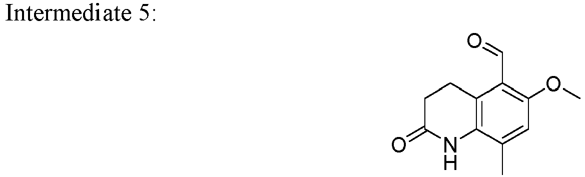

Titanium tetrachloride (1.5 g) was added to a solution of intermediate 4 (500 mg) in dichloromethane (3 mL) at -78 °C under nitrogen atmosphere. After the reaction mixture was stirred for 5 min, dichloromethyl ether (900 mg, 7.80 mmol) was slowly added. The reaction mixture was naturally warmed to room temperature and successively stirred at room temperature for 16 h. After the reaction was completed, the reaction mixture was diluted with dichloromethane (20 mL), washed with (5 mL), dried over Na₂SO₄ and filtered. The filtrate was concentrated. The resulting residue was purified by a silica gel column (dichloromethane:methanol = 10:1) to give intermediate 5 as a yellow solid (35 mg, yield: 7%). MS m/z (ESI): 220.0[M+H].

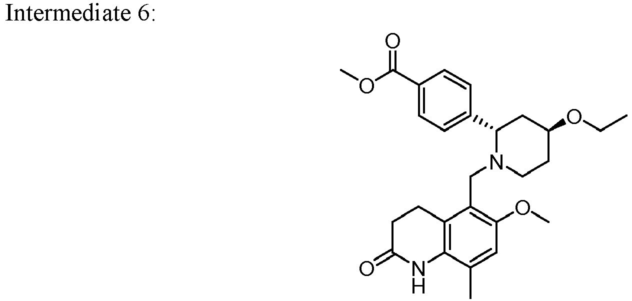

Intermediate 7 (42 mg) of Example 1 was added to a solution of intermediate 5 (35 mg) in 1,2-dichloroethane (5 mL) under nitrogen atmosphere. After the reaction mixture was stirred at room temperature for 16 h, sodium borohydride acetate (18.15 mg) was added, and the mixture was successively stirred at room temperature for 16 h. After the reaction was completed, the reaction mixture was directly concentrated. The residue was purified by a silica gel column (dichloromethane:methanol = 10: 1) to give intermediate 6 as a yellow solid (30 mg, yield: 86%). MS m/z (ESI): 467.2[M+H].

### Target compound:

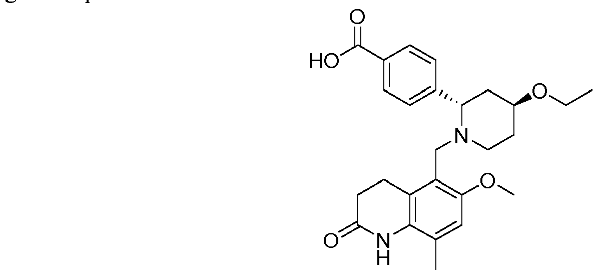

To a 50 mL single-neck flask were successively added methanol (1 mL), water (1 mL), intermediate 6 (30 mg), and sodium hydroxide (7.7 mg). The reaction mixture was stirred at room temperature for 3 h. After the reaction was completed, diluted hydrochloric acid (1 M, 0.5 mL) was added to the reaction mixture with cooling in an ice bath to make it neutral. Then the mixture was directly concentrated under reduced pressure. The resulting residue was purified by preparative high-pressure liquid chromatography (column: -Xbridge-C18 150 × 19 mm, 5 µm; mobile phase: acetonitrile-water (0.1% formic acid); gradient: 20-40%) to give the target compound as a white solid (1.1 mg, yield: 3.66%, containing 0.4 equivalents of formic acid). ¹H NMR (400 MHz, CD₃OD) *δ* 8.48 (bs, 1H), 8.07 (d, *J* = 8.0 Hz, 2H), 7.56 (d, *J* = 8.0 Hz, 2H), 6.74 (s, 1H), 4.40 - 4.30 (m, 1H), 3.92 - 3.83 (m, 2H), 3.82 - 3.76 (m, 1H), 3.72 (s, 3H), 3.60 - 3.52 (m, 2H), 3.15 - 2.87 (m, 3H), 2.81 - 2.70 (m, 1H), 2.50 - 2.41 (m, 2H), 2.25 (s, 3H), 2.17 - 2.11 (m, 2H), 2.06 - 1.96 (m, 2H), 1.27 (t, *J* = 7.0 Hz, 3H). MS m/z (ESI): 453.1 [M+H].

**The following examples were prepared according to the methods described above.**

| | | |
|---|---|---|
| 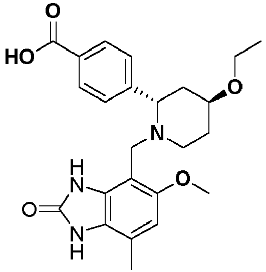 MS m/z (ESI): 440.2 [M+H] | | 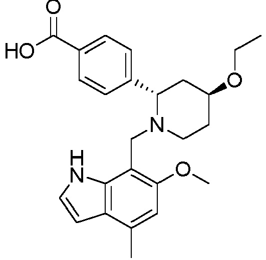 MS m/z (ESI): 423.2 [M+H] |
| 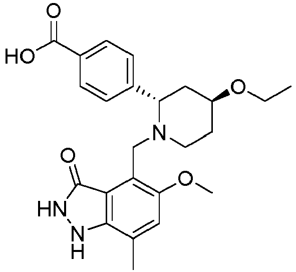 MS m/z (ESI): 440.2 [M+H] | 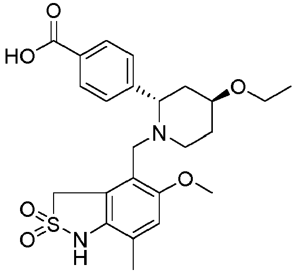 MS m/z (ESI): 475.2 [M+H] | 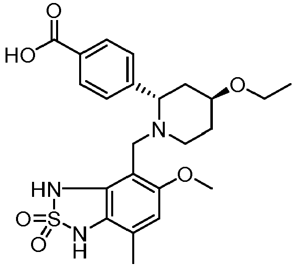 MS m/z (ESI): 476.2 [M+H] |
| 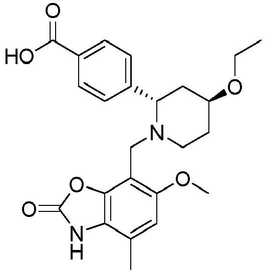 MS m/z (ESI): 441.2 [M+H] | 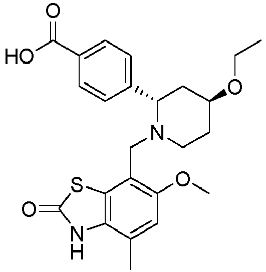 MS m/z (ESI): 457.2 [M+H] | 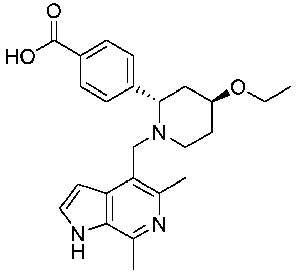 MS m/z (ESI): 408.2 [M+H] |
| 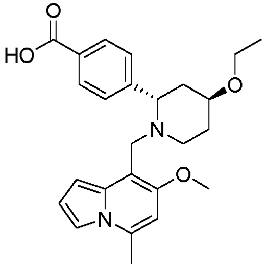 MS m/z (ESI): 423.2 [M+H] | 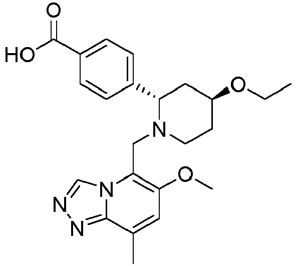 MS m/z (ESI): 425.2 [M+H] | 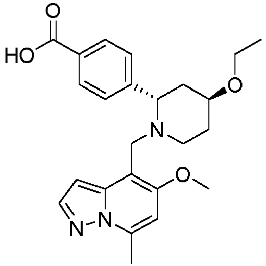 MS m/z (ESI): 424.2 [M+H] |
| 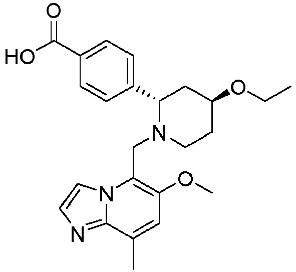 MS m/z (ESI): 424.2 [M+H] | 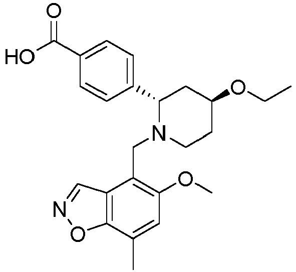 MS m/z (ESI): 425.2 [M+H] | 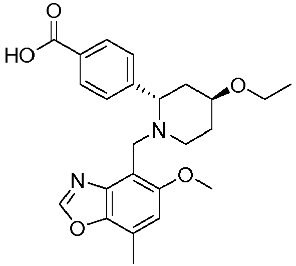 MS m/z (ESI): 425.2 [M+H] |
| 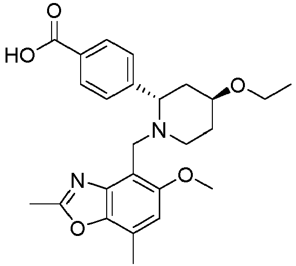 MS m/z (ESI): 439.2 [M+H] | 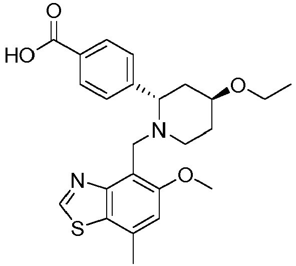 MS m/z (ESI): 441.2 [M+H] | 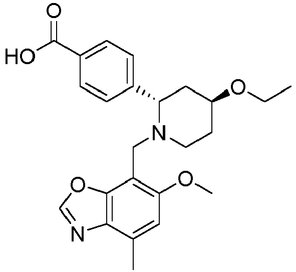 MS m/z (ESI): 425.2 [M+H] |
| 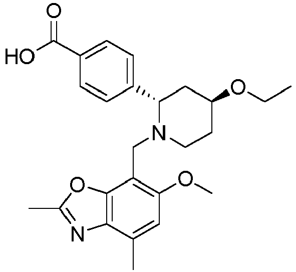 MS m/z (ESI): 439.2 [M+H] | 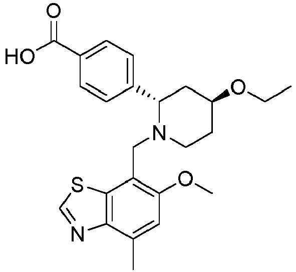 MS m/z (ESI): 441.2 [M+H] | 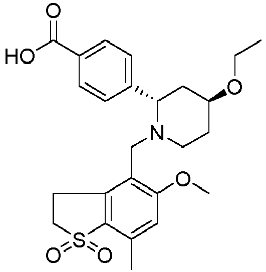 MS m/z (ESI): 474.2 [M+H] |
| 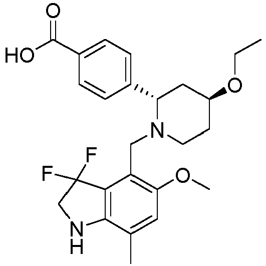 MS m/z (ESI): 461.2 [M+H] | 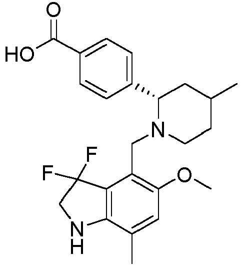 MS m/z (ESI): 431.2 [M+H] | 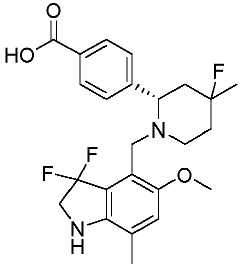 MS m/z (ESI): 449.2 [M+H] |
| 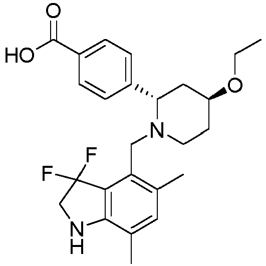 MS m/z (ESI): 445.2 [M+H] | 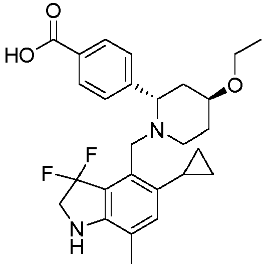 MS m/z (ESI): 471.2 [M+H] | 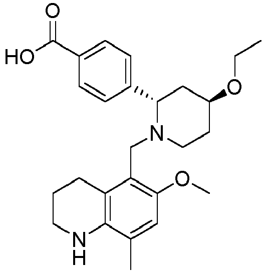 MS m/z (ESI): 439.3 [M+H] |
| | 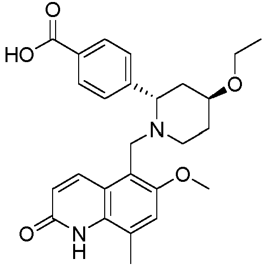 MS m/z (ESI): 451.2 [M+H] | 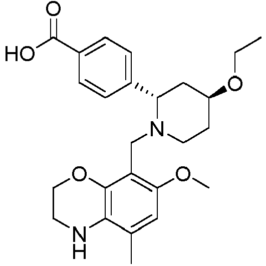 MS m/z (ESI): 441.2 [M+H] |
| 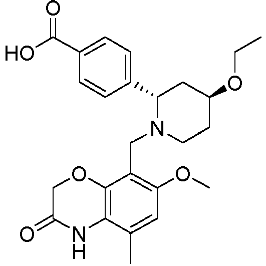 MS m/z (ESI): 455.2 [M+H] | 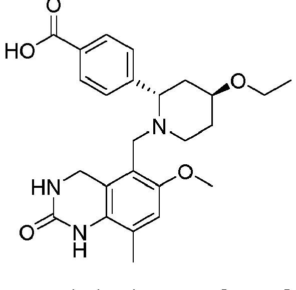 MS m/z (ESI): 454.2 [M+H] | 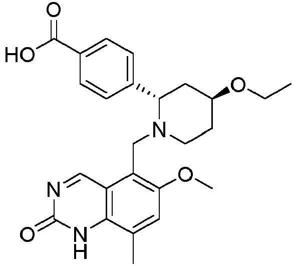 MS m/z (ESI): 452.2 [M+H] |
| 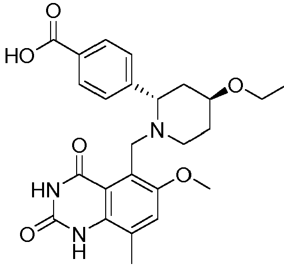 MS m/z (ESI): 468.2 [M+H] | 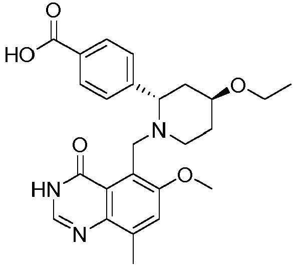 MS m/z (ESI): 452.2 [M+H] | 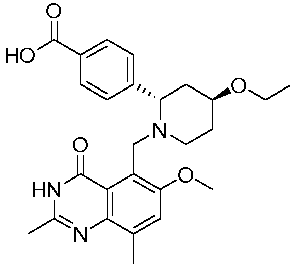 MS m/z (ESI): 466.2 [M+H] |
| 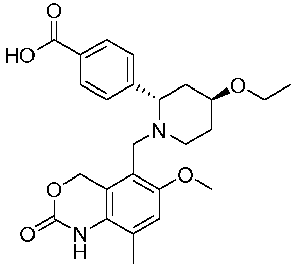 MS m/z (ESI): 455.2 [M+H] | 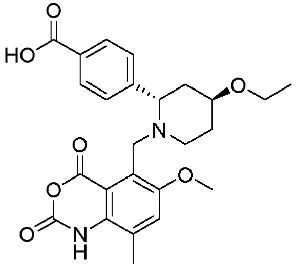 MS m/z (ESI): 469.2 [M+H] | 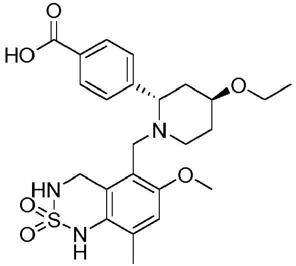 MS m/z (ESI): 490.2 [M+H] |
| 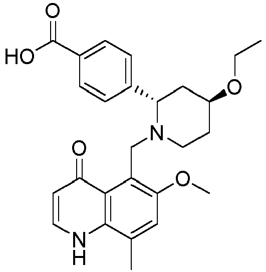 MS m/z (ESI): 451.2 [M+H] | 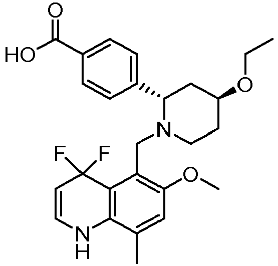 MS m/z (ESI): 473.2 [M+H] | 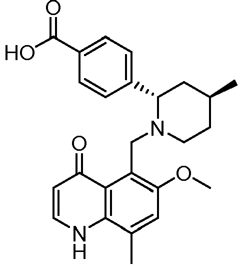 MS m/z (ESI): 421.2 [M+H] |
| 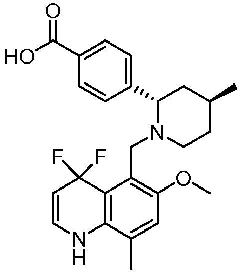 MS m/z (ESI): 443.2 [M+H] | 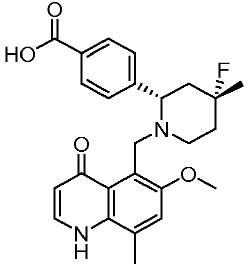 MS m/z (ESI): 439.2 [M+H] | 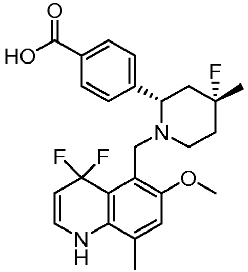 MS m/z (ESI): 461.2 [M+H] |
| 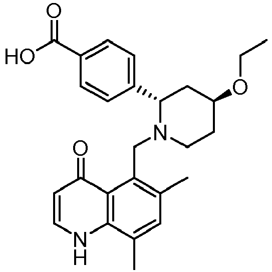 MS m/z (ESI): 435.2 [M+H] | 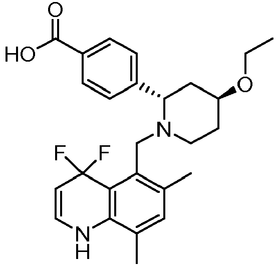 MS m/z (ESI): 457.2 [M+H] | 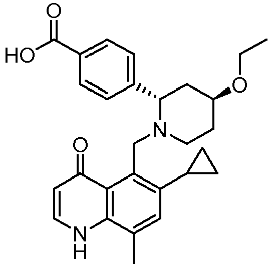 MS m/z (ESI): 461.2 [M+H] |
| 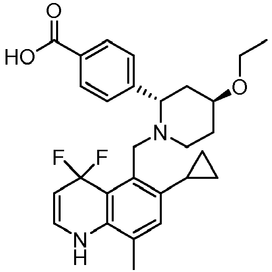 MS m/z (ESI): 483.2 [M+H] | 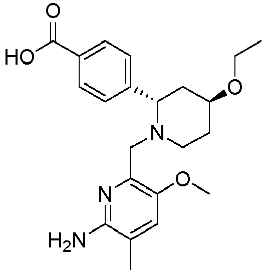 MS m/z (ESI): 400.2 [M+H] | 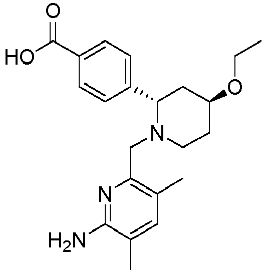 MS m/z (ESI): 384.2 [[M+H] |
| 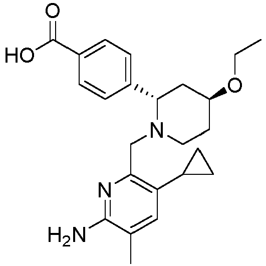 MS m/z (ESI): 410.2 [M+H] | 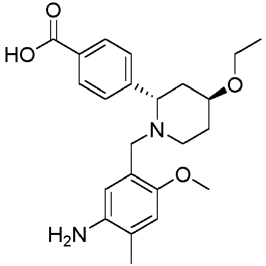 MS m/z (ESI): 399.2 [M+H] | 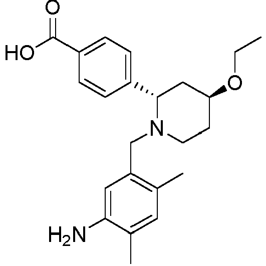 MS m/z (ESI): 383.2 [M+H] |
| 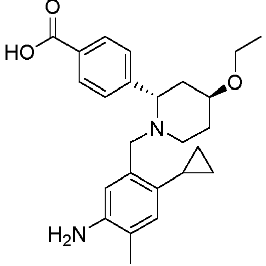 MS m/z (ESI): 409.2 [M+H] | 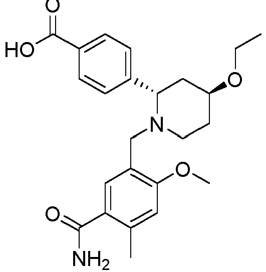 MS m/z (ESI): 427.2 [M+H] | 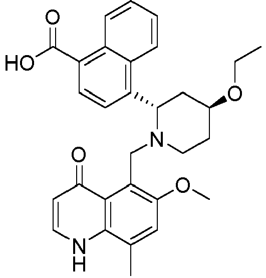 MS m/z (ESI): 501.2 [M+H] |
| 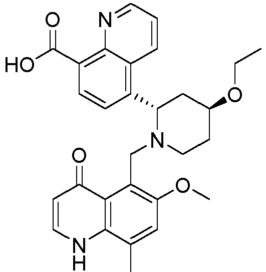 MS m/z (ESI): 502.2 [M+H] | 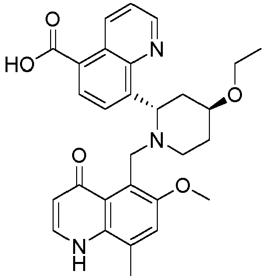 MS m/z (ESI): 502.2 [M+H] | 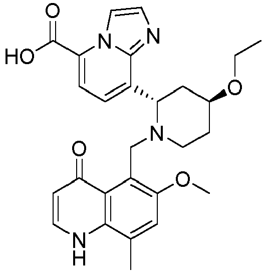 MS m/z (ESI): 491.2 [M+H] |
| 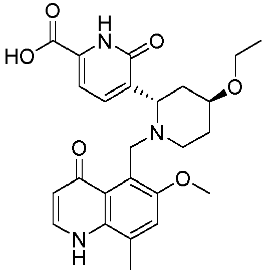 MS m/z (ESI): 468.2 [M+H] | 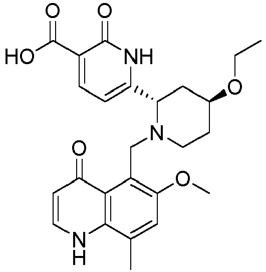 MS m/z (ESI): 468.2 [M+H] | 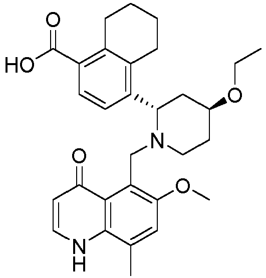 MS m/z (ESI): 505.3 [M+H] |
| 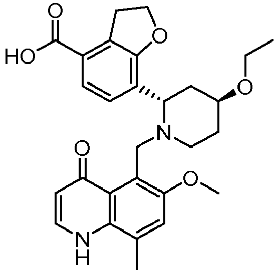 MS m/z (ESI): 493.2 [M+H] | 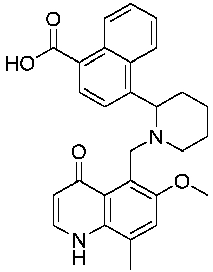 MS m/z (ESI): 457.2 [M+H] | 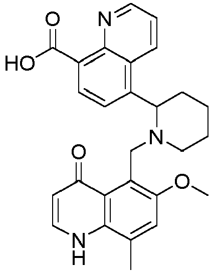 MS m/z (ESI): 458.2 [M+H] |
| 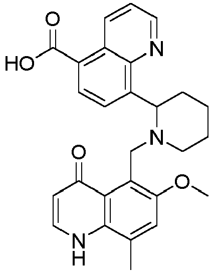 MS m/z (ESI): 458.2 [M+H] | 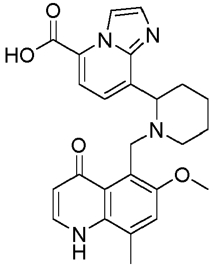 MS m/z (ESI): 447.2 [M+H] | 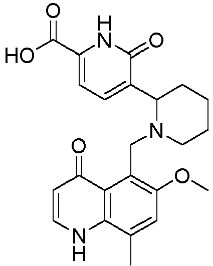 MS m/z (ESI): 424.2 [M+H] |
| 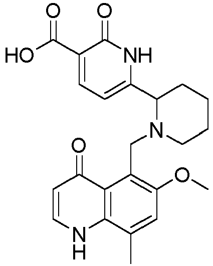 MS m/z (ESI): 424.2 [M+H] | 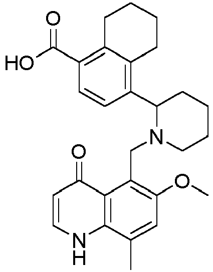 MS m/z (ESI): 461.2 [M+H] | 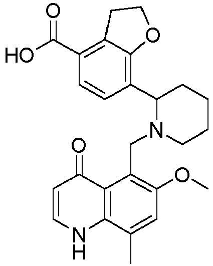 MS m/z (ESI): 449.2 [M+H] |

### Biological Assays:

### 1. Optical surface plasmon resonance (SPR) binding force assay

An SPR experiment was carried out at 25 °C. In the experiment, a PBS buffer supplemented with 0.05% (v/v) P20 and 5% DMSO was used as a running buffer, and the analytical instrument Biacore 8K of GE Healthcare was used. A CM7 ship (GE Healthcare) was activated with 400 mM EDC and 100 mM NHS at a flow rate of 30 µL/min for 420 s. Complement factor B was diluted to 50 µg/mL with 10 mM sodium acetate (pH 4.0) and then covalently immobilized to the assay chip by coupling at a flow rate of 10 µL/min for 1200 s (protein immobilization level at 25000 RU). Then the assay chip was treated with 1 M ethanolamine hydrochloride at a flow rate of 10 µL/min for 300 s for chip blocking. The concentration of the test compound was 500 µM, the binding time was 120 s, and the dissociation time was 300 s. Data analysis was performed using a 1:1 binding model (Biacore Insight Evalution Software, Version2.0.15.12933).

### 2. TR-FRET binding force assay

Competitive binding experiments using a small-molecule inhibitor fluorescently labeled with Cy5 as the probe were carried out to screen compounds for inhibitory activity against human complement factor B. After complement factor B and EZ-Link^{™} Sulfo-NHS-LC-LC-Biotin in a ratio of 1:2 were incubated on ice for 1 h, 1 M Tris (pH 7.5) was added to terminate the reaction. The mixture was then purified twice through a 2 mL Zeba^{™} desalt spin column to give a biotin-labeled complement factor B (EZ-LinkTM Sulfo-NHS-LC-Biotin instructions). In the experiments, 10 nM biotin-labeled complement factor B was pre-incubated with different concentrations of compounds in the buffer at room temperature for 1 h. The reaction was initiated by adding a Cy5 fluorescently labeled probe and europium chelate-labeled streptavidin (petroleum ether rkin Elmer, #AD0060) at final concentrations of 75 nM and 5 nM, respectively. Kinetic readings were taken on a microplate reader (excitation light at 337 nm, emitted light at 665 nm, 70 µs time-gated) and time-resolved fluorescence resonance energy transfer (TR-FRET) data were read to determine IC50.

### 3. Complement system hydrolysis C3 activity assay

Test compounds were 3-fold diluted from a starting concentration of 10 µM to 7 concentrations, and single-well assays were performed. Test compounds were diluted in a 96-well plate with DMSO into solutions with 1000× final concentration and then diluted with Diluent (WIESLAB^{®}COMPLEMENT SYSTEM ALTERNATIVE PATHWAY AP330) into solutions with 5× final concentration. 30 µL was transferred into a 96-well plate, and 120 µL of ready-to-use serum was added. The plate was incubated at room temperature for 15 min. To a positive control well was added 30 µL of 5 ‰ DMSO and 120 µL of ready-to-use serum. To a negative control well was added 30 µL of 5 ‰ DMSO and 120 µL of Diluent. (3) 100 µL was added to the reaction plate, and the plate was incubated at 37 °C for 60 min. The liquids in the wells were discarded, and each well was washed 3 times with 300 µL of washing liquid. To each well was added 100 µL of Conjugate (WIESLAB^{®}COMPLEMENT SYSTEM ALTERNATIVE PATHWAY AP330). The plate was incubated at room temperature for 30 min. The liquids in the wells were discarded, and each well was washed 3 times with 300 µL of washing liquid. Then 100 µL of substrate was added to each well. The plate was incubated at room temperature for 30 min. OD405 values were read using a microplate reader (Perkin Elmer, EnSight).

### 4. Complement hemolytic activity assay

The hemolysis experiment was carried out by referring to the description in Xuan Yuan et al., Haematologica. (2017) 102:466-475. Prior to the experiment, the optimal concentration of normal human serum (NHS) required to achieve 100% lysis of rabbit erythrocytes (REs) was obtained by titration testing. In this experiment, NHS (collected from healthy volunteers) was diluted in a GVBO buffer (0.1% gelatin, 5 mM Veronal, 145 mM NaCl, 0.025% NaN₃, pH 7.3, Complement technology) containing 10 mM Mg-EGTA and incubated with various concentration gradients of test compounds at 37 °C for 15 min. REs (collected from healthy Japanese big-ear white rabbits) freshly suspended in a GVBO buffer containing 10 mM Mg-EGTA were added to a final concentration of 1×108 cells/mL and incubated at 37 °C for 30 min. A GVBO buffer containing 10 mM Mg-EGTA and containing NHS and RE but no test compound was used as a positive control group (100% lysis). A GVBO buffer containing 10 mM Mg-EGTA and containing inactivated NHS (heated at 56 °C for 30 min or at 65 °C for 5 min) and RE but no test compound was used as a negative control group (0% lysis). The samples were centrifuged at 2000 g for 5 min, and the supernatant was collected. Absorbance at 415 nm (A415) was measured using a microplate reader (Molecular Devices, SpectraMax i3X). IC₅₀ values were calculated from percent hemolysis as a function of test compound concentration by non-linear regression. The assay results are shown in Table 1.

**Table 1:**

| **Example compound No.** | **IC₅₀ (µM)** | **Example No.** | **IC₅₀ (µM**) |
|---|---|---|---|
| Example 1 | 1.5 | Example 11 | >5 |
| Example 4 | 0.9 | Example 12 | >5 |
| Example 5 | 1.2 | Example 13 | 1 |
| Example 6 | >5 | Example 14 | >5 |
| Example 8 | >5 | Example 15 | >5 |
| Example 10 | 1.3 | Example 16 | >5 |

Although the specific embodiments of the present disclosure have been described above, it should be understood by those skilled in the art that these embodiments are merely illustrative and that many changes or modifications can be made to these embodiments without departing from the principle and spirit of the present disclosure. Therefore, the protection scope of the present disclosure is defined by the appended claims.

## Claims

1. A heterocyclic compound represented by formula I or a pharmaceutically acceptable salt, an isotopic analog or a prodrug thereof, which is optionally presented in a pharmaceutically acceptable carrier: 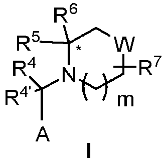 wherein,
W is O or C(R^{7'}R^{7"});
R⁷, R^{7'} and R^{7"} are independently hydrogen, hydroxy, halogen, C₁-C₄ alkyl or C₁-C₄ alkyl-O-;
R⁶ is hydrogen, C₁-C₄ alkyl or hydroxy C₁-C₄ alkyl;
R^{4'} and R⁴ are independently hydrogen;
m is 0, 1 or 2;
R⁵ is 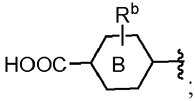 wherein the ring B is phenyl or 6-membered heteroaryl comprising 1, 2 or 3 heteroatoms selected from N, O and S;
R^{b} is H, hydroxy, =O, or a group ortho-fused to ring B, wherein the group is selected from phenyl, 3- to 6-membered cycloalkyl, 5- to 6-membered heterocycloalkyl and 5- to 6-membered heteroaryl; wherein the 5- to 6-membered heterocycloalkyl comprises 1, 2 or 3 heteroatoms selected from N, O, S, S(=O) and S(=O)₂; the 5- to 6-membered heteroaryl comprises 1, 2 or 3 heteroatoms selected from N, O and S; when multiple substituents are present, they are the same or different
A is 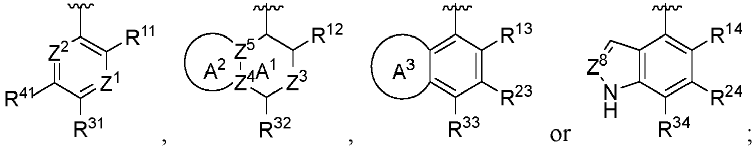
Z¹ is C(R²¹) or N; Z² is C(R⁵¹) or N; R⁴¹ is NH₂ or C(=O)NH₂;
ring A¹ is pyridinyl; wherein, Z³ is C(R²²) or N; Z⁴ and Z⁵ are independently C or N;
R²¹, R²² and R⁵¹ are independently hydrogen;
ring A² is 5- to 6-membered heterocycloalkyl, 5- to 6-membered heterocycloalkenyl or 5- to 6-membered heteroaryl, or 5- to 6-membered heterocycloalkyl, 5- to 6-membered heterocycloalkenyl or 5- to 6-membered heteroaryl substituted with one or more R^{a1}; wherein the 5- to 6-membered heterocycloalkyl and the 5- to 6-membered heterocycloalkyl of the 5- to 6-membered heterocycloalkyl substituted with one or more R^{a1} comprise 1, 2 or 3 heteroatoms selected from N, O, S, S(=O) and S(=O)₂ respectively; the 5- to 6-membered heterocycloalkenyl and the 5- to 6-membered heterocycloalkenyl of the 5- to 6-membered heterocycloalkenyl substituted with one or more R^{a1} comprise 1, 2 or 3 heteroatoms selected from N, O, S, S(=O) and S(=O)₂ respectively; the 5- to 6-membered heteroaryl and the 5- to 6-membered heteroaryl of the 5- to 6-membered heteroaryl substituted with one or more R^{a1} comprise 1, 2 or 3 heteroatoms selected from N, O and S respectively; when multiple substituents are present, they are the same or different;
ring A³ is 5- to 6-membered heterocycloalkyl, 5- to 6-membered heterocycloalkenyl, 6-membered heteroaryl or 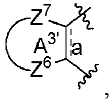 or 5- to 6-membered heterocycloalkyl, 5- to 6-membered heterocycloalkenyl, 6-membered heteroaryl or 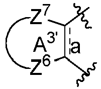 substituted with one or more R^{a2};
wherein the 5- to 6-membered heterocycloalkyl and the 5- to 6-membered heterocycloalkyl of the 5- to 6-membered heterocycloalkyl substituted with one or more R^{a2} comprise 1, 2 or 3 heteroatoms selected from N, O, S, S(=O) and S(=O)₂ respectively; the 5- to 6-membered heterocycloalkenyl and the 5- to 6-membered heterocycloalkenyl of the 5- to 6-membered heterocycloalkenyl substituted with one or more R^{a2} comprise 1, 2 or 3 heteroatoms selected from N, O, S, S(=O) and S(=O)₂ respectively; the 6-membered heteroaryl and the 6-membered heteroaryl of the 5- to 6-membered heteroaryl substituted with one or more R^{a2} comprise 1, 2 or 3 heteroatoms selected from N, O and S respectively; when multiple substituents are present, they are the same or different; ring A³ is ortho-fused to a benzene ring;
A^{3'} is 5-membered heteroaryl; wherein the 5-membered heteroaryl comprises 1 or 2 heteroatoms selected from N, O and S; and Z⁷ is N, O or S, and/or Z⁶ is CH, O or S;
R^{a1} and R^{a2} are independently hydroxy, =O, halogen, CN, C₁-C₄ alkyl or C₁-C₄ alkyl-O-;
R¹¹, R³¹, R¹², R³², R¹³ and R³³ are independently C₁-C₄ alkyl, C₁-C₄ alkyl-O- or 3- to 6-membered cycloalkyl;
Z⁸ is CH or N; R¹⁴ is C₁-C₄ alkyl-O-; R²³ and R²⁴ is H; R³⁴ is C₁-C₄ alkyl or 3- to 6-membered cycloalkyl;
the carbon atom with "*" means that when it is a chiral carbon atom, the compound has an S configuration or an R configuration, or a mixture thereof.

2. The heterocyclic compound represented by formula I or the pharmaceutically acceptable salt, the isotopic analog or the prodrug thereof, which is optionally presented in the pharmaceutically acceptable carrier according to claim 1, wherein,
W is C(R^{7'}R^{7"});
and/or, R^{7'} and R^{7"} are independently hydrogen, halogen, C₁-C₄ alkyl or C₁-C₄ alkyl-O-;
and/or, R⁷ is hydrogen;
and/or, m is 1;
and/or, R^{b} is H;
and/or, Z¹ is CH, and Z² is N; or Z¹ is CH, and Z² is CH; or Z¹ is N, and Z² is CH;
and/or, Z⁴ is N, or Z⁵ is N;
and/or, ring A² is 5- to 6-membered heteroaryl; or ring A² is 5- to 6-membered heterocycloalkyl, 5- to 6-membered heterocycloalkenyl or 6-membered heteroaryl, or 5- to 6-membered cycloalkenyl, 5- to 6-membered heterocycloalkyl, 5- to 6-membered heterocycloalkenyl or 6-membered heteroaryl substituted with one or more R^{a1};
and/or, ring A³ is 5- to 6-membered heterocycloalkyl, 5- to 6-membered heterocycloalkenyl or 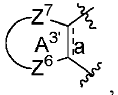 or 5- to 6-membered heterocycloalkyl, 5- to 6-membered heterocycloalkenyl, 6-membered heteroaryl or 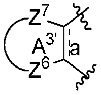 substituted with one or more R^{a2};
and/or, R^{a1} and R^{a2} are independently hydroxy, halogen, =O or C₁-C₄ alkyl;
and/or, R¹¹, R¹² and R¹³ are independently C₁-C₄ alkyl or C₁-C₄ alkyl-O-;
and/or, R³¹, R³² and R³³ are independently C₁-C₄ alkyl;
and/or, R¹⁴ is C₁-C₄ alkyl-O-;
and/or, Z⁸ is N, and R³⁴ is C₁-C₄ alkyl; or Z⁸ is CH, and R³⁴ is 3- to 6-membered cycloalkyl.

3. The heterocyclic compound represented by formula I or the pharmaceutically acceptable salt, the isotopic analog or the prodrug thereof, which is optionally presented in the pharmaceutically acceptable carrier according to claim 1, wherein the heterocyclic compound represented by formula I is any one of the following solutions:
solution 1:
the heterocyclic compound represented by formula I is represented by formula Ia, Ib or Ic below: 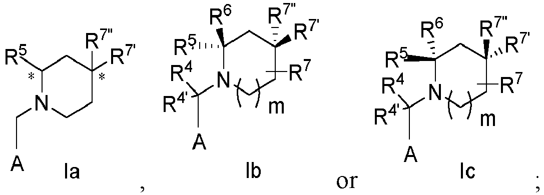
solution 2:
W is C(R^{7'}R^{7"}); R⁷ is hydrogen;
R^{7'} and R^{7"} are independently hydrogen, halogen, C₁-C₄ alkyl or C₁-C₄ alkyl-O-;
R⁶ is hydrogen;
R^{4'} and R⁴ are independently hydrogen;
m is 1;
R⁵ is 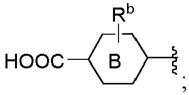 ring B is phenyl or 6-membered heteroaryl;
R^{b} is H, hydroxy, =O, or a group ortho-fused to ring B, wherein the group is selected from phenyl, 3- to 6-membered cycloalkyl, 5- to 6-membered heterocycloalkyl and 5- to 6-membered heteroaryl;
A is 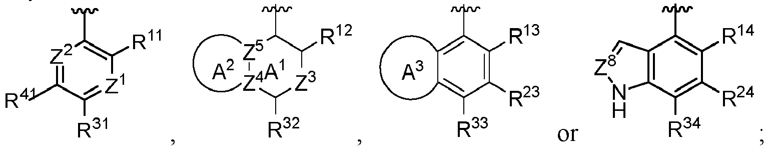
Z¹ is CH or N; Z² is CH or N; R⁴¹ is NH₂ or C(=O)NH₂;
ring A¹ is pyridinyl; Z³ is CH or N; Z⁴ and Z⁵ are independently C or N;
ring A² is 5- to 6-membered heterocycloalkyl, 5- to 6-membered heterocycloalkenyl or 5- to 6-membered heteroaryl, or 5- to 6-membered heterocycloalkyl, 5- to 6-membered heterocycloalkenyl or 5- to 6-membered heteroaryl substituted with one or more R^{a1};
ring A³ is 5- to 6-membered heterocycloalkyl, 5- to 6-membered heterocycloalkenyl, 6-membered heteroaryl or 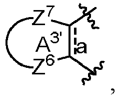 or 5- to 6-membered heterocycloalkyl, 5- to 6-membered heterocycloalkenyl, 6-membered heteroaryl or 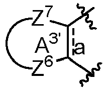 substituted with one or more R^{a2};
R^{a1} and R^{a2} are independently hydroxy, halogen, =O or C₁-C₄ alkyl;
R¹¹, R³¹, R¹², R³², R¹³, R²³ and R³³ are independently C₁-C₄ alkyl, C₁-C₄ alkyl-O- or 3- to 6-membered cycloalkyl;
Z⁸ is CH or N; R¹⁴ is C₁-C₄ alkyl-O-; R²³ and R²⁴ is H; R³⁴ is C₁-C₄ alkyl or 3- to 6-membered cycloalkyl;
solution 3:
W is C(R^{7'}R^{7"}); R⁷ is hydrogen;
R^{7'} and R^{7"} are independently hydrogen or C₁-C₄ alkyl-O-;
R⁶ is hydrogen;
R^{4'} and R⁴ are independently hydrogen;
m is 1;
R⁵ is 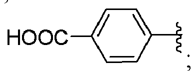
A is 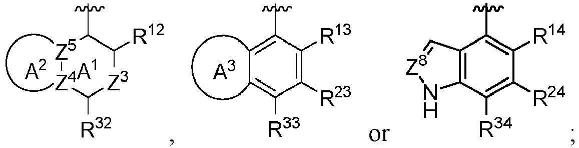
ring A¹ is pyridinyl; Z³ is N; Z⁴ and Z⁵ is C;
ring A² is 5-membered heteroaryl or 5-membered heteroaryl substituted with one or more R^{a1};
ring A³ is 5- to 6-membered heterocycloalkyl, 5- to 6-membered heterocycloalkenyl, 6-membered heteroaryl or 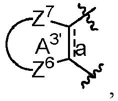 or 5- to 6-membered heterocycloalkyl, 5- to 6-membered heterocycloalkenyl, 6-membered heteroaryl or 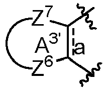 substituted with one or more R^{a2};
R^{a1} and R^{a2} are independently hydroxy, =O or C₁-C₄ alkyl;
R¹² and R¹³ are independently C₁-C₄ alkyl-O-;
R³² and R³³ are independently C₁-C₄ alkyl;
Z⁸ is CH or N; R¹⁴ is C₁-C₄ alkyl-O-; R²³ and R²⁴ is H; R³⁴ is C₁-C₄ alkyl or 3- to 6-membered cycloalkyl;
solution 4:
W is C(R^{7'}R^{7"}); R⁷ is hydrogen;
R^{7'} and R^{7"} are independently hydrogen or C₁-C₄ alkyl-O-;
R⁶ is hydrogen;
R^{4'} and R⁴ are independently hydrogen;
m is 1;
R⁵ is 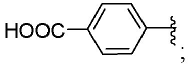
A is 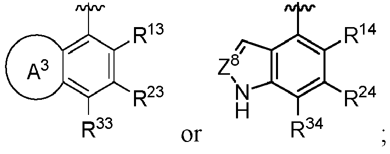
ring A³ is 5-membered heterocycloalkyl, 5-membered heterocycloalkenyl or 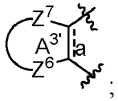
R¹³ is C₁-C₄ alkyl-O-;
R³³ is C₁-C₄ alkyl;
Z⁸ is CH or N; R¹⁴ is C₁-C₄ alkyl-O-; R²³ and R²⁴ are H; R³⁴ is C₁-C₄ alkyl or 3- to 6-membered cycloalkyl.

4. The heterocyclic compound represented by formula I or the pharmaceutically acceptable salt, the isotopic analog or the prodrug thereof, which is optionally presented in the pharmaceutically acceptable carrier according to any one of claims 1-3, wherein,
when R⁷, R^{7'} and R^{7"} are independently C₁-C₄ alkyl or C₁-C₄ alkyl-O-, the C₁-C₄ alkyl or the C₁-C₄ alkyl of the C₁-C₄ alkyl-O- is methyl, ethyl, n-propyl, isopropyl, n-butyl, isobutyl, sec-butyl or tert-butyl, e.g., methyl;
and/or, when R⁶ is C₁-C₄ alkyl or hydroxy C₁-C₄ alkyl, the C₁-C₄ alkyl and the C₁-C₄ alkyl of the hydroxy C₁-C₄ alkyl is methyl, ethyl, n-propyl, isopropyl, n-butyl, isobutyl, sec-butyl or *tert-*butyl, e.g., methyl;
and/or, when ring B is 6-membered heteroaryl, the 6-membered heteroaryl is pyridinyl, e.g., 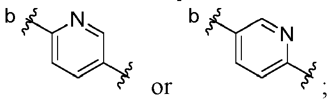
and/or, when R^{b} is 3- to 6-membered cycloalkyl, the 3- to 6-membered cycloalkyl is cyclopropyl, cyclobutyl, cyclopentyl and cyclohexyl, e.g., cyclopentyl;
and/or, when R^{b} is 5- to 6-membered heterocycloalkyl, the 5- to 6-membered heterocycloalkyl is tetrahydrofuranyl, e.g., 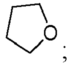
and/or, when R^{b} is 5- to 6-membered heteroaryl, the 5- to 6-membered heteroaryl is pyridinyl or imidazolyl; e.g., 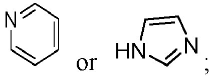
and/or, when ring A² is 5- to 6-membered heterocycloalkyl, the 5- to 6-membered heterocycloalkyl is 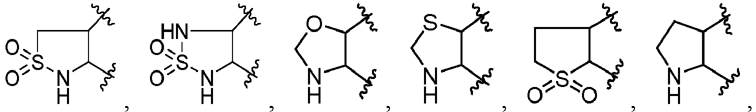 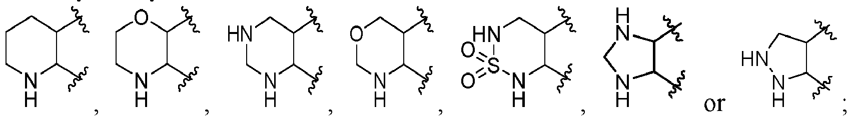
and/or, when ring A² is 5- to 6-membered heterocycloalkenyl, the 5- to 6-membered heterocycloalkenl is 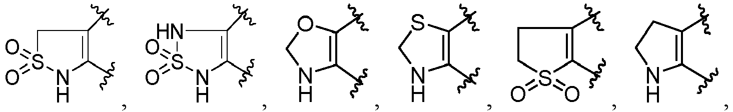 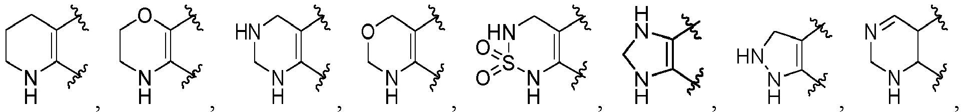 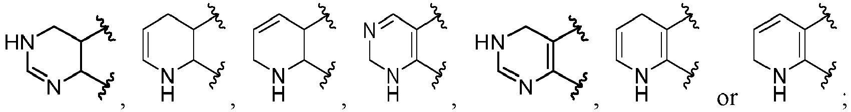
and/or, when ring A² is 5- to 6-membered heteroaryl, the 5- to 6-membered heteroaryl is 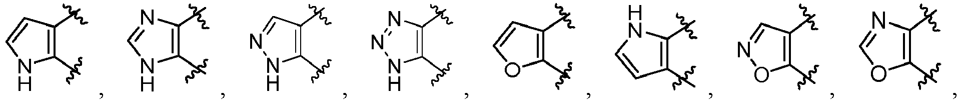 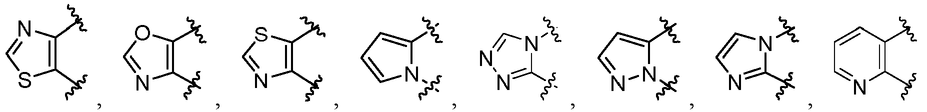 or 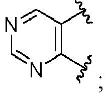
and/or, when ring A³ is 5- to 6-membered heterocycloalkyl, the 5- to 6-membered heterocycloalkyl is 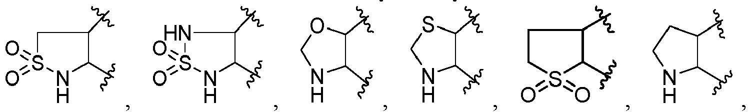 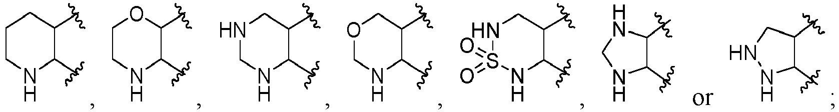
and/or, when ring A³ is 5- to 6-membered heterocycloalkenyl, the 5- to 6-membered heterocycloalkenyl is 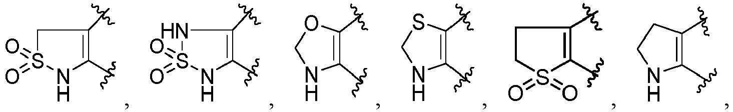 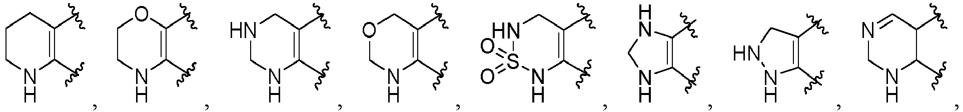 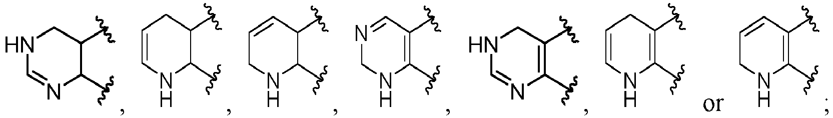
and/or, when ring A³ is 6-membered heteroaryl, the 6-membered heteroaryl is 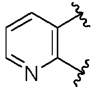 or 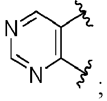
and/or, when ring A³ is 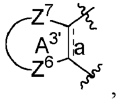 the A^{3'} is 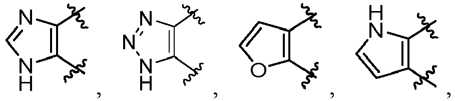 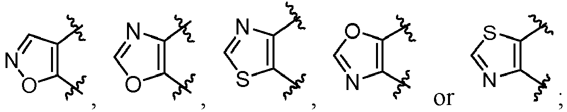
and/or, when R^{a1} and R^{a2} are independently C₁-C₄ alkyl or C₁-C₄ alkyl-O-, the C₁-C₄ alkyl or the C₁-C₄ alkyl of the C₁-C₄ alkyl-O- is methyl, ethyl, *n*-propyl, isopropyl, *n*-butyl, isobutyl, *sec-*butyl or *tert*-butyl, e.g., methyl;
and/or, when R¹¹, R³¹, R¹², R³², R¹³, R²³ and R³³ are independently C₁-C₄ alkyl or C₁-C₄ alkyl-O-, the C₁-C₄ alkyl and the C₁-C₄ alkyl of the C₁-C₄ alkyl-O- is methyl, ethyl, *n*-propyl, isopropyl, *n*-butyl, isobutyl, *sec*-butyl or *tert*-butyl, e.g., methyl;
and/or, when R¹¹, R³¹, R¹², R³², R¹³, R²³ and R³³ are independently 3- to 6-membered cycloalkyl, the 3- to 6-membered cycloalkyl is cyclopropyl, cyclobutyl, cyclopentyl and cyclohexyl, e.g., cyclopropyl;
and/or, when R¹⁴ is C₁-C₄ alkyl-O-, the C₁-C₄ alkyl of the C₁-C₄ alkyl-O- is methyl, ethyl, *n-*propyl, isopropyl, *n*-butyl, isobutyl, *sec*-butyl or *tert*-butyl, e.g., methyl;
and/or, when R³⁴ is C₁-C₄ alkyl, the C₁-C₄ alkyl is methyl, ethyl, *n*-propyl, isopropyl, *n*-butyl, isobutyl, *sec-*butyl or *tert-*butyl*,* e.g., methyl;
and/or, when R³⁴ is 3- to 6-membered cycloalkyl, the 3- to 6-membered cycloalkyl is cyclopropyl, cyclobutyl, cyclopentyl and cyclohexyl, e.g., cyclopropyl.

5. The heterocyclic compound represented by formula I or the pharmaceutically acceptable salt, the isotopic analog or the prodrug thereof, which is optionally presented in the pharmaceutically acceptable carrier according to claim 4, wherein,
R^{7'} and R^{7"} are independently hydrogen, F, methyl or ethyl-O-; for example, W is 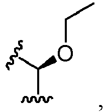 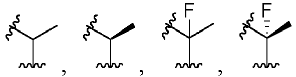 or methylene;
and/or, R⁵ is 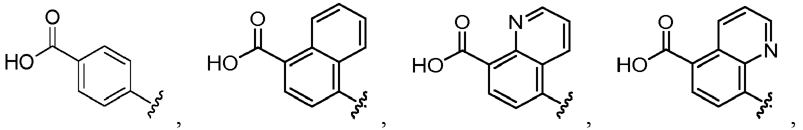 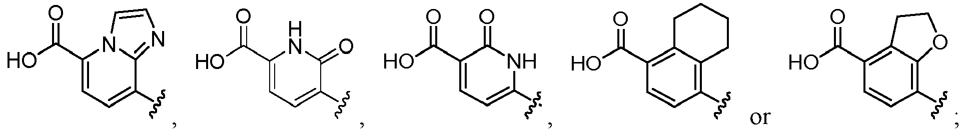
for example, 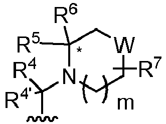 is 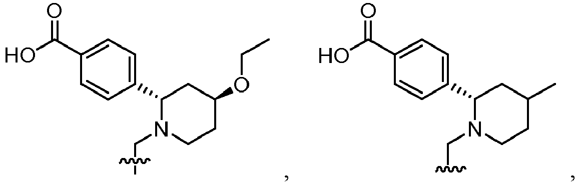 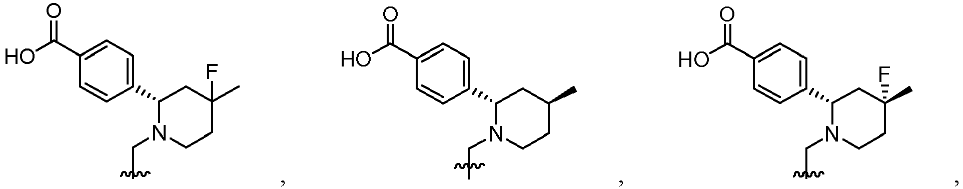 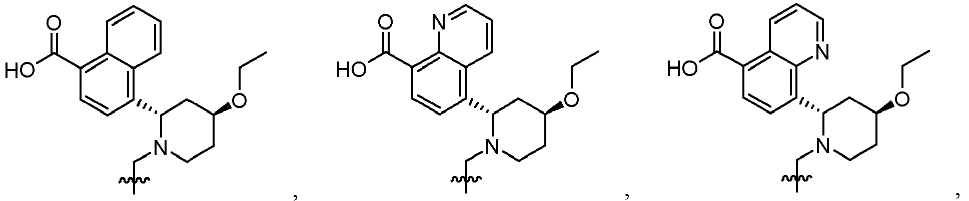 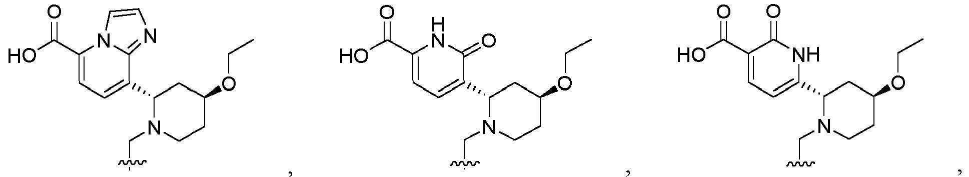 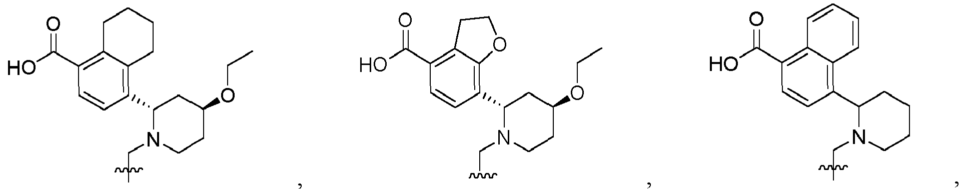 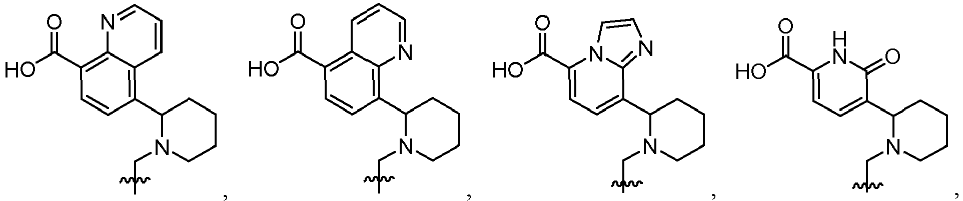 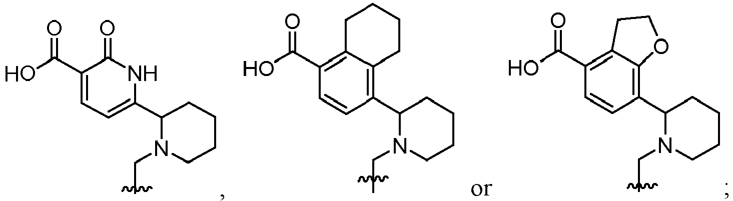
and/or, R^{a1} and R^{a2} are independently hydroxy, =O or methyl;
and/or, R¹¹, R¹² and R¹³ are independently methyl, methyl-O- or cyclopropyl;
and/or, R³¹, R³² and R³³ are independently methyl;
and/or, R¹⁴ is methyl-O-;
and/or, R³⁴ is methyl or cyclopropyl;
and/or, when A is 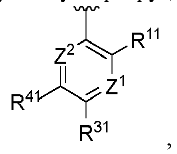 A is 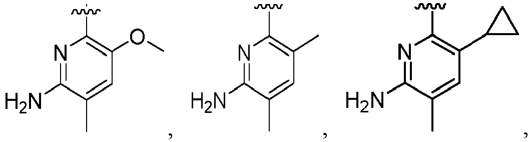 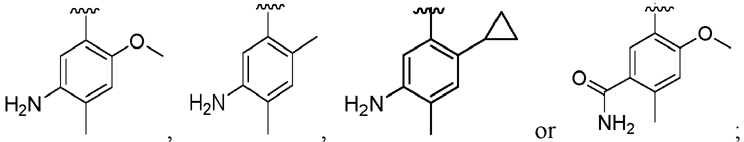
and/or, when A is 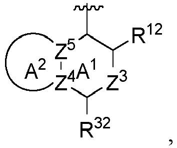 A is 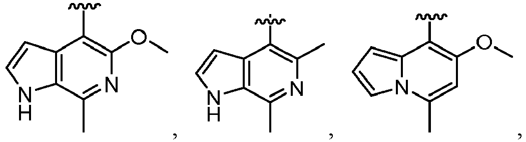 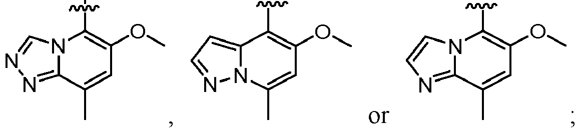
and/or, when A is 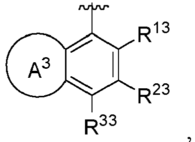 A is 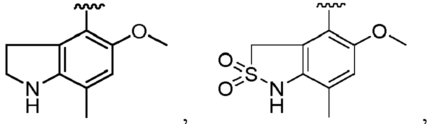 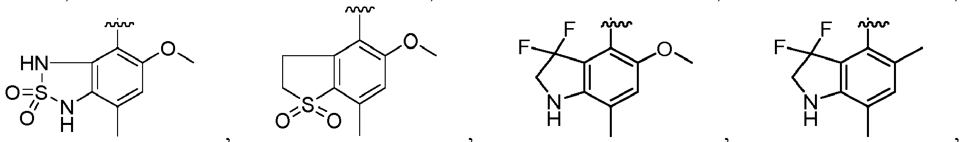 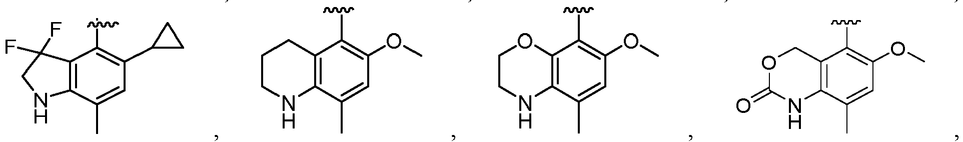 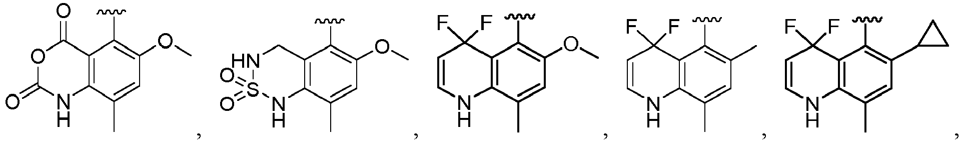 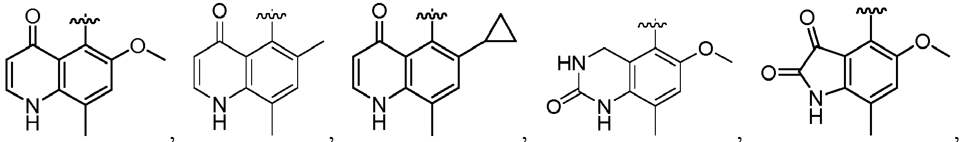 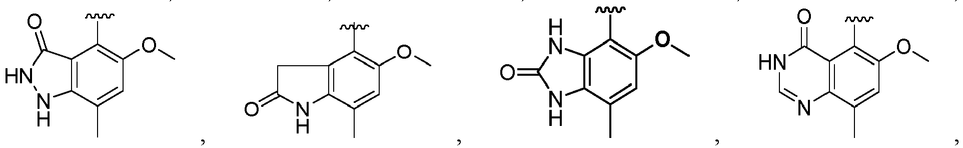 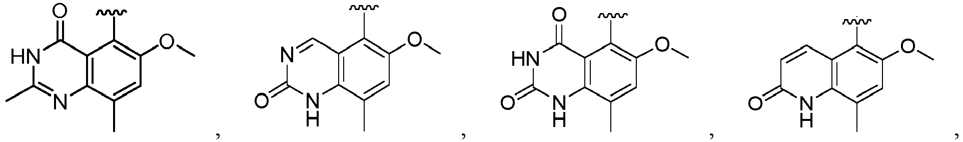 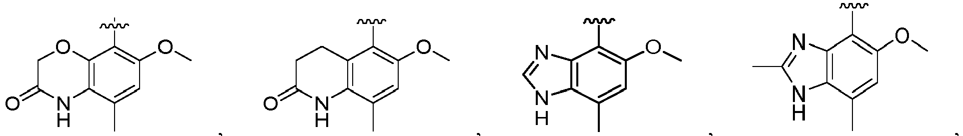 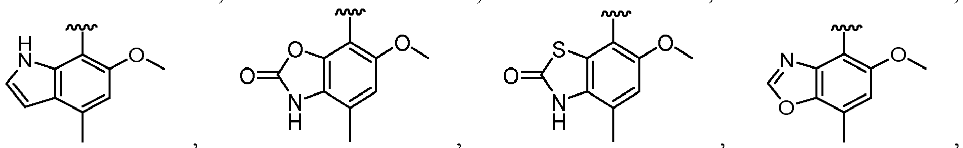 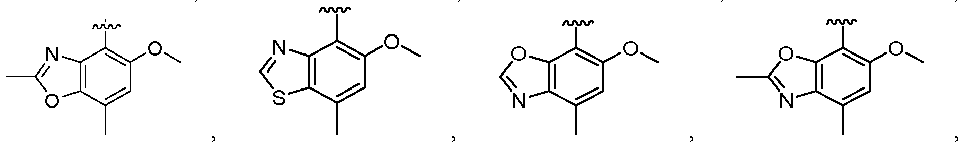 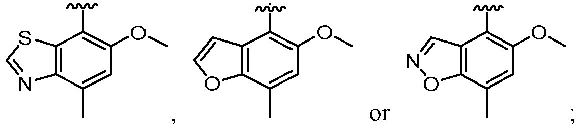
and/or, when A is 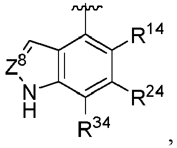 A is

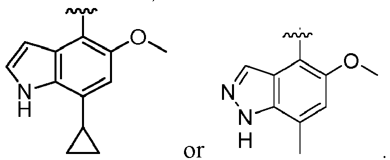

6. The heterocyclic compound represented by formula I or the pharmaceutically acceptable salt, the isotopic analog or the prodrug thereof, which is optionally presented in the pharmaceutically acceptable carrier according to claim 1, wherein the heterocyclic compound represented by formula I is any one of the following structures: 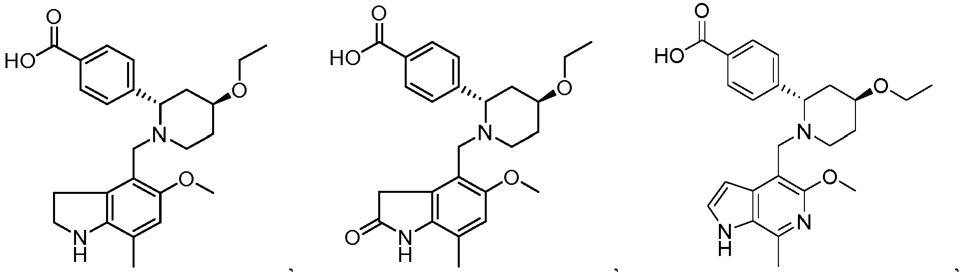 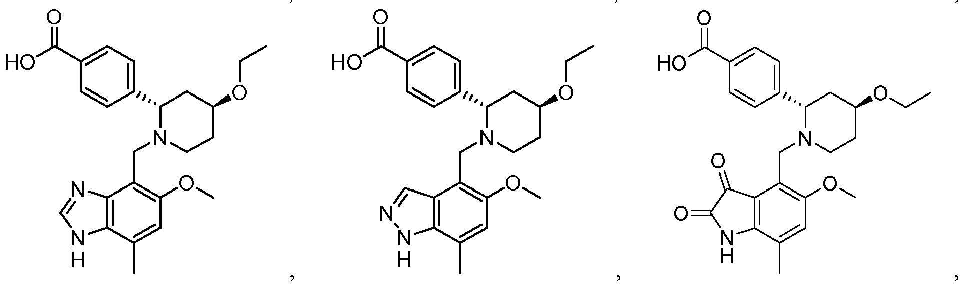 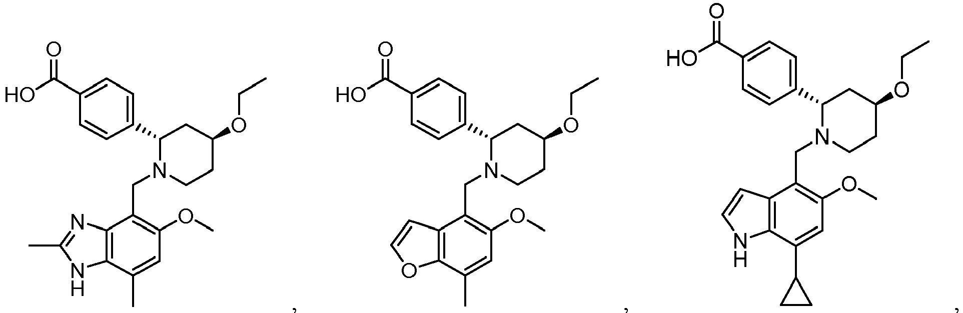 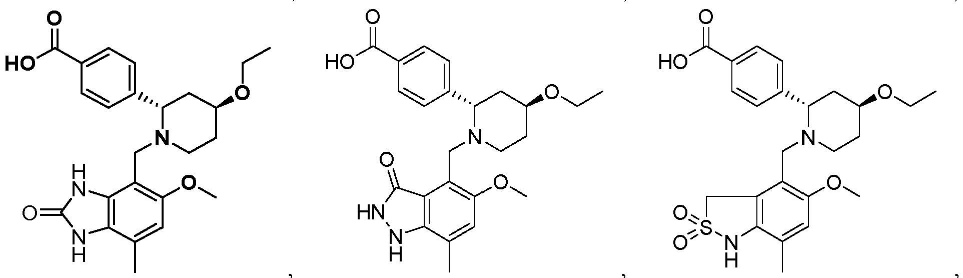 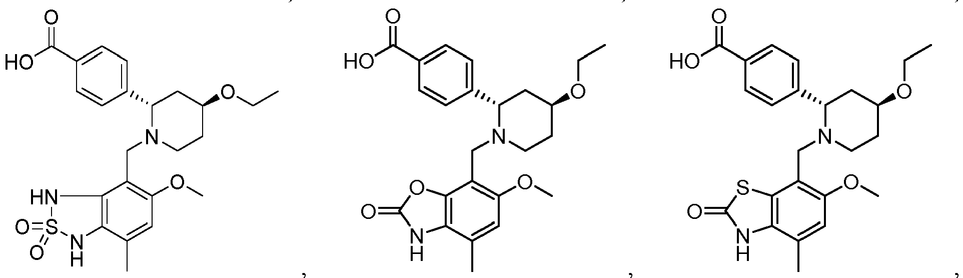 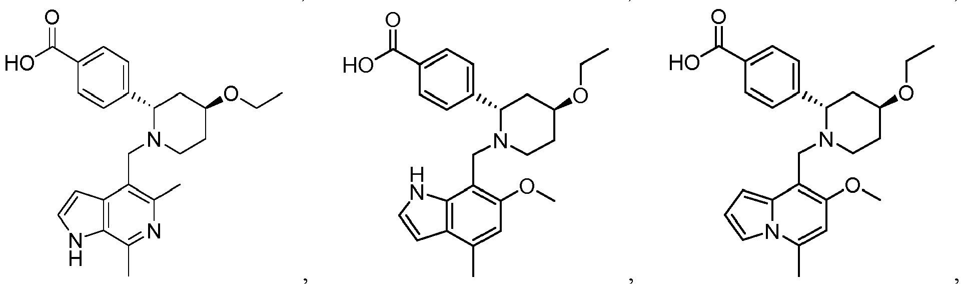 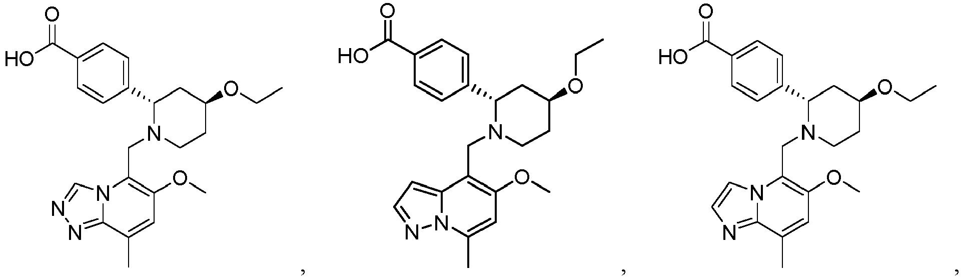 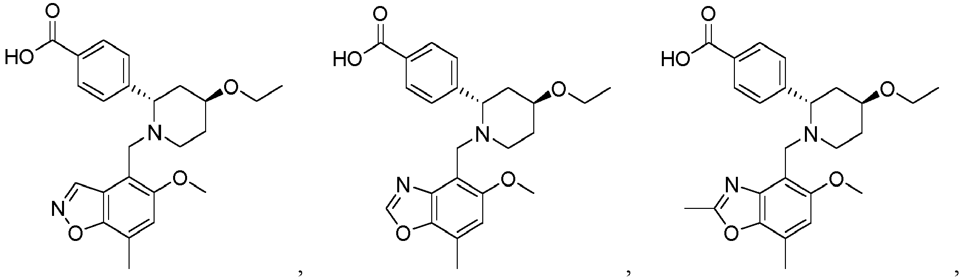 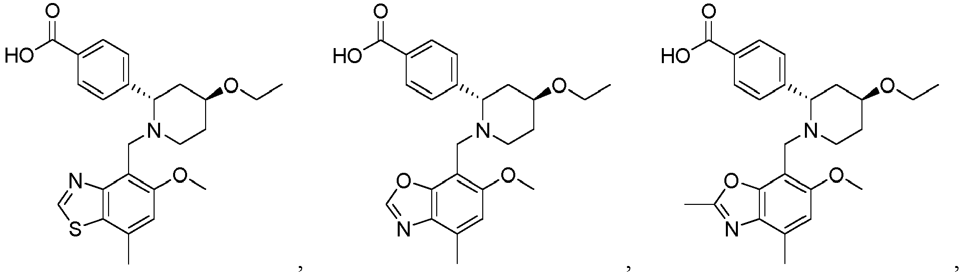 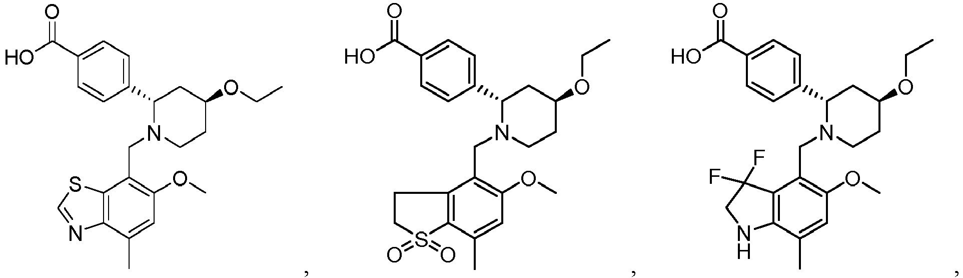 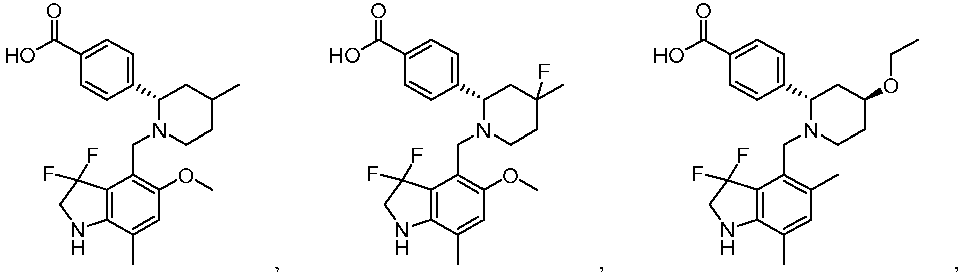 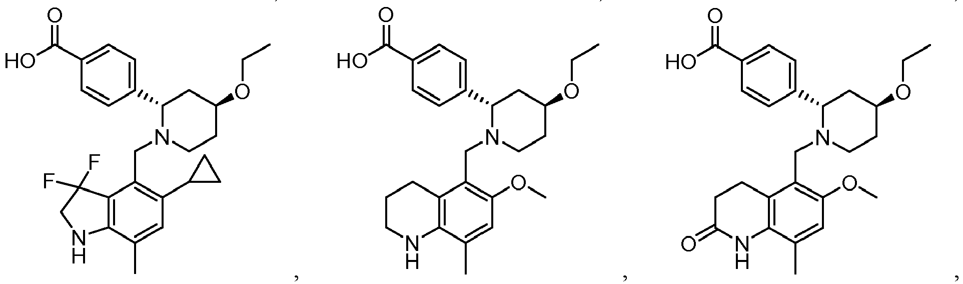 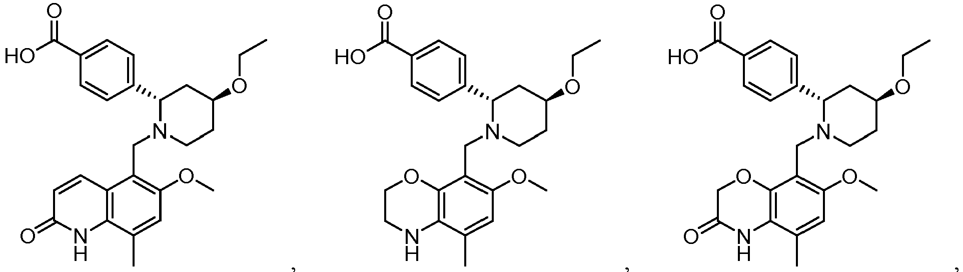 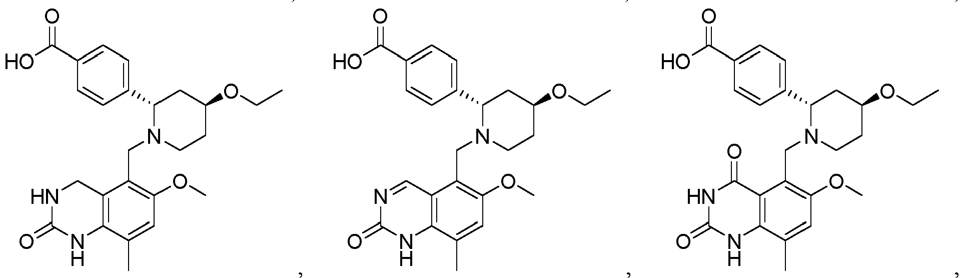 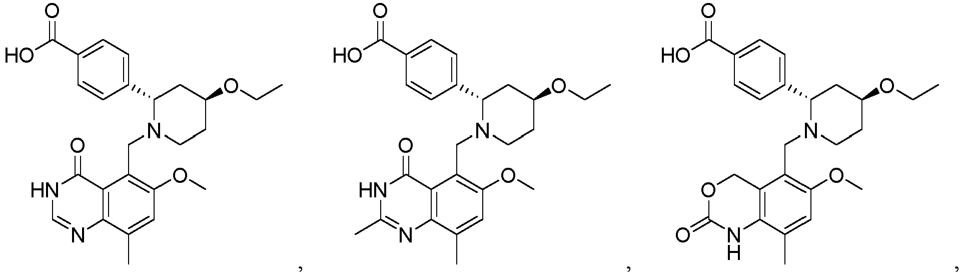 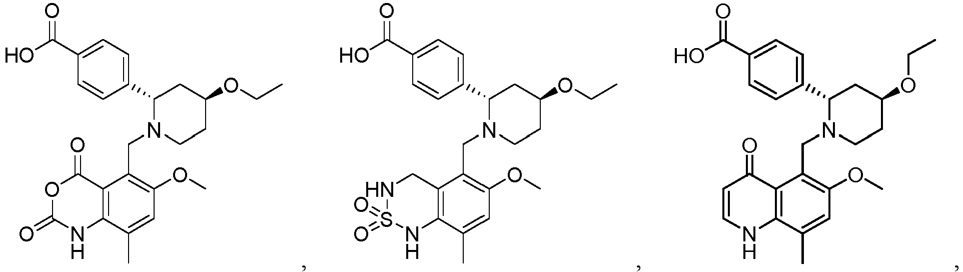 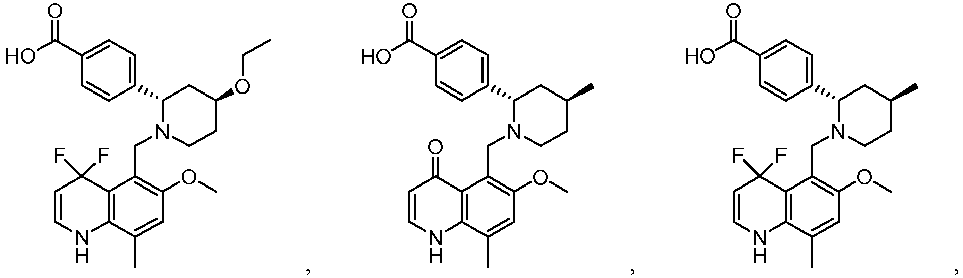 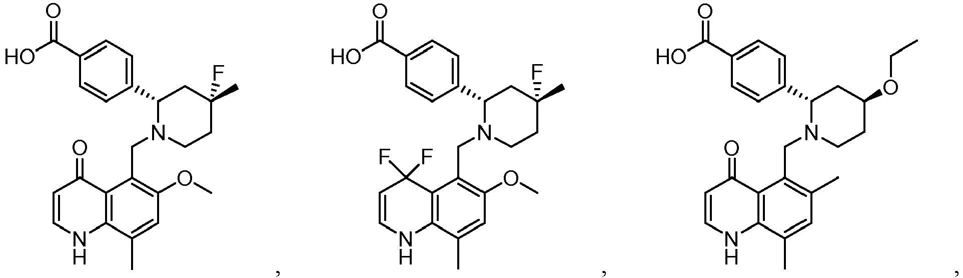 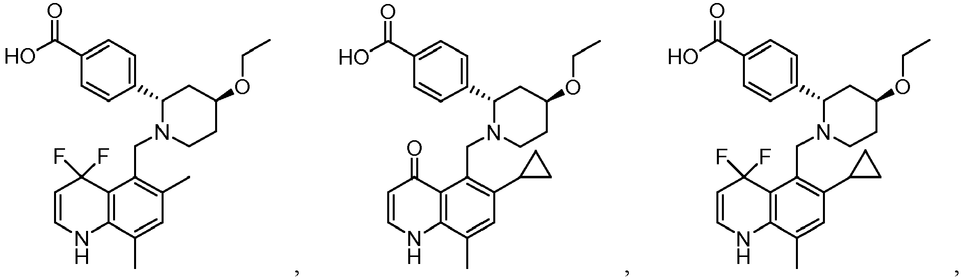 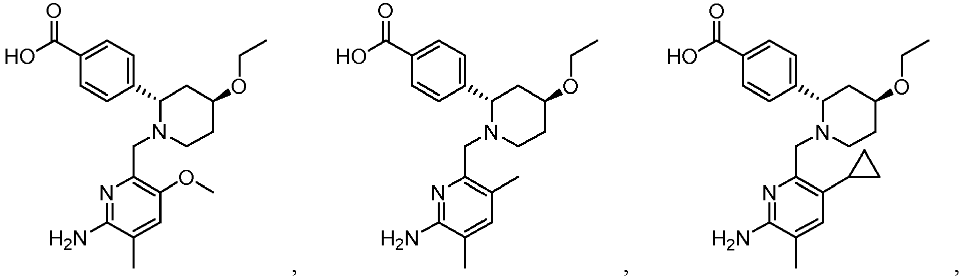 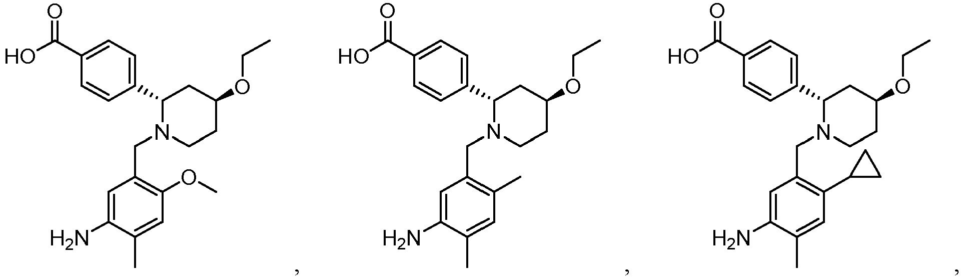 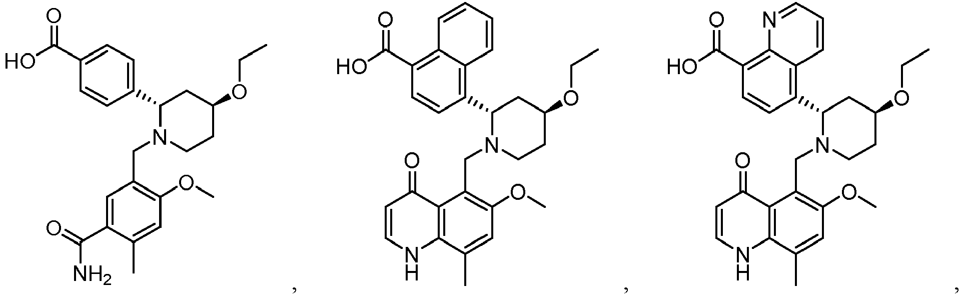 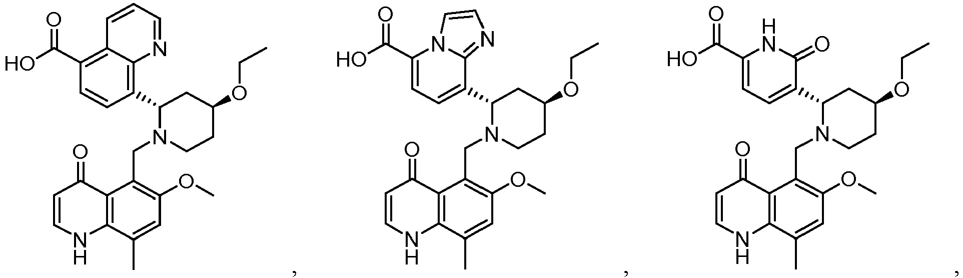 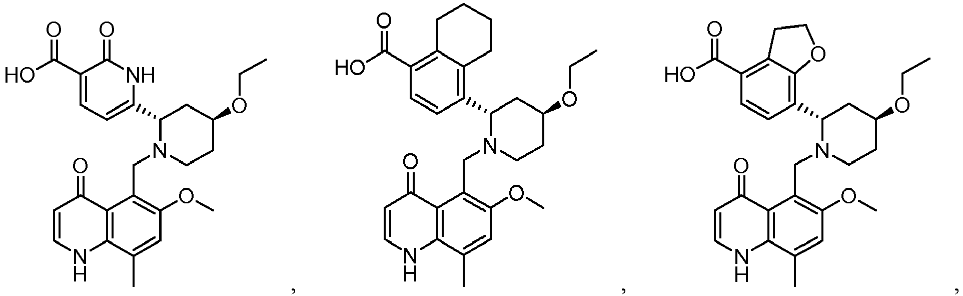 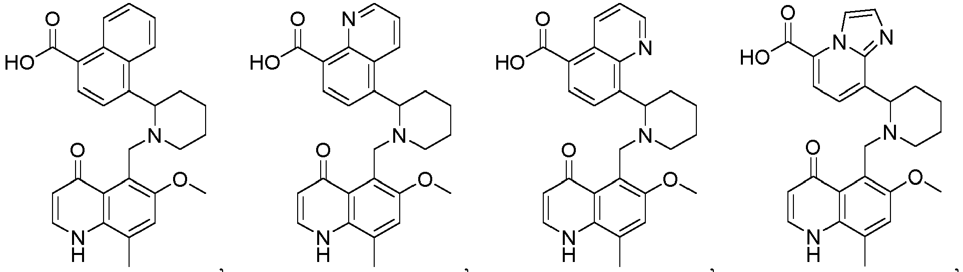 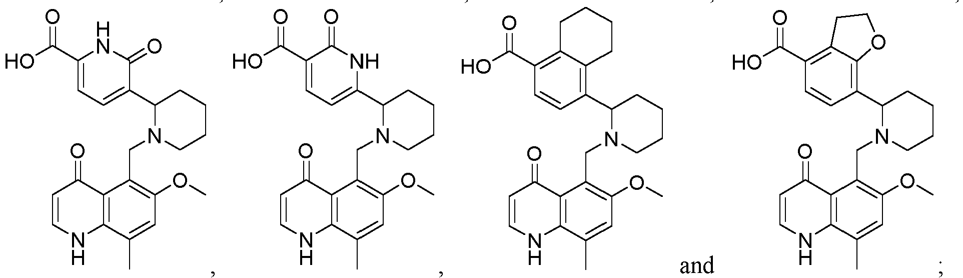 and/or, the pharmaceutically acceptable salt of the heterocyclic compound represented by formula I is any one of the following structures: 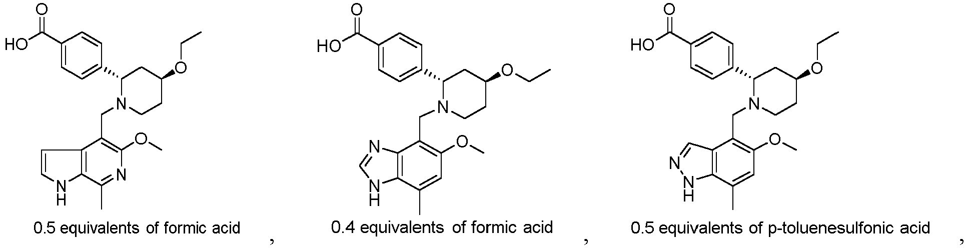 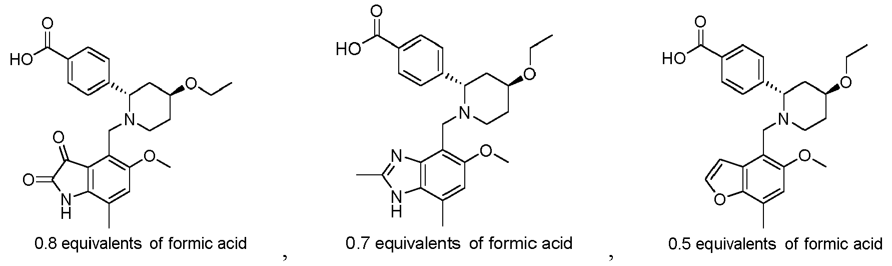 and

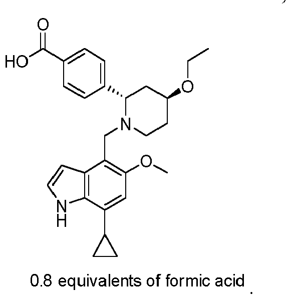

7. A preparation method for the heterocyclic compound represented by formula I according to any one of claims 1-6, comprising the following steps:
subjecting a compound represented by formula II to a de-esterification reaction as shown below in a solvent in the presence of a base to give the heterocyclic compound represented by formula I: 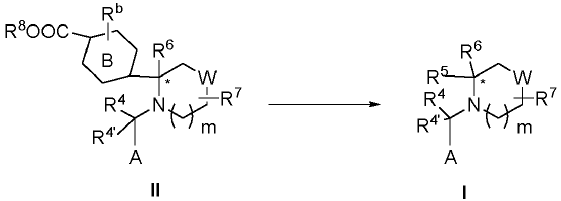 wherein R⁸ is C₁-C₄ alkyl; R^{b}, R⁴, R^{4'}, R⁶, R⁷, A, W, m and * are as defined in any one of claims 1-6.

8. A heterocyclic compound represented by formula II, 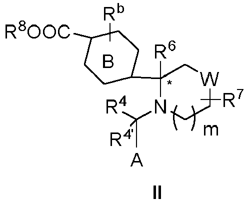 wherein R⁸ is C₁-C₄ alkyl; R^{b}, R⁴, R⁴, R⁶, R⁷, A, W, m and * are as defined in any one of claims 1-6;
for example, 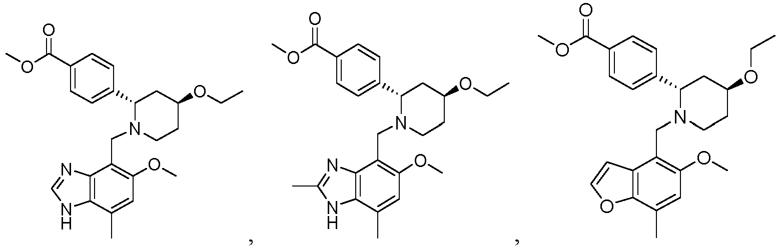 or

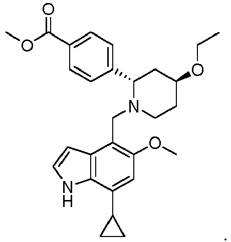

9. A pharmaceutical composition, comprising the heterocyclic compound represented by formula I or the pharmaceutically acceptable salt, the isotopic analog or the prodrug thereof according to any one of claims 1-6, and one or more pharmaceutically acceptable carriers.

10. Use of the heterocyclic compound represented by formula I or the pharmaceutically acceptable salt, the isotopic analog or the prodrug thereof according to any one of claims 1-6 as a complement factor B inhibitor or for the manufacturing of a medicament, wherein the medicament can be used for treating a disease associated with abnormal activation of complement factor B or occur in normal functioning of complement factor B; or, the medicament can be used for treating a disease selected from blood, autoimmune, inflammatory and neurodegeneration diseases and the like.
